# EUROPEAN PATENT SPECIFICATION

(11) **EP 2 129 788 B1**
(45) Date of publication and mention of the grant of the patent: **03.09.2014**
(21) Application number: 08724992.6
(22) Date of filing: 30.01.2008
(51) Int. Cl.: C12Q 1/68

(54) **LUMINESCENT COMPOUNDS**
LUMINESZENTE VERBINDUNGEN
COMPOSES LUMINESCENTS

(30) Priority: 30.01.2007 US 887311 P
(43) Date of publication of application: 09.12.2009
(73) Proprietor: SETA BioMedicals, LLC, Urbana, IL 61801 (US)
(72) Inventor: TERPETSCHNIG, Ewald, A., Urbana, IL 61801 (US); PATSENKER, Leonid, D., Kharkov, 61001 (UA); KLOCHKO, Oleksii, Kharkov, 61204 (UA); KUDRYAVTSEVA, Yuliia, Kharkov, 61204 (UA); TATARETS, Anatoliy, Kharkov, 61135 (UA); YERMOLENKO, Inna, Kharkov, 61204 (UA); POVROZIN, Yevgen, Kharkov, 61072 (UA)
(74) Representative: Willett, Christopher David
(86) International application number: PCT/US2008/001274
(87) International publication number: WO 2008/094637

(56) References cited:
- WO-A1-02/24816
- WO-A2-02/100975
- WO-A2-03/087052
- US-A- 5 039 818
- US-A- 5 567 687
- US-A- 5 569 587
- US-A- 6 083 485
- US-A1- 2002 077 487
- US-A1- 2004 166 515
- US-A1- 2006 141 530
- US-A1- 2006 159 738
- US-A1- 2006 160 068
- US-B1- 6 417 402
- US-B2- 6 599 605
- US-B2- 6 995 274
- EASWARAN ARUNKUMAR ET AL: "Squaraine-Derived Rotaxanes: Sterically Protected Fluorescent Near-IR Dyes", JOURNAL OF THE AMERICAN CHEMICAL SOCIETY, vol. 127, no. 10, 1 March 2005 (2005-03-01), pages 3288-3289, XP55033297, ISSN: 0002-7863, DOI: 10.1021/ja042404n
- EASWARAN ARUNKUMAR ET AL: "Singlet oxygen generation using iodinated squaraine and squaraine-rotaxane dyes", NEW JOURNAL OF CHEMISTRY, vol. 31, no. 5, 1 January 2007 (2007-01-01), page 677, XP55033299, ISSN: 1144-0546, DOI: 10.1039/b616224j
- ARUNKUMAR ET AL.: 'Squaraine-Derived Rotaxanes: Highly Stable, Fluorescent Near-IR Dyes' CHEM. EUR. J. 2006 vol. 12, 31 March 2006, pages 4684 - 4690, XP008115111

## Description

### Technical Field

The invention relates to compounds based on cyanines, squaric acid, among others. More particularly, the invention relates to compounds based on cyanines, squaric acid, among others, that are useful as dyes and luminescent reporters.

### Background

Colorimetric and/or luminescent compounds may offer researchers the opportunity to use color and light to analyze samples, investigate reactions, and perform assays, either qualitatively or quantitatively. Generally, brighter, more photostable reporters may permit faster, more sensitive, and more selective methods to be utilized in such research.

While a colorimetric compound absorbs light, and may be detected by that absorbance, a luminescent compound, or luminophore, is a compound that emits light. A luminescence method, in turn, is a method that involves detecting light emitted by a luminophore, and using properties of that light to understand properties of the luminophore and its environment. Luminescence methods may be based on chemiluminescence and/or photoluminescence, among others, and may be used in spectroscopy, microscopy, immunoassays, and hybridization assays, among others.

Photoluminescence is a particular type of luminescence that involves the absorption and subsequent re-emission of light. In photoluminescence, a luminophore is excited from a low-energy ground state into a higher-energy excited state by the absorption of a photon of light. The energy associated with this transition is subsequently lost through one or more of several mechanisms, including production of a photon through fluorescence or phosphorescence.

Photoluminescence may be characterized by a number of parameters, including extinction coefficient, excitation and emission spectrum, Stokes' shift, luminescence lifetime, and quantum yield. An extinction coefficient is a wavelength-dependent measure of the absorbing power of a luminophore. An excitation spectrum is the dependence of emission intensity upon the excitation wavelength, measured at a single constant emission wavelength. An emission spectrum is the wavelength distribution of the emission, measured after excitation with a single constant excitation wavelength. A Stokes' shift is the difference in wavelengths between the maximum of the emission spectrum and the maximum of the absorption spectrum. A luminescence lifetime is the average time that a luminophore spends in the excited state prior to returning to the ground state. A quantum yield is the ratio of the number of photons emitted to the number of photons absorbed by a luminophore.

Luminescence methods may be influenced by extinction coefficient, excitation and emission spectra, Stokes' shift, and quantum yield, among others, and may involve characterizing fluorescence intensity, fluorescence polarization (FP), fluorescence resonance energy transfer (FRET), fluorescence lifetime (FLT), total internal reflection fluorescence (TIRF), fluorescence correlation spectroscopy (FCS), fluorescence recovery after photobleaching (FRAP), and their phosphorescence analogs, among others.

Luminescence methods have several significant potential strengths. First, luminescence methods may be very sensitive, because modern detectors, such as photomultiplier tubes (PMTs) and charge-coupled devices (CCDs), can detect very low levels of light. Second, luminescence methods may be very selective, because the luminescence signal may come almost exclusively from the luminophore.

Despite these potential strengths, luminescence methods may suffer from a number of shortcomings, at least some of which relate to the luminophore. For example, the luminophore may have an extinction coefficient and/or quantum yield that is too low to permit detection of an adequate amount of light. The luminophore also may have a Stokes' shift that is too small to permit detection of emission light without significant detection of excitation light. The luminophore also may have an excitation spectrum that does not permit it to be excited by wavelength-limited light sources, such as common lasers and arc lamps. The luminophore also may be unstable, so that it is readily bleached and rendered nonluminescent. The luminophore also may have an excitation and/or emission spectrum that overlaps with the well-known autoluminescence of biological and other samples; such autoluminescence is particularly significant at wavelengths below about 600 nm. The luminophore also may be expensive, especially if it is difficult to manufacture. The luminophore may be highly quenched when labeled to proteins or other biomolecules at higher dye-to-biomolecule ratios.

### Brief Description of the Drawings

Figure1. A plot comparing the photostability (decrease of fluorescence intensity upon exposure to light of a 200 W Xenon lamp) of compounds 4 and 4a in water. Also shown is the relative photostability of the commercially available label Cy5.
Figure 2. A plot comparing the relative emission maxima of squaraine-rotaxane compound 10 and squaraine compound 10a in water.
Figure 3. A plot comparing the photostability (decrease of fluorescence intensity upon exposure to light of a 200 W Xenon lamp) of rotaxane compound 10, squaraine compound 10a and rotaxane conjugate compound 10-IgG in aqueous buffer.
Figure 4. A plot showing the absorption and emission spectra of the squaraine-rotaxane conjugate compound 10-IgG in phosphate buffer (pH 7.4, Dye-to-protein ratio = 1.0).
Figure 5. A plot comparing quantum yield vs. dye-to-protein ratio for squaraine-rotaxane-conjugate compound 10-IgG and a Cy5-IgG conjugate in phosphate buffer (pH 7.4)
Figure 6. A plot of normalized fluorescence intensity vs. pH for rotaxane compound 4 showing a pKa of around pH 11.
Figure 7. A plot comparing the relative luminescent intensity changes of rotaxane compound 4 and squaraine precursor compound 4a upon exposure to a hydrogen peroxide solution.
Figure 8. A plot comparing the stability of compounds **4** and **4a** in the presence of NaOH solution at pH 13. After 60 min incubation the solutions were neutralized with acid and the emission was measured. The intensities were then compared to the intensity at time = 0.

### Abbreviations

The following abbreviations, among others, may be used in this application:

| **Abbreviation** | **Definition** |
|---|---|
| BSA | bovine serum albumin |
| Bu | butyl |
| DMF | dimethylformamide |
| D/P | dye-to-protein ratio |
| Et | ethyl |
| g | grams |
| h | hours |
| HSA | human serum albumin |
| L | liters |
| m | milli (10⁻³) |
| M | molar |
| Me | methyl |
| mol | moles |
| M.P. | melting point |
| nm | nanometer (10⁻⁹ meter) |
| NHS | N-hydroxysuccinimide |
| NIR | near infrared region |
| PBS | phosphate-buffered saline |
| Prop | propyl |
| TMS | tetramethylsilane |
| TSTU | N,N,N',N'-tetramethyl(succinimido)uronium tetrafluoroborate |
| µ | micro (10⁻⁶) |

### Description of the Preferred Embodiments

The invention relates generally to photoluminescent compounds and their synthetic precursors, and to methods of synthesizing and using such compounds. These photoluminescent compounds may be useful in both free and conjugated forms, as probes, labels, and/or indicators. This usefulness may reflect in part enhancement of one or more of the following: quantum yield, fluorescent lifetime, Stokes' shift, extinction coefficients, photostability and chemical stability. This usefulness also may reflect excitation and emission spectra in relatively inaccessible regions of the spectrum, including the red and near infrared.

More particularly, the invention provides rotaxane reporter compounds based on cyanines, squaric acid, among others, reactive intermediates used to synthesize the reporter compounds, and methods of synthesizing and using the reporter compounds, among others.

Rotaxanes are a class of mechanically-interlocked molecular complexes. In the case of rotaxanes, the complex includes an at least partially "dumbbell-shaped" molecule that is threaded through the central cavity of a macrocyclic molecule. Although there are typically no covalent bonds between the two components of the rotaxane, the two components remain interlocked, because the ends of the "dumbbell-shaped" molecule are larger than the internal diameter of the macrocycle cavity. Where the molecule threaded through the macrocycle is a luminescent reporter molecule, the presence of the macrocycle can confer enhanced stability on the reporter molecule.

The remaining discussion includes (1) an overview of structures, (2) an overview of synthetic methods, and (3) a discussion of the applications of the invention.

### Overview of Structures

The invention relates to reporter compounds defined as a rotaxane according to claim 1.

Alternatively, or in addition, A, B, C, and D may be chosen so that the compound is photoluminescent.

The particular substituents on the substituted rings may be chosen quite broadly, and may include the various component listed above, among others. Selection of a particular combination of substituents may be used to fine-tune the spectral properties of the reporter compound, alter the hydrophilicity or hydrophobicity or the reporter compound, or otherwise tailor the properties of the reporter compound to a particular application.

### Reporter compounds

Where the reporter compound is a colorimetric dye and/or a photoluminescent compound based on an aromatic center, B and C are typically chosen from W¹ and W², and B and C, are separated by A and D.

Depending on the embodiment, A, B, C and D may be subject to additional limitations. In some embodiments, the compound also includes at least one of O, OR, S, Se, Te, N-R^{a}, C(R^{b})(R^{c}). In other embodiments, at least one of X¹ through X⁴ of W¹, W² or W³ is or includes a heteroatom or a reactive linker. In yet other embodiments, the compound may include a reactive group and/or a carrier. The reporter compounds may be colorimetric dyes, useful as stains and for colorimetric detection. Alternatively or in addition, the reporter compounds may be photoluminescent, particularly fluorescent, and may have utility in photoluminescence assays and methods, as discussed above.

Important precursors for these rotaxanes are described in the experimental section.

### Reactive groups R^{x}

The substituents of Z may include one or more reactive groups, where a reactive group generally is a group capable of forming a covalent attachment with another molecule or substrate. Such other molecules or substrates may include proteins, carbohydrates, nucleic acids, and plastics, among others. Reactive groups vary in their specificity, and may preferentially react with particular functionalities and molecule types. Thus, reactive compounds generally include reactive groups chosen preferentially to react with functionalities found on the molecule or substrate with which the reactive compound is intended to react.

The compounds of the invention are optionally substituted, either directly or via a substituent, by one or more chemically reactive functional groups that may be useful for covalently attaching the compound to a desired substance. Each reactive group, or R^{x}, may be bound to the compound directly by a single covalent bond, or may be attached via a covalent spacer or linkage, L, and may be depicted as -L-R^{x}.

The reactive functional group of the invention R^{x} is selected from the following functionalities: activated carboxylic esters, acyl azides, acyl halides, acyl nitriles, acyl nitriles, aldehydes, ketones, alkyl halides, alkyl sulfonates, anhydrides, aryl halides, aziridines, boronates, carboxylic acids, carbodiimides, diazoalkanes, epoxides, haloacetamides, halotriazines, imido esters, isocyanates, isothiocyanates, maleimides, phosphoramidites, silyl halides, sulfonate esters, and sulfonyl halides, and
a) N-hydroxysuccinimide esters, isothiocyanates, and sulfonylchlorides, which form stable covalent bonds with amines, including amines in proteins and amine-modified nucleic acids;
b) iodoacetamides and maleimides, which form covalent bonds with thiol-functions, as in proteins;
c) Carboxyl functions and various derivatives, including N-hydroxybenztriazole esters, thioesters, p-nitrophenyl esters, alkyl, alkenyl, alkynyl, and aromatic esters, and acyl imidazoles;
d) Alkylhalides, including iodoacetamides and chloroacetamides;
e) Hydroxyl groups, which can be converted into esters, ethers, and aldehydes;
f) Aldehydes and ketones and various derivatives, including hydrazones, oximes, and semicarbozones;
g) Isocyanates, which may react with amines;
h) Activated C=C double-bond-containing groups, which may react in a Diels-Alder reaction to form stable ring systems under mild conditions;
i) Thiol groups, which may form disulfide bonds and react with alkylhalides (such as iodoacetamide);
j) Alkenes, which can undergo a Michael addition with thiols, e.g., maleimide reactions with thiols;
k) Phosphoramidites, which can be used for direct labeling of nucleosides, nucleotides, and oligonucleotides, including primers on solid or semi-solid supports;
l) Primary amines that may be coupled to variety of groups including carboxyl, aldehydes, ketones, and acid chlorides, among others;
m) Boronic acid derivatives that may react with sugars;
n) Pyrylium moieties react with primary amines;
o) Haloplatinates form stable platinum complexes with amines, thiols and heterocycles; and
p) Aryl halides react with thiols and amines.

### R groups

The R moieties associated with the various substituents of Z may include any of a number of groups, as described above, including but not limited to alicyclic groups, aliphatic groups, aromatic groups, and heterocyclic rings, as well as substituted versions thereof.

Aliphatic groups may include groups of organic compounds characterized by straight- or branched-chain arrangement of the constituent carbon atoms. Aliphatic hydrocarbons comprise three subgroups: (1) paraffins (alkanes), which are saturated and comparatively unreactive; (2) olefins (alkenes or alkadienes), which are unsaturated and quite reactive; and (3) acetylenes (alkynes), which contain a triple bond and are highly reactive. In complex structures, the chains may be branched or cross-linked and may contain one or more heteroatoms (such as polyethers and polyamines, among others).

As used herein, "alicyclic groups" include hydrocarbon substituents that incorporate closed rings. Alicyclic substituents may include rings in boat conformations, chair conformations, or resemble bird cages. Most alicyclic groups are derived from petroleum or coal tar, and many can be synthesized by various methods. Alicyclic groups may optionally include heteroalicyclic groups that include one or more heteroatoms, typically nitrogen, oxygen, or sulfur. These compounds have properties resembling those of aliphatics and should not be confused with aromatic compounds having the hexagonal benzene ring. Alicyclics may comprise three subgroups: (1) cycloparaffins (saturated), (2) cycloolefins (unsaturated with two or more double bonds), and (3) cycloacetylenes (cyclynes) with a triple bond. The best-known cycloparaffins (sometimes called naphthenes) are cyclopropane, cyclohexane, and cyclopentane; typical of the cycloolefins are cyclopentadiene and cyclooctatetraene. Most alicyclics are derived from petroleum or coal tar, and many can be synthesized by various methods.

Aromatic groups may include groups of unsaturated cyclic hydrocarbons containing one or more rings. A typical aromatic group is benzene, which has a 6-carbon ring formally containing three double bonds in a delocalized ring system. Aromatic groups may be highly reactive and chemically versatile. Most aromatics are derived from petroleum and coal tar. Heterocyclic rings include closed-ring structures, usually of either 5 or 6 members, in which one or more of the atoms in the ring is an element other than carbon, e.g., sulfur, nitrogen, etc.. Examples include pyridine, pyrole, furan, thiophene, and purine. Some 5-membered heterocyclic compounds exhibit aromaticity, such as furans and thiophenes, among others, and are analogous to aromatic compounds in reactivity and properties.

Any substituent of the compounds of the invention, including any aliphatic, alicyclic, or aromatic group, may be further substituted one or more times by any of a variety of substituents, including without limitation, F, Cl, Br, I, carboxylic acid, sulfonic acid, CN, nitro, hydroxy, phosphate, phosphonate, sulfate, cyano, azido, amine, alkyl, alkoxy, trialkylammonium or aryl. Aliphatic residues can incorporate up to six heteroatoms selected from N, O, S. Alkyl substituents include hydrocarbon chains having 1-22 carbons, more typically having 1-6 carbons, sometimes called "lower alkyl".

As described in WO 2001/11370, sulfonamide groups such as -(CH₂)ₙ-SO₂-NH-SO₂-R, - (CH₂)ₙ-CONH-SO₂-R, -(CH₂)ₙ-SO₂-NH-CO-R, and -(CH₂)ₙ-SO₂NH-SO₃H, where R is aryl or alkyl and n = 1 - 6, can be used to reduce the aggregation tendency and have positive effects on the photophysical properties of cyanines and related dyes, in particular when these functionalities are directly associated with the benzazole ring in position 1 (the nitrogen atom in the azole ring).

Where a substituent is further substituted by a functional group R^{±} that is ionically charged, such as, for example, a carboxylic acid, sulfonic acid, phosphoric acid, phosphonate or a quaternary ammonium group, the ionic substituent R^{±} may serve to increase the overall hydrophilic nature of the compound.

As used herein, functional groups such as "carboxylic acid," "sulfonic acid," and "phosphoric acid" include the free acid moiety as well as the corresponding metal salts of the acid moiety, and any of a variety of esters or amides of the acid moiety, including without limitation alkyl esters, aryl esters, and esters that are cleavable by intracellular esterase enzymes, such as alpha-acyloxyalkyl ester (for example acetoxymethyl esters, among others).

The compounds of the invention are optionally further substituted by a reactive functional group R^{x}, or a conjugated substance S_{c}, as described below.

The compounds of the invention may be depicted in structural descriptions as possessing an overall charge, it is to be understood that the compounds depicted include an appropriate counter ion or counter ions to balance the formal charge present on the compound. Further, the exchange of counter ions is well known in the art and readily accomplished by a variety of methods, including ion-exchange chromatography and selective precipitation, among others.

### Carriers and Conjugated Substances S_{c}

The reporter compounds of the invention, including synthetic precursor compounds, may be covalently or noncovalently associated with one or more substances. Covalent association may occur through various mechanisms, including a reactive functional group as described above, and may involve a covalent linkage, L, separating the compound or precursor from the associated substance (which may therefore be referred to as -L-S_{c}).

The covalent linkage L binds the reactive group R^{x}, the conjugated substance S_{c} or the ionic group R^{±} to the dye molecule, either directly (L is a single bond) or with a combination of stable chemical bonds, that include single, double, triple or aromatic carbon-carbon bonds; carbon-sulfur bonds, carbon-nitrogen bonds, phosphorus-sulfur bonds, nitrogen-nitrogen bonds, nitrogen-oxygen or nitrogen-platinum bonds, or aromatic or heteroaromatic bonds; L includes ether, thioether, carboxamide, sulfonamide, urea, urethane or hydrazine moieties. Preferrable L's include a combination of single carbon-carbon bonds and carboxamide or thioether bonds.

Where the substance is associated noncovalently, the association may occur through various mechanisms, including incorporation of the compound or precursor into or onto a solid or semisolid matrix, such as a bead or a surface, or by nonspecific interactions, such as hydrogen bonding, ionic bonding, or hydrophobic interactions (such as Van der Waals forces). The associated carrier may be selected from the group consisting of polypeptides, polynucleotides, carbohydrades, nucleic acids, nucleotide triphosphates, polysaccharides, haptens, RNAs, PNAs, beads, microplate well surfaces, metal surfaces, semiconductor and non-conducting surfaces, nano-particles, and other solid surfaces.

The associated or conjugated substance may be associated with or conjugated to more than one reporter compound, which may be the same or different. Generally, methods for the preparation of dye-conjugates of biological substances are well-known in the art. See, for example, Haugland, MOLECULAR PROBES HANDBOOK OF FLUORESCENT PROBES AND RESEARCH CHEMICALS, Sixth Edition (1996), which is hereby incorporated by reference. Typically, the association or conjugation of a chromophore or luminophore to a substance imparts the spectral properties of the chromophore or luminophore to that substance.

Sc is a conjugated substance selected from, amino acids, peptides, proteins, nucleosides, nucleotides, nucleic acids, carbohydrates, haptens, lipids, ion-chelators, nonbiological polymers, cells, or a cellular component, or is selected from a protein, carbohydrates, nucleic acids, and nonbiological polymers such as plastics, metallic nanoparticles such as gold, silver and carbon nanostructures among others, or is selected from the group consisting of a peptide, a nucleotide, a polypeptide, a polynucleotide, a bead, a microplate well surface, a phospholipid, a metallic nanoparticle, an amino acid, a nucleic acid, a sugar, polysaccaride, oligosaccharide, and a second fluorescent dye. The substance to be conjugated may be protected on one or more functional groups in order to facilitate the conjugation, or to insure subsequent reactivity.

Where the substance is a peptide, the peptide may be a dipeptide or larger, and typically includes 5 to 36 amino acids. Where the conjugated substance is a protein, it may be for example, an enzyme, an antibody, lectin, protein A, protein G, hormones, or a phycobiliprotein. The conjugated substance may be a nucleic acid polymer, such as for example DNA oligonucleotides, RNA oligonucleotides (or hybrids thereof), or single-stranded, double-stranded, triple-stranded, or quadruple-stranded DNA, or single-stranded or double-stranded RNA. Another class of carriers includes carbohydrates that are polysaccharides, such as dextran, heparin, glycogen, starch and cellulose.

Where the substance is an ion chelator, the resulting conjugate may be useful as an ion indicator (calcium, sodium, magnesium, zinc, potassium and other important metal ions) particularly where the optical properties of the reporter-conjugate are altered by binding a target ion. Preferred ion-complexing moieties are crown ethers (U.S. Patent No. 5,405,957) and BAPTA chelators (U.S. Patent No. 5,453,517).

The associated or conjugated substance may be a member of a specific binding pair, and therefore useful as a probe for the complementary member of that specific binding pair, each specific binding pair member having an area on the surface or in a cavity which specifically binds to and is complementary with a particular spatial and polar organization of the other. The conjugate of a specific binding pair member may be useful for detecting and optionally quantifying the presence of the complementary specific binding pair member in a sample, by methods that are well known in the art.

Representative specific binding pairs may include ligands and receptors, and may include but are not limited to the following pairs: antigen-antibody, biotin-avidin, biotin-streptavidin, IgG-protein A, IgG-protein G, carbohydrate-lectin, enzyme-enzyme substrate; ion-ion-chelator, hormone-hormone receptor, protein-protein receptor, drug-drug receptor, DNA-antisense DNA, and RNA-antisense RNA. Further carrier systems include cellular systems (animal cells, plant cells, bacteria). Reactive dyes can be used to label groups at the cell surface, in cell membranes, organelles, or the cytoplasm.

Finally these compounds can be linked to small molecules such as amino acids, vitamines, drugs, haptens, toxins, environmental pollutants. Another important ligand is tyramine, where the conjugate is useful as a substrate for horseradish peroxidase. Additional embodiments are described in U.S. Patent Application Publication No. US2002/0077487.

### Synthesis

### a) Dye components

The synthesis of the disclosed reporter compounds typically is achieved in a multi-step reaction, starting with the synthesis of a methylene base. The synthesis of suitable methylene bases can be achieved based on literature or novel methods. Generally, the spectral properties of the reporter compounds, including excitation and emission wavelengths for luminescent compounds, may be strongly dependent on the type of methylene base used. Typical starting materials include quartemized indolenines, benzthiazoles, benzoxazoles, benzimidazoles among others, and either squaric acid and its derivatives, or N,N'-diphenylformamidine or malonaldehyde bis(phenylimine) monohydrochloride. For aniline-based squaraine dyes typical starting materials are various aniline-derivatives and squaric acid.

The dye molecules of this invention typically consist of a bridging unit and the heterocyclic bases W¹ and W², W³ and W⁴, W⁵ and W⁶, W⁷ and W⁸ or a combination thereof.

With regards to the aniline based dyes the aniline-moiety may be directly connected to the squaraine center. When there is a bridging unit it is either a simple polymethine chain of various lengths or it can additionally contain an aromatic squaraine ring system. When the bridging unit is a polymethine chain the coupling agent can be N,N-diphenylformamidine, triethylorthoformate, or malonaldehyde bis(phenylimine) hydrochloride, 1,1,3-trimethoxypropane, 1,1,3,3-tetramethoxypropane and glutaconaldehyde dianil monohydrochloride.

Squaraine dyes are synthesized using squaric acid or one of its derivatives as starting materials and reacting them with a methylene base to form the dye. The synthesis of starting materials and some of the most important precursors for the synthesis of cyanine and squaraine dyes are described in Example 1.

Squaric acid is a dibasic acid that undergoes a series of nucleophilic substitution reactions with various reagents, including amines, phenols, and CH-acidic compounds such as 1,2,3,3-tetramethylbenzindole. The squaraine bridge in the resulting compounds stabilizes the conjugated chain and shifts the excitation and emission wavelength of these dyes as compared to cyanine-based dyes. The exchange of the oxygen in the squaraine moiety by an imino (=N-R), sulfur (=S), or a methylene (=CR₂) moiety was also shown to be a pathway to dyes with useful luminescent properties. The spectral properties of squaraine dyes are modified by thio-, imino- and methylenederivatization of the squaraine bridge.

In general squaraine-based markers exhibit low to moderate quantum yields in water (φ = 0.05 - 0.3) and very high quantum yields (up to φ = 0.7) when covalently on non-covalently bound to biomolecules or in organic solvents. The absorption and emission wavelengths of the reporter compounds may be tuned by variation of the methylene base (e.g. indolenine vs. benzthiazole) and by an increase or decrease of the length of the conjugated carbon chain. Thus, the indoleninesquaraines absorb around 635 nm to 650 nm in water. The absorption and emission spectra of benzothiazolium and benzoselenazolium based dyes are shifted towards longer wavelengths. The emission maxima for benzothiazole based squaraine dyes in organic solvents are around 680 nm to 690 nm and can be found beyond 700 nm for benzoselenzole derivatives.

Benzoxazole and oxazole based squaraines absorb and emit even at shorter wavelengths than indolenine-based squaraines. The synthesis of benzoxazole-squaraines and other squaraines based on five-membered heterocyclic rings is described in WO 2003/087052.

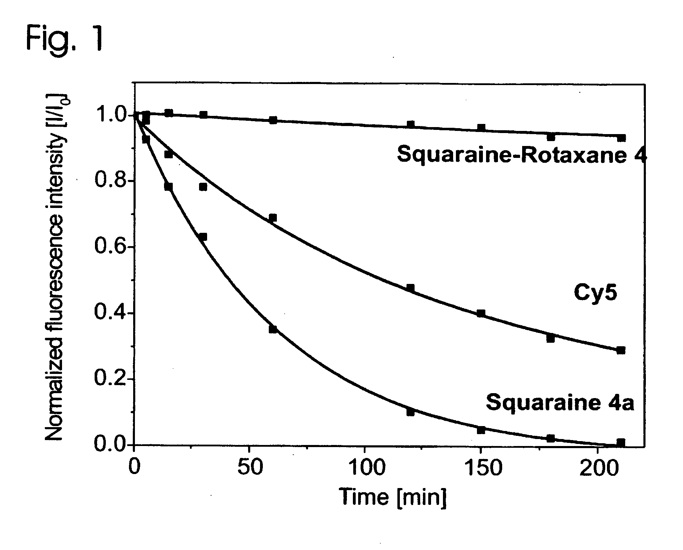

Importantly, the Stokes' shift increases in these longer wavelength-emitting benzoselenazolim and thiazolium dyes, which increases the sensitivity of a fluorescent measurement. Nevertheless the photostability of these dyes are comparably lower as for the indolenine based dyes. Encapsulation by rotaxane-formation helps to improve on the photostabilities of squaraine dyes which is exemplified in Figure 1.

Starting materials for the synthesis of squaraines are described in Example 1. The synthesis of cyanine dyes that include a cycloalkene-bridging element are described in U.S. Patent Application Publication No. US2004/0014981 A1 and by J. Flanagan et al. in Bioconjugate Chem. 8, 751-756 (1997), which are hereby incorporated by reference.

To enhance water-solubility, sulfonic acid or other groups such as including quaternary ammonium, polyether, carboxyl, and phosphate, among others, may be introduced into the heterocyclic ring systems. In order to facilitate covalent attachment to proteins, reactive N-hydroxysuccinimide ester (NHS ester) or other reactive derivatives may be synthesized.

The synthesis of cyanine dyes is described in Mujumdar et al., Bioconjugate Chem. 4(2) 105-111, 1993 and in several other patent applications (U.S. Patent Application Publication No. US2002/0077487 A1, U.S. Patent No. 5,569,587, U.S. Patent No. 5,672,027, U.S. Patent No. 5,808,044). The cyanine dyes incorporated in this invention may exhibit absorption maxima in the range between 500 and 850 nm. In addition to a variety of other structural parameters, the selection of a monomethine, trimethine, or pentamethine linkages permits the spectral properties of the resulting compound to be altered according to the characteristics desired. For example, where the remainder of the compound is held constant, shifting from a monomethine to a trimethine, to a pentamethine linkage in a W¹ or W² substituent typically results in a shift of the absorption and emission wavelengths of the resulting compounds to progressively longer wavelengths. The absorption maxima can be fine-tuned by additional introduction of functional groups to match the emission lines of a frequency-doubled Nd-Yag laser (532 nm), Kr-ion laser (568 and 647 nm), the HeNe laser (543 nm and 633 nm) and diode lasers (370 nm, 405 nm, 436 nm, 635 nm, 650 nm, 780 nm etc.). Unlike some squaraines, cyanine dyes exhibit a lesser tendency to change their quantum yields upon changing the environment (e.g. labelling to a protein).

Many compounds of the invention have an overall electronic charge. It is to be understood that when such electronic charges are present, that they are balanced by an appropriate counter-ion, which may or may not be identified.

### b) Rotaxanes

The encapsulation procedures that are utilized to synthesize the rotaxanes of these inventions depend on the type of the encapsulating species used: one way to synthesize these rotaxanes is using the templated amide macrocylization chemistry developed by Leigh et al. [Angew. Chem. Int. Ed. 36(7), 728-732 (1997); Chem. Eur. J. 2004, 10, 4960-4969].

In this approach the dye component is dissolved in an inert solvent (depending on solubility of the compounds chloroform, or solvent mixtures might be used) and then titrated with equimolar amounts of diacid dichloride and xylylenediamine (e.g. 2,6 pyridinedicarbonyl dichloride or isophthaloyl dichloride and p-xylylenediamine) in presence of NEt₃ as base. Depending on the nature of the rotaxanes formed under these conditions they are purified either by reversed-phase or silica column chromatography. The reported synthetic yields are around 5 and 30%.

An encapsulation procedure for a non-reactive, aniline-type squaraine dye was reported by Arunkumar et al. in Chem. Eur. J. 2006, 12, 4684-4690.

Another way to produce water-soluble encapsulated dyes was described by Anderson at al. in Angew. Chem Int. Ed. Engl. 1997, 36 (12), 1310-13. In this procedure the dye is synthesized in the presence of cyclodextrins (-CD, -CD) or a synthetic cyclophane macrocycle at 0-5 °C. Paper chromatography was used to purified these compounds that were isolated in 12 and 15% yields. The hydrophobic effect was used to direct the synthesis of azo-dye rotaxanes with both CD and cyclophane macrocycles. In most solvents all of these rotaxanes were more soluble than the non-rotaxanated dyes and in addition they exhibited a lesser tendency to aggregate than their non-rotaxanated analogues.

There are several important factors that need to be considered when synthesizing novel Leigh-type aminde rotaxane structures:

Aniline-based squaraines containing ortho-hydroxy groups do not form rotaxanes. This can most likely be attributed to sterical hindrance caused by the o-hydroxy groups of the aniline ring:

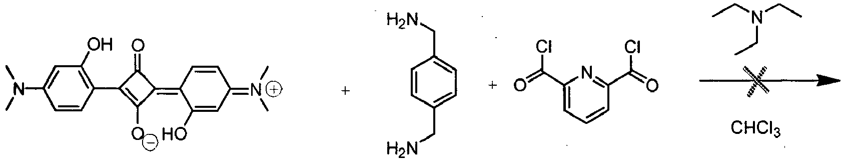

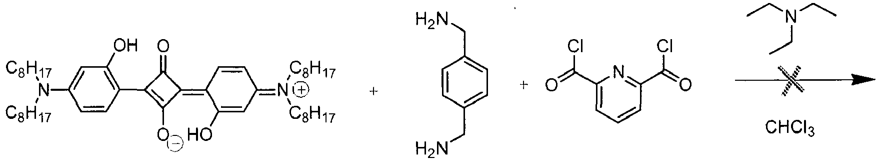

Also in the rotaxane-synthesis with indolenine-, benzthiazole-, and selenazole-type squaraine dyes sterical considerations need to be considered: Reactions of hydrophobic, indolenine-based squaraines containing either methyl or long-chain alkyl groups at the indolenine-nitrogen did not yield rotaxanes, which is evidence that sterical considerations indeed play an important role.

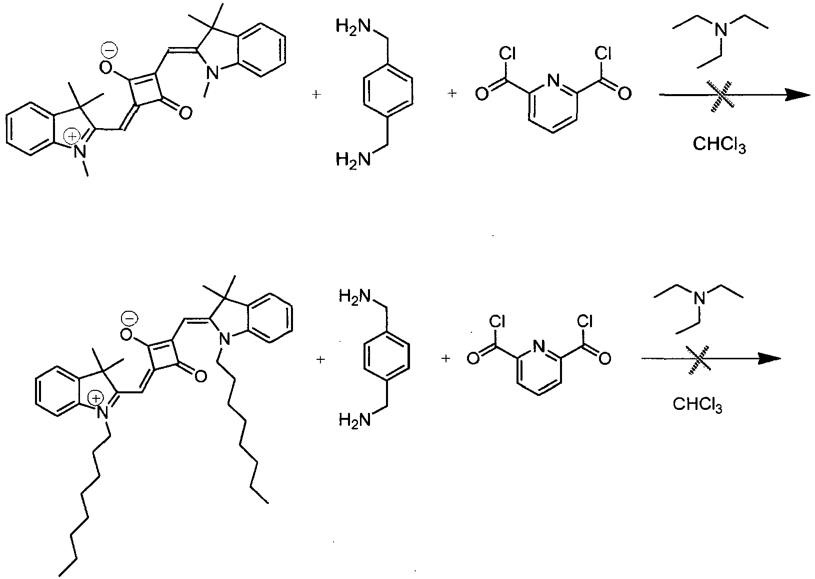

On the other hand, NH-substituted indolenines-, N-alkyl-benzothiazole- and N-alkylbenzoselenazole-based squaraines form rotaxanes with very favorable spectral and photophysical properties (see below).

It is also understood that the building blocks in Leigh-type aminde rotaxanes are exchangeable and they can be substituted with a variety of substituents:
Dimethoxy-substituted versions of p-xylylenediamine, synthesized via reduction of 2,5-dicyano-hydrochinon-dimethylether with LiAlH₄ have been described by Schill et al. in Liebigs Ann. Chem. 1973, 2055. A nitro-derivative of p-xylylenediamine that can be reduced to a reactive amino-function after the rotaxane ring is synthesized was described by Lustig in Chem. Ber. 28, 2987(1985).

The pyridine heterocycle of the dicarbonyl dichloride component in rotaxanes can be replaced with other heterocyclic components such as a thiophene, pyrrol or furan. 2,5-thiophenedicarbonyl dichloride is commercially available from Aldrich. 3,4-dimethoxy-2,5-furandicarboxylic acid, the precursor for the synthesis of the acid chloride is also available from Aldrich and can be converted into the acid chloride by treatment with PCl₅ or acetyl chloride according to Klinkhardt, J. Prakt. Chem. (2), 25, 1882, 51 or Lewkowski, Pol. J. Chem. 75, 12, 1943-46 (2001). The 1 H-pyrrole-2,5-dicarbonyl dichloride synthesis from dicarboxylic acid precursors which are available from Aldrich is described by Zielinski et al., Tetrahedron 61 (16), 4081-90 (2005). Starting materials like 3,4-Diethyl-1 H-pyrrole-2,5-dicarboxylic acid (L164216), 3,4-Ethylenedioxypyrrole-2,5-dicarboxylic acid (637203), 4-Methyl-3-(2-nitroethyl)-2,5-pyrroledicarboxylic acid (S951072), 3,4-Bis(2,2,3,3,4,4,4-heptafluorobutyl)-1H-pyrrole-2,5-dicarboxylic acid (L165859) are available from Aldrich. Other precursors are described in Example 1.

### Spectral and Photophysical Properties

The macro-cyclic encapsulation procedure helps to improve on the aggregation tendencies and the quenching effect that causes a reduction in quantum yield in aqueous solution. In addition such encapsulation may yield luminescent compounds that have longer luminescent lifetimes in water as compared to conventional non-encapsulated dyes. Such encapsulation procedure could also help to improve in the properties of cyanine dyes in particular where such encapsulation prevents the occurrence of photo-induced isomerization reactions.

Dye compositions that are described in this invention can be encapsulated to yield luminescent compounds with improved properties for luminescence detection in for bioanalytical and imaging applications.

The novel dye compositions that are introduced herein are aimed at improving the shortcomings of cyanines, in particular squaraine dyes such as short lifetimes and low quantum yields in aqueous solution. The short lifetime and low quantum yields can mostly be attributed to quenching and aggregation of the dye molecules in aqueous solution. The novel structural features that are introduced in this invention might also help to reduce the self-aggregation and quenching tendencies of these labels. Further these dye compositions should also exhibit improved chemical and photochemical stability. Most importantly, the invention aims at producing luminescent bio- and protein-conjugates that are less prone to reducing their quantum yields at higher dye-to-protein ratios as is the case with many other luminescent labels, including squaraines.

The following examples should help to demonstrate the favorable properties that are obtained when squaraine dyes (both - aniline and non-aniline type) are "rotaxanated":
The chemical structures of dyes **4a** and the rotaxane **4** are shown below. Both, **4a** and **4** contain the same symmetrical 5-sulfo-substituted squaraine dye but surprisingly, have very different photophysical properties (see Table below):

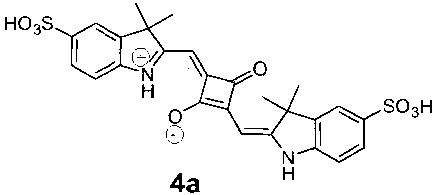

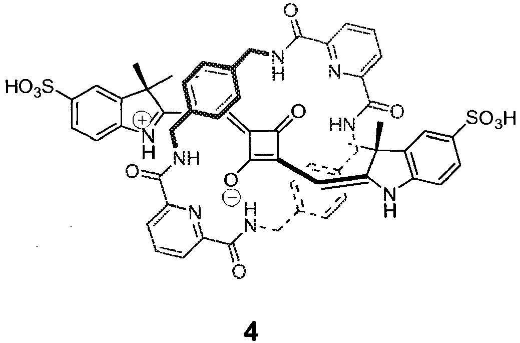

| **Dye** | **ₘₐₓ (abs)** | **ₘₐₓ (em)** | **Solvent** | **Stokes' Shift** | **Photostability t_{1/2} [min]** | **Quantum Yield [%]** | **Lifetime [ns]** |
|---|---|---|---|---|---|---|---|
| **4a** | 637 | 654 | water | 17 | ~ 40 | 26 | 3.3 |
| **4** | 640 | 656 | water | 16 | ~ 2000 | 40 | 4.4 |

While the absorption, emission wavelengths and Stokes' shifts of squaraine dye **4a** and squaraine-rotaxane **4** are similar, squaraine **4a** exhibits a mean fluorescence lifetime of 3.3 ns in water, while the lifetime of rotaxane **4** is in the order of 4.4 ns. Moreover, the rotaxane **4** has an approximately 50% higher quantum yield in water as compound to **4a**. The quantum yield of 40% that was determined for the rotaxane-dye **4** in water represents an extremely high value, and is even higher than the quantum yield for one of the brightest commercially available cyanine dyes (Alexa Fluor 647) which is reported to be 33% in aqueous solution. The squaraine-rotaxane also has a substantially higher photostability as can be seen from Figure 1 and the reported half-lifetimes (t_{1/2}) of these compounds upon exposure to light.

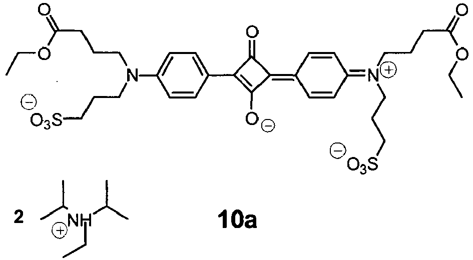

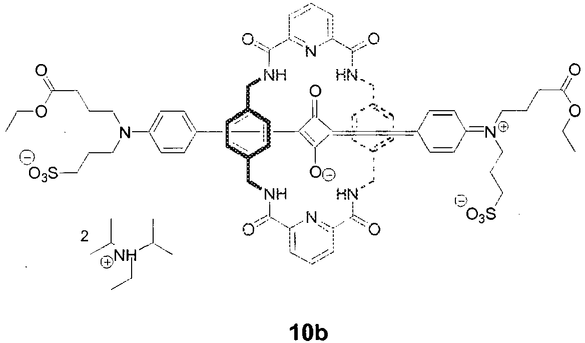

| **Dye** | **ₘₐₓ (abs)** | **ₘₐₓ (em)** | **Solvent** | **Stokes' Shift** | **Photostability t_{1/2}[min]** | **Quantum Yield [%]** | **Lifetime [ns]** |
|---|---|---|---|---|---|---|---|
| **10a** | 644 | 670 | water | 26 | 80 | 3 | 1.5 |
| **10b** | 655 | 673 | water | 18 | > 2000 | 25 | 2.3 |
| **10-IgG** | 657 | 675 | PB 7.4 | 18 | > 2000 | 31 | 3.2 |

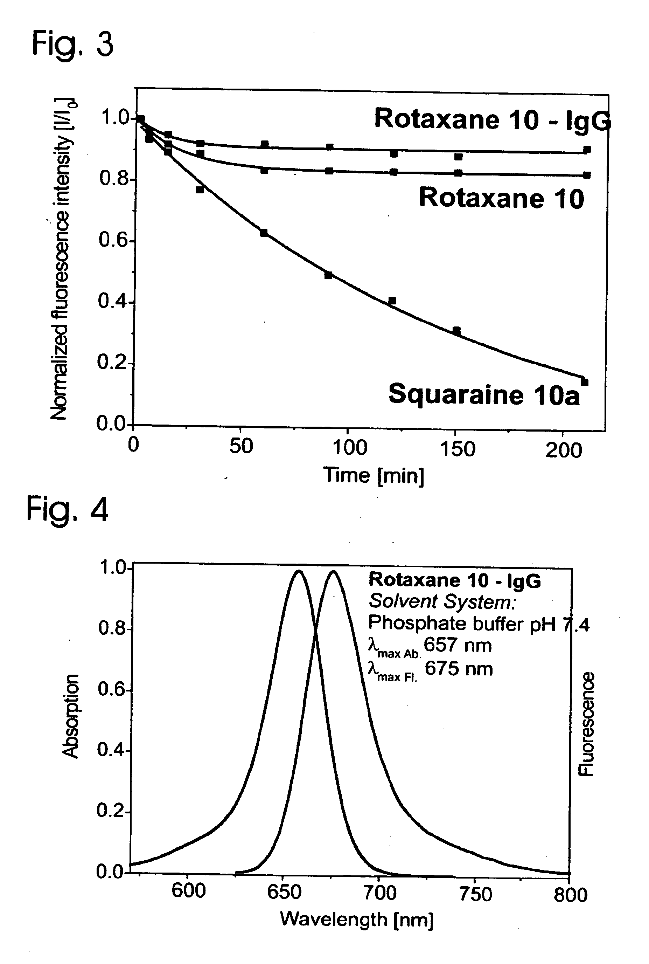

A similar behaviour is also exhibited by aniline-based squaraines. Rotaxane-formation leads to a bathochromic shift of the absorption and emission maxima in rotaxane **10b** as compared to the free squaraine dyes **10a**. Upon rotaxination the aniline-squaraine **10a** shows a dramatic increase in quantum yield from 3% to 25% in water, which is accompanied by a lifetime change from 1.5 to 2.3 ns. Importantly also the photostability of aniline-type squaraines increases substantially upon rotaxination (Figure 3).

Increased brightness and photostability are desirable features for fluorescent labels because they both permit enhanced detection, and therefore allow more sensitive measurements. Both, aniline and non-aniline type squaraines show improved features upon encapsulation by a rotaxane structure.

### EXAMPLE 1

### Synthesis of precursors and intermediates

This section describes the synthesis of various precursors and intermediates for the synthesis of novel cyanine dyes. p-hydrazinobenzenesulfonic acid (Illy et al., J. Org. Chem. 33, 4283-4285 (1968), 2,3,3-trimethylindole-5-sulfonic acid potassium salt (**1a**), 1-(5-carboxypentyl)-2,3,3-trimethyl-3H-5-indoliumsulfonate (**1b**), 1-(4-sulfonatobutyl)-2,3,3-trimethylindoleninium-5-sulfonate (**1h**) (Mujumdar et al., Bioconjugate Chem. 4(2), 105-111, 1993), and 1,2,3,3-tetramethylindoleninium-5-sulfonate (**1c**) were synthesized using literature procedures. **1d - 1f** are synthesized according to the procedures provided in U.S. Patent Application Publication No. US2002/0077487. 1-(2-phosphonethyl)-2,3,3-trimethylindoleninium-5-sulfonate (**1i**) is described in PCT Patent Application Publication No. WO 2001/36973.

The synthesis of unsymmetrical squaraine dyes is described in S. Yagi et al., J. Chem. Soc., Perkin Trans. 1, 2000, 599-603. The synthesis of sulfonated benz-indolenines and other cyclo-condensed heterocycles is described in U.S. Patent Application Publication No. US 2002/0077487 and U.S. Patent No. 6,140,494 and by S. Mujumdar et al., Bioconjugate Chem. 7, 356-362 (1996).

It is also understood that the additional aromatic ring can be fused at different positions onto the parent heterocycle (see WO02/26891 A1) and Mujumdar et al., Bioconjugate Chem. 7, 356-362 (1996).

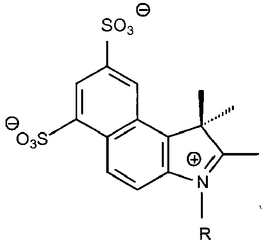

The synthesis of azolium compounds containing additional heteroatoms is also described in U.S. Patent Application Publication No. US2002/0077487.

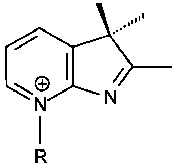

The synthesis of other fluorescent dyes and dye precursors is described in U.S. Patent Application Publication Nos. US2005/0202565, US2004/0166515 and US2002/0147354. Other squaraines and precursors are described in U.S. Patent No. 6,417,402.

Starting materials for the synthesis of the rotaxane ring systems (2,6 pyridinedicarbonyl dichloride or isophthaloyl dichloride and p-xylylenediamine) including a variety of cyclodextrins are available from Aldrich. Additional precursors are described below.

### Synthesis of 2,3,3-trimethylindole-5-sulfonic acid, potassium salt (1a)

### Synthesis of p-Hydrazinobenzenesulfonic acid

33 g of sodium carbonate was added to a suspension of 104 g (0.6 mol) of p-aminobenzenesulfonic acid in 400 mL of hot water. The solution was cooled to 5°C in an ice-bath, and 70 g of concentrated sulfuric acid were added slowly under rapid stirring. A solution of 42 g of sodium nitrite in 100 mL of water was then added under cooling. A light yellow diazo-compound precipitate formed, which was filtered and washed with water, but not dried.

The wet diazo-compound was added under stirring and cooling (5 °C) to a solution of 170 g of sodium sulfite in 500 mL of water. The solution, which turned orange, was stirred under cooling for 1 h, and then heated to reflux. Finally, 400 mL of concentrated hydrochloric acid were added. The solution turned yellow, and the product precipitated as a white solid. For complete decoloration, 1-2 g of powdered zinc were added. The reaction mixture was cooled overnight, and the precipitate was filtered, washed with water, and dried in an oven at 100 °C.

Yield: 96 g (85%), white powder; M.P. = 286 °C (Lit. = 285 °C); R_{f}: 0.95 (RP-18, water:MeOH 2:1).

### Synthesis of 2,3,3-trimethylindole-5-sulfonic acid 1a

18.2 g (0.12 mol) of p-hydrazinobenzenesulfonic acid and 14.8 g (0.17 mol) of isopropylmethylketone were stirred in 100 mL of glacial acetic acid at room temperature for 1 h. The mixture was then refluxed for 4 h. The mixture was cooled to room temperature, and the resulting pink solid precipitate was filtered and washed with ether.

The precipitate was dissolved in methanol, and a concentrated solution of potassium hydroxide in 2-propanol was added until a yellow solid completely precipitated. The precipitate was filtered, washed with ether, and dried in a desiccator over P₂O₅.

Yield: 20.4 g (71%), off-white powder; M.P. = 275°C; R_{f}: 0.40 (silica gel, isopropanol:water:ammonia 9:0.5:1).

### 1-(5-carboxypentyl)-2,3,3-trimethyl-3H-5-indoliumsulfonate (1b)

15.9 g (57 mmol) of 2,3,3-trimethylindolenium-5-sulfonic acid potassium salt **1a** and 12.9 g (66 mmol) of 6-bromohexanoic acid were refluxed in 100 mL of 1,2-dichlorobenzene for 15 min under a nitrogen atmosphere. The solution was cooled to room temperature, and the resulting pink precipitate was filtered, washed with chloroform, and dried.

Yield: 15.8 g (58%), pink powder; R_{f}: 0.75 (RP-18, MeOH:water 2:1).

### Synthesis of 1,2,3,3-tetramethylindolium-5-sulfonate (1c)

1.1 g of 2,3,3-trimethylindoleninium-5-sulfonate were suspended in 30 mL of methyl iodide. The reaction mixture was heated to boiling for 25 h in a sealed tube. After the mixture was cooled, excess methyl iodide was decanted, and the residue was suspended in 50 mL of acetone. The solution was filtered, and the residue was dried in a desiccator over CaCl₂. The resulting light yellow powder was used without further purification.

Yield: 90%, light yellow powder.

### Synthesis of 3-(5-carboxypentyl)-2,3-dimethyl-5-sulfo-1-(3-sulfopropyl) indolium sodium salt (1d), (Scheme I)

A mixture of 25 g of ethyl 2-methylacetoacetate (I), 64 ml of 21% sodium ethoxide solution in ethanol and 34 mL of ethyl-6-bromohexanoate is refluxed in 200 mL of ethanol overnight. The mixture is filtered and the solvent is removed under reduced pressure. The residue is partitioned between 1 M HCl and chloroform.

The organic layer is dried over magnesium sulfate and purified on silica gel using 1:10 ethyl acetate/hexane as the eluent to yield 22 g of ethyl 2-(5-carboethoxypentyl)-2-methylactoacetate (**IIa**).

The above compound is dissolved in 300 ml of methanol. A solution of 10 g NaOH in 100 mL water is added. The mixture is heated at 50 C overnight. The solution is reduced to about 50 mL, acidified to pH 1 and extracted with ethyl acetate. The organic phase is collected, dried over MgSO₄ and evaporated to yield 13.5 g of 7-methyl-8-oxononanonic acid (**IIIa**).

The nonanonic acid is refluxed in 110 mL of acetic acid with 13.5g of 4-hydrazinobenzenesulfonic acid for 4 hours. The acetic acid is evaporated and the product is purified on silica gel to yield 23 g of the product (**IVa**).

To the methanol solution of 11g of Compound **IVa** is added 3.4 g of anhydrous sodium acetate. The mixture is stirred for five minutes. The solvent is evaporated and the resulting sodium salt is heated with 24.4 g of propane sultone at 110 C for 1 hour to generate the final product **1d**.

### Synthesis of 3-(6-hydroxyhexyl)-2,3-dimethyl-5-sulfo-1-(3-sulfopropyl) indolium, sodium salt (1e)

Another starting material **1e** is synthesized analogously using ethyl 2-methylacetoacetate and 6-benzoyl-1-bromo-hexane in the presence of 1.2 equivalents of sodium hydride in THF according to **1d.** After isolating the 3-(6-hydroxyhexyl)-2,3-dimethyl-5-sulfo-indolium, inner salt (the hydroxy group is again protected and the compound is quarternized using propanesultone. Deprotection is achieved using dilute NaOH.

**1f** is synthesized analogously taking into account the more polar nature of the sulfonic groups that are introduced either by reaction with 2-bromo-ethane-sulfonic acid, propane- or butanesultone. Sulfogroups can also be introduced by reaction of a 3-carboxy-alkyl-substituted compound like **1d** with taurine according to Terpetschnig et al., Anal. Biochem. 217, 197-204 (1994).

Using 4-hydrazino-benzoic acid as described in Anal. Biochem. 217, 197-204 (1994) or 4-hydrazino-phenyl-acetic acid as described in Cytometry 11(3), 418-30 (1990) and reacting them in a Fischer indole synthesis with 7-methyl-8-oxononanonic acid or one of the other functionalized precursors as described above, 5-carboxy-derivatized indoles that contain a spacer group in position 3 can be synthesized.

Other starting materials that contain functional groups in both R₃ and R₄ can be synthesized accordingly and used as starting materials for cyanine dyes of this invention. R₃ and R₄ can also be a part of an aliphatic ring system as described in U.S. Patent Application Publication No. US2002/0077487.

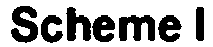

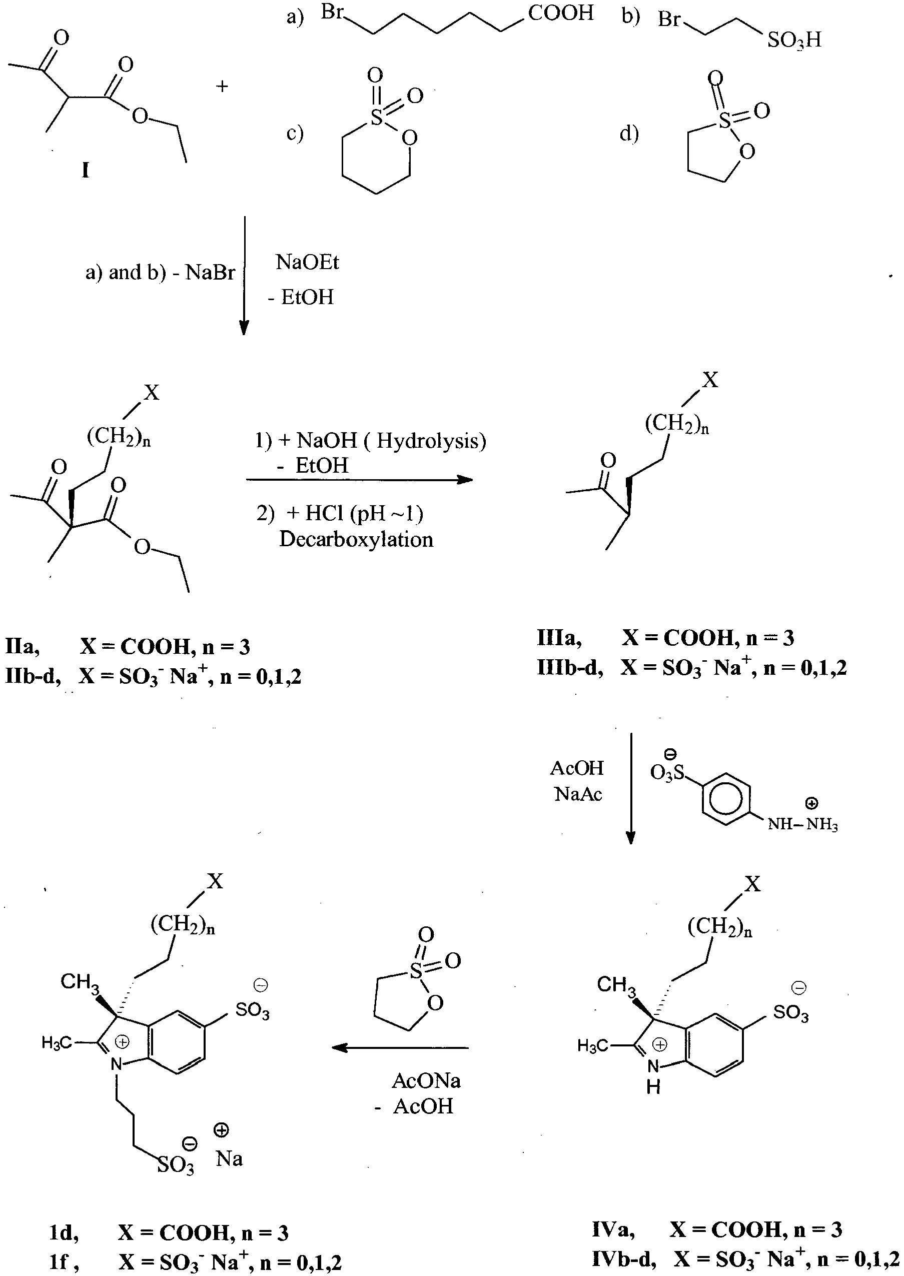

The following is a proposed procedure for the synthesis of the indolenines **1k** and **1l**: **Indolenine (1k)**

### Synthesis of p-nitrobenzaldoxime (according to U.S. Patent No. 4,751,328 A1)

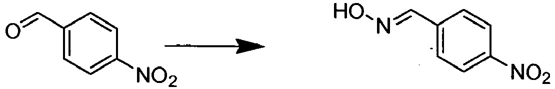

453 g (3 mol) of p-nitrobenzaldehyde was dissolved in 1350 mL of methanol and then, an aqueous solution of 241 g (3.3 mol) of hydrochloric acid hydroxylamine hydrochloride in 300 ml of water was added dropwise over 30 minutes while maintaining the reaction temperature at 30 °C. Thereafter, stirring was continued at the same temperature for two hours and the mixture was diluted with 2000 ml of water. The white crystals that formed were filtered, washed with water and dried. 480 g of p-nitrobenzaldoxime was obtained (yield, 96.5%). M.P.: 128 - 131 °C.

### Synthesis of p-nitrobenzylamine (according to U.S. Patent No. 4,751,328 A1)

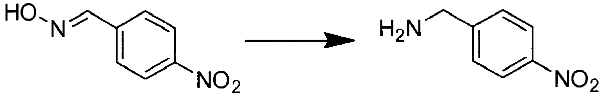

33.2 g (0.2 mol) of p-nitrobenzaldoxime, 18.3 g (0.25 mol) of boric anhydride, 1 g of a 5% Pt-C catalyst and 100 ml of methanol were charged into a hermetically sealed glass container and vigorously stirred while charging hydrogen. Reaction was continued at temperatures of 25 to 30 C for 13.5 hours and 22.2 L of hydrogen was absorbed. Next, this reaction mixture was filtered to remove the catalyst and methanol was distilled off by concentration under reduced pressure. A yellow viscous liquid thus obtained was neutralized by adding 57 g (0.5 mol) of a 35% aqueous solution of sodium hydroxide and then, the liquid was separated to two layers. The lower layer, a colorless and transparent aqueous solution of sodium borate was removed and then, a brown and oily crude p-aminobenzylamine was obtained. This crude p-nitrobenzylamine was subject to vacuum distillation at pressure of 5 to 6 mmHg and 22.5 g of fractions between 129.5 and 130 °C was obtained (yield: 92%).

### Synthesis of 3-[4-nitro(3-sulfonatopropyl)anilino]-1-propanesulfonate

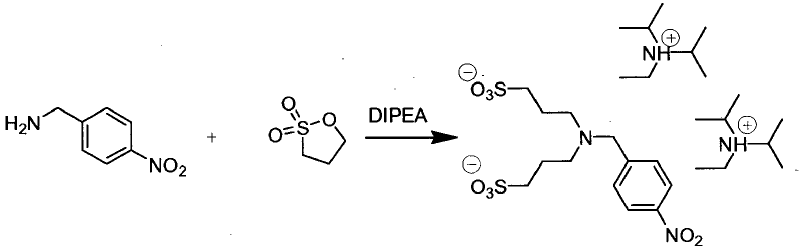

The reaction should follow analogously as described for aniline (below).

### Synthesis of 3-[4-amino(3-sulfonatopropyl)anilino]-1-propanesulfonate

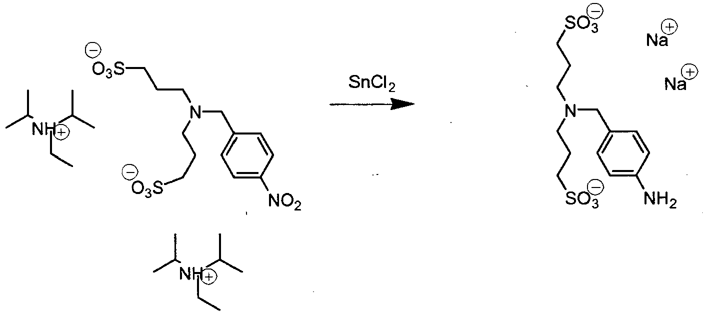

A solution of SnCl₂ (80 mmol) in 20 ml HCl is added dropwise to a boiling solution of di[ethyl(diisopropyl)ammonium] 3-[4-nitro(3-sulfonatopropyl)anilino]-1-propane sulfonate (10 mmol) in ethanol (50 mL). Then mixture is refluxed for 1 h and the solvent was evaporated. Then the residue is dissolved and stirred for 30 minutes in a NaOH solution to destroy the Sn-salt and the pH adjusted to pH 3 and the product is extracted with methylene chloride. The obtained organic extracts are dried with Na₂SO₄ and the solvent is removed under reduced pressure to yield the di-substituted aniline.

The indolenine is either synthesized via the Bischler reaction (Bischler et al.; Chem. Ber. 1892, 25, 2860) or via the Fischer Indole synthesis.

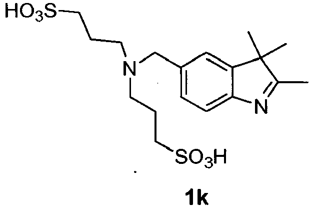

### Synthesis of the indolenine 1l

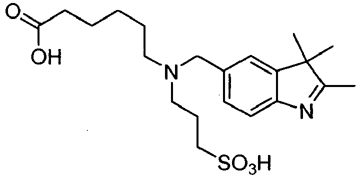

Indolenine **1l** is synthesized analogously to **1k** but using brono-hexanoic acid ethylester as reagent in the 2^{nd} alkylation reaction of the nitro-aniline (see Example 9).

Selected indolenine-type precursors are summarized in the table below:

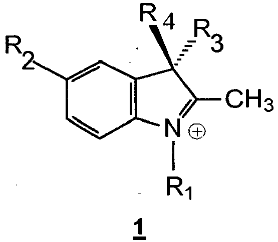

| 1 | R₁ | R₂ | R₃ (x = 2,3,4) | R₄ | (Patent) Literature |
|---|---|---|---|---|---|
| a | none | SO₃⁻ | CH₃ | CH₃ | US2005/0202565 |
| b | (CH₂)₅COOH | SO₃K | CH₃ | CH₃ | US2004/0166515 |
| c | CH₃ | SO₃⁻ | CH₃ | CH₃ | Bioconj. Chem. 4(2), 105, 1993 |
| d | (CH₂) ₃SO₃Na | SO₃⁻ | (CH₂)₅COOH | CH₃ | US2002/0077487 |
| e | (CH₂)₃SO₃Na | SO₃⁻ | (CH₂)₆OH | CH₃ | US2002/0077487 |
| f | none or (CH₂)₃SO₃⁻ | SO₃⁻ | (CH₂)₄SO₃Na | CH₃ | WO 2004/039894 |
| g | none or (CH₂)₄SO₃⁻ | COOH | CH₃ | CH₃ | Anal. Biochem. 217, 197-204 (1994**)** |
| h | (CH₂)₃SO₃Na | SO₃⁻ | CH₃ | CH₃ | US 6,140,494 |
| i | (CH₂)₂PO(OH)₂ | SO₃⁻ | CH₃ | CH₃ | WO 2001/36973 |
| j | none | CH₂-COOH | CH₃ | CH₃ | US 4,981,977 |

### Synthesis of aniline precursors

Aniline-type squaraines and precursors are described in U.S. Patent Nos. 5,101,015, 5,039,818, 5,416,214, 4,830,786 and 6,417,402 and U.S. Patent Application Publication No. US2002/0147354, and in references listed therein. Additional precursors and dyes are described in the Examples below. The synthesis of unsymmetrical squaraine dyes is described in S. Yagi et al., J. Chem. Soc., Perkin Trans. 1, 2000, 599-603.

### Synthesis of a disulfonated aniline-derivative

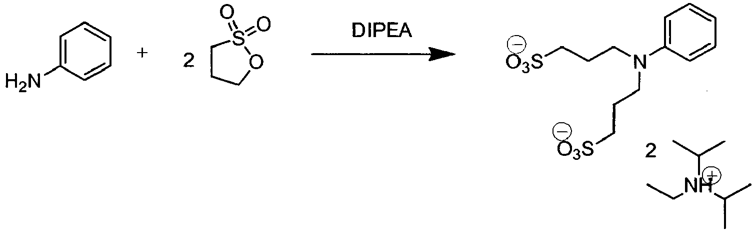

A mixture of aniline (7 mmol), N,N-diisopropylethylamine (DIPEA, 18 mmol) and propane sultone (16 mmol) was heated in a pressure tube in 10 mL of acetonitrile at 120-130 °C for 9 h under argon atmosphere. The solvent was evaporated and ethylacetate was added. The precipitated DIPEA bromide was filtered off and the filtrate was evaporated. The residue was treated with ether to give di[ethyl(diisopropyl)ammonium] 3-(3-sulfonatopropylanilino)-1-propanesulfonate as a white or pinky-white solid. Yield: 94%.

### New Aniline-precursors

Stating materials for the synthesis of novel aniline-type squaraine-rotaxanes dyes that are claimed in this application are described here. It should be also noted that the procedures for the synthesis of squaraines including the subsequent conversion to Leigh-type aminde-rotaxanes follows a standard protocol that sometimes require adjustments in regards to the solvent, temperature and/or the reaction time. Several of these procedures are described in the Experimental part below or in the cited literature references.

### Synthesis of sulfonated benzylaniline-intermediates

The synthesis of 4-(bromomethyl)benzenesulfonic acid for novel polysulfonated aniline-based rotaxanes from p-toluenesulfonyl chloride is achieved according to a procedure described by Yee et al., J. Med. Chem. 1990, 33, 2437-2451 and L. Blangey, et al., Helv. Chim. Acta, 1942, 25, 1162. Di-benzylated anilines are synthesized according to literature procedures (see above).

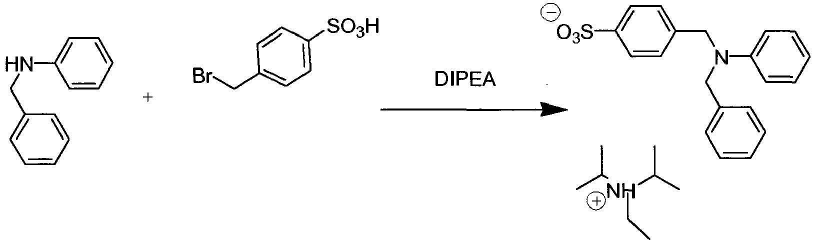

A mixture of the N-benzyl-aniline (1.0 mmol), 4-(bromomethyl)benzenesulfonic acid (1.2 mmol) and N,N-diisopropylethylamine (DIPEA, 2.2 mmol) was heated in 10 mL of acetonitrile at 120-130 °C for 9 h in a pressure tube under argon atmosphere. The solvent was removed under reduced pressure and ethyl acetate was added. The precipitated DIPEA bromide was filtered and the filtrate was evaporated. After drying, the raw product was used without further purification.

### Synthesis of thiophene-intermediates

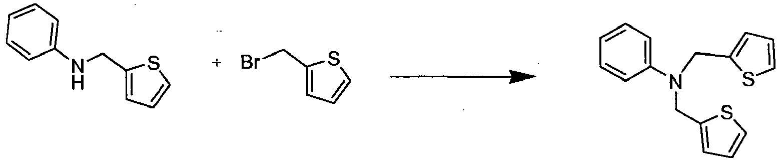

A mixture of aniline-N-methylthiophene (10 mmol), (synthesized 2-formyl-thiophene and aniline), 2-bromomethylthiophene (12 mmol) and N,N-diisopropylethylamine (DIPEA, 22 mmol) was heated in 10 mL of acetonitrile at 120-130 °C for 9 h in a pressure tube under argon atmosphere. The solvent was removed under reduced pressure and ethyl acetate was added. The precipitated DIPEA bromide was filtered and the filtrate was evaporated. After drying, the raw product was purified by flash chromatography on silica gel, eluent: hexane/triethylamine.

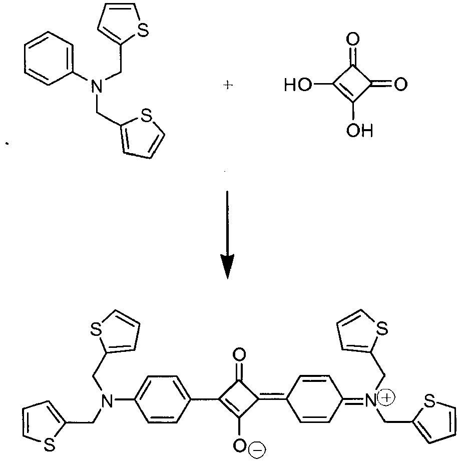

A mixture of 2-(2-thienylmethylanilinomethyl)thiophene (1.83 mmol) and squaric acid (0.92 mmol) was refluxed in a mixture of n-butanol (15 mL) and toluene (30 mL) for 12 h. After cooling, the solvent was removed on a rotary evaporator, and the residue was purified by column chromatography using silica gel (gradient 0-2% MeOH in CHCl3) to yield the relevant squaraine dye.

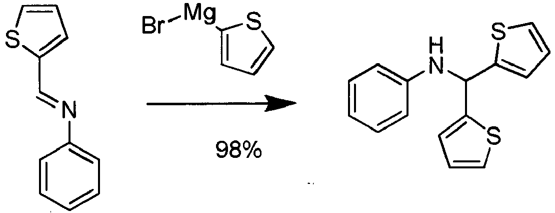

The above reaction is described in J. Org. Chem. 1989, 54, 6120-6123. 2-thienylmagnesium bromide can be replaced by any heterocycle-magnesium bromide.

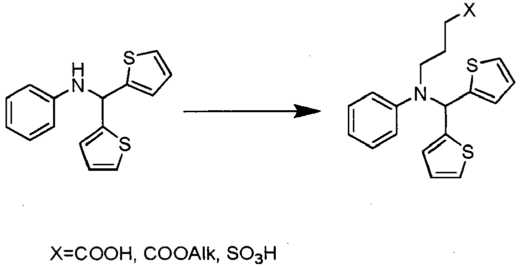

The alkylation reaction above should proceed according to the alkylation of a secondary amine that is described below.

### Synthesis of reactive thiophene-intermediates

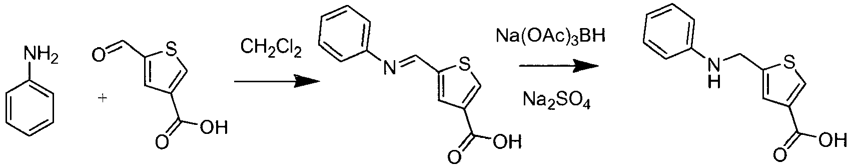

To a solution of aniline (0.24 mmol) in 1,2-dichloroethane (4 mL) were sequentially added 5-formyl-3-thiophenecarboxylic acid (0.045 g, 0.29 mmol) and sodium sulfate (150 mgs) under a nitrogen atmosphere at room temperature as described in J. Med. Chem. 49 (24), 6946 (2006). The contents were stirred at room temperature for twenty hours and sodium triacetoxyborohydride (0.075 g, 0.336 mmol) was added. The reaction was allowed to continue at room temperature for six hours and quenched by the addition of water (15 mL). Dichloromethane (15 mL) was added, and sulfurdioxide gas was bubbled through the reaction mixture for ten minutes and the contents transferred into a separating funnel. The organic layer was separated, washed with brine (2 x 20 mL), dried over sodium sulfate, concentrated under reduced pressure and purified by silica gel column chromatography using dichloromethane and MeOH to yield the title compound as a solid.

### Synthesis of novel N-disubsituted anilines

A mixture of aniline-N-methy-thiophene (10 mmol), 1,3-propansultone or 4-(2-bromoethyl)-1-benzenesulfonic acid (12 mmol) and N,N-diisopropylethylamine (DIPEA, 12 mmol) was heated in 10 -15 mL of acetonitrile in a pressure tube at 120-130 °C for 9 h under argon atmosphere. The solvent was removed under reduced pressure and crude product was purified by flash chromatography using silica gel.

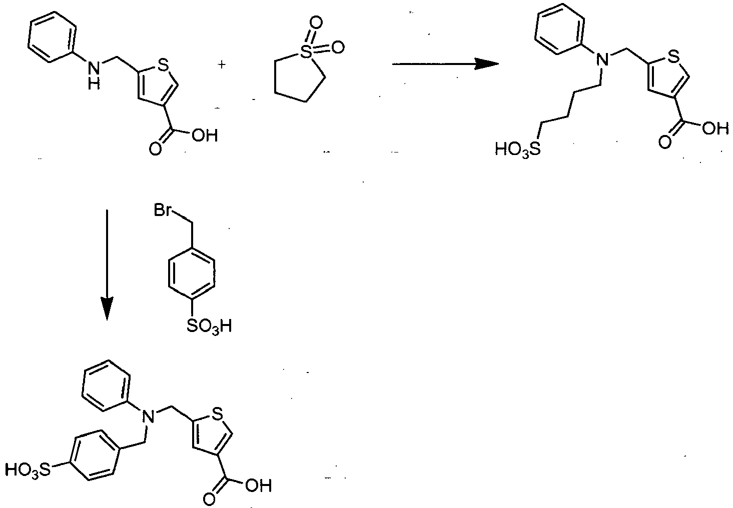

### Synthesis of anilino-pyridine precursors

The synthesis of 3-anilinomethylpyridine precursors should proceed similar to the synthesis of N-benzylaniline described in Example 7 except with nicotin-aldehyde as the reagent:

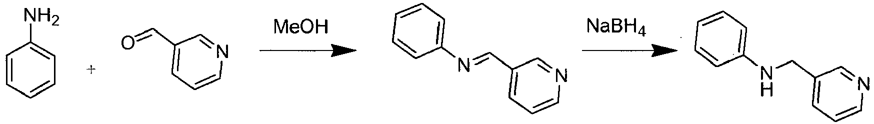

### Synthesis of a reactive anilino-pvridine precursor

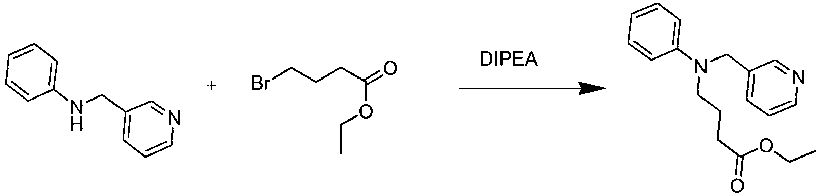

A mixture of 3-anilinomethylpyridine (12 mmol), ethyl 4-bromobutanoate (14.4 mmol) and N,N-diisopropylethylamine (DIPEA, 26 mmol) is heated in 15 mL of acetonitrile at 120-130 °C for 9 h in a pressure tube under argon atmosphere. The solvent was removed under reduced pressure and ethyl acetate was added. The precipitated DIPEA bromide was filtered and the filtrate was evaporated. After drying, the raw product was purified by flash chromatography on silica gel.

### Julolidine-based Squaraines

Julolidine is commercially available from Alrdrich. A dihyrdoxy-substituted julolidine, 2,3,6,7-tetrahydro-1H,5H-benzo<ij>quinolizine-2,6-diol, was described Silhankova et al., Collect. Czech. Chem. Commun. 50(5) 1048-1056 (1985).

### Thiophene-type Squaraines

The synthesis of thiophene- and thiazole-heterocycle-containing squaraine dyes were described by D. Keil and H. Hartmann in Liebigs Ann. 1995, 979-984.

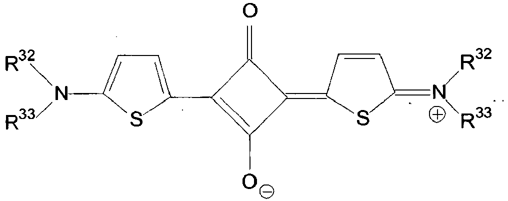

### Squaric acid precursors

Compounds **2a** and **2b** are commercially available. 1,3-Dithiosquaric acid disodium salt (**2c**) and triethylammonium 2-butoxy-3-dicyanomethylene-4-oxo-1-cyclobuten-1-olate (**2d**) were synthesized according to Seitz et al., Chem. Ber. 112, 990-999, (1979) and Gerecht et al., Chem. Ber. 117, 2714-2729 (1984), respectively.

The 3-cyanoimino-4-oxo-1-cyclobutene-1,2-diolate (**2e**) is synthesized starting from dibutylsquarate according to the procedure of K. Köhler et al. Chem. Ber. 118, 1903-1916 (1985).

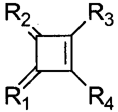

| 2 | R₁ | R₂ | R₃ | R₄ |
|---|---|---|---|---|
| a | O | O | OH | OH |
| b | O | O | OC₄H₉ | OC₄H₉ |
| c | S | O | S⁻ Na⁺ | O⁻ Na⁺ |
| d | O | C(CN)₂ | OC₄H₉ | O⁻ HNEt₃⁺ |
| e | N-CN | O | O⁻ K⁺ | O⁻ K⁺ |

### Synthesis of intermediates for ring-substituted squaraines

The syntheses of these intermediates are described in Tatarets et al., Analytica Chimica Acta 570, 214-223 (2006), Tatarets et al., Dyes & Pigments 64, 125-134 (2005) and in PCT Application No. PCT/US2003/010995.

### Synthesis of precursors for Leigh-type amide rotaxanes

### Synthesis of 1,4-Bis(aminomethyl)-2,5-dimethoxybenzene

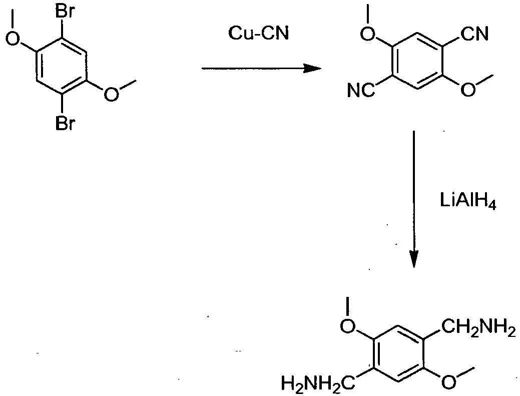

The starting material 1,4-dibromo-2,5-dimethoxybenzene is commercially available from Aldrich: catalog number: 461105. The dicyano compound was synthesized from this starting material by reacting it with CuCN according to Neidlein et al., Chem. Ber. 119(3), 844-849 (1986), in N-methylpyrrolidone at 190 °C and the 1,4-bis(aminomethyl)-2,5-dimethoxybenzene (CAS registry number: 2745-67-7) is synthesized via reduction with LiAlH₄ in ether, analogously to Sasaki et al., J. Org. Chem. 65 (2); 2000; 275-283.

### Synthesis of modified rotaxane ring-structures

A possible route to a more hydrophilic rotaxane macrocycles that contains hydroxy-methyl groups is described by Fieser et al. in Am. Soc. 68 (1946), 2249.

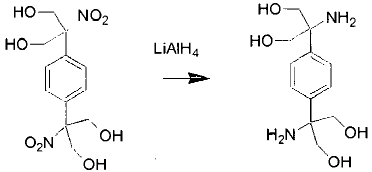

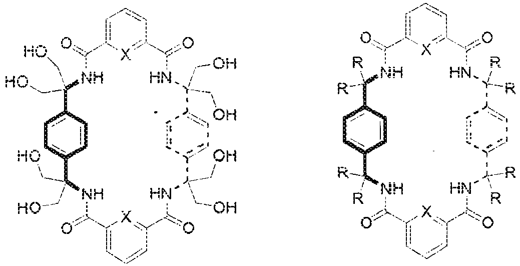

### Synthesis of ring-substituted 2,6- pyridine-dicarboxylic acid dichlorides

These starting materials are described in the following references: The synthesis of 4-methoxy-pyridine-2,6-dicarbonyl dichloride was described by Haldar et al., Tetrahedron 63 (27), 6322-6330 (2007). The synthesis of 4-(3-cyano-propoxy)-pyridine-2,6-dicarbonyl dichloride by Chaubet et al., Tetrahedron Lett. 31(40), 5729-5732 (1990). 4-hydroxypyridine-2,6-dicarbonyl dichloride was described by Barsu et al., Chem. Commun. 45, 4744-4746 (2006). The synthesis of 4-chloro-, 4-methoxy- and 4-nitro-pyridine-2,6-dicarbonyl dichloride was described by Bradshaw et al., J. Am. Chem. Soc.102 (2) 467-474, 1980. It is understood that the nitro-group can be reduced to an amino-group and the amino-group can subsequently be protected with a group that is easily cleaved after rotaxane formation. A large number of protecting groups form various amino-groups are available from the literature. The amino-group can also be converted into an isothiocyante, which reacts readily with amines. These are just a few examples of ring-substituted starting materials that can be utilized as precursors to synthesize reactive and hydrophilic rotaxane structures.

### EXAMPLE 2

### Synthesis of the hydrophobic squaraine-rotaxane 3:

Squaraine dye **3a** is synthesized analogously to procedures that are described in U.S. Patent Application Publication No. US2005/0202565. **3a**: λₘₐₓ (abs) 647 nm (DMF/water = 1:1), λₘₐₓ (em) 666 nm (DMF/water = 1:1), Q.Y. 29% (DMF/water = 1:1), λₘₐₓ (abs) 645 nm (DMF/water = 1:2), λₘₐₓ (em) 664 nm (DMF/water = 1:2).

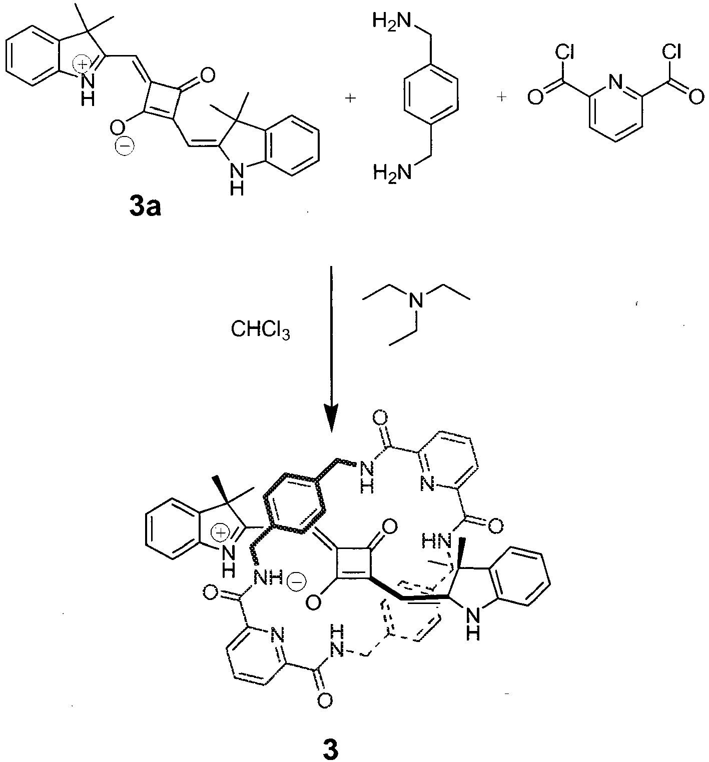

Clear solutions of 2,6-pyridinedicarbonyl dichloride (1.28 mmol) in 5 mL of chloroform and p-xylylenediamine (1.28 mmol) in 5 mL of chloroform were simultaneously added dropwise over 5 h to a stirred solution of **3a** (0.32 mmol) and triethylamine (3.2 mmol) in 40 mL of CHCl₃. After overnight stirring, the mixture containing rotaxane **3** and polymer co-products was filtered, washed with chloroform (100 mL) to extract rotaxane **3**, and the filtrate was concentrated. Purification of the crude product by column chromatography using silica gel (gradient 0-1.5% of MeOH in CH₂Cl₂) gave rotaxane **3**. λₘₐₓ (abs): 642 nm (DMF/water = 1:2), λₘₐₓ (em): 659 nm (DMF/water = 1:2); Q.Y. : 35% (DMF/water = 1:2); ¹H-NMR δ_{H} (200 MHz, CDCl₃): 11.94 (s, 2H), 9.69 (d, J = 9.7 Hz, 4H), 8.59 (d, J = 7.5 Hz, 4H), 8.22 (t, J = 7.5 Hz, 2H), 7.35-7.05 (m, 8H), 6.88 (s, 8H), 5.45 (d, J = 10.3 Hz, 2H), 5.38 (d, J = 10.3 Hz, 2H), 4.78 (s, 2H), 3.78 (d, J = 14.5 Hz, 4H), 1.58 (s, 12H).

### EXAMPLE 3

### Synthesis of squaraine-rotaxane 4:

Squaraine dye **4a** was synthesized as described in U.S. Patent Application Publication No. US2005/0202565. **4a:** λₘₐₓ (abs) = 638 nm, λₘₐₓ (em) = 654 nm, Q.Y.: 25% (water). ₁= 1.55 ns (73%), ₂= 8.17 (27%), ₘₑₐₙ = 3.34 ns (water).

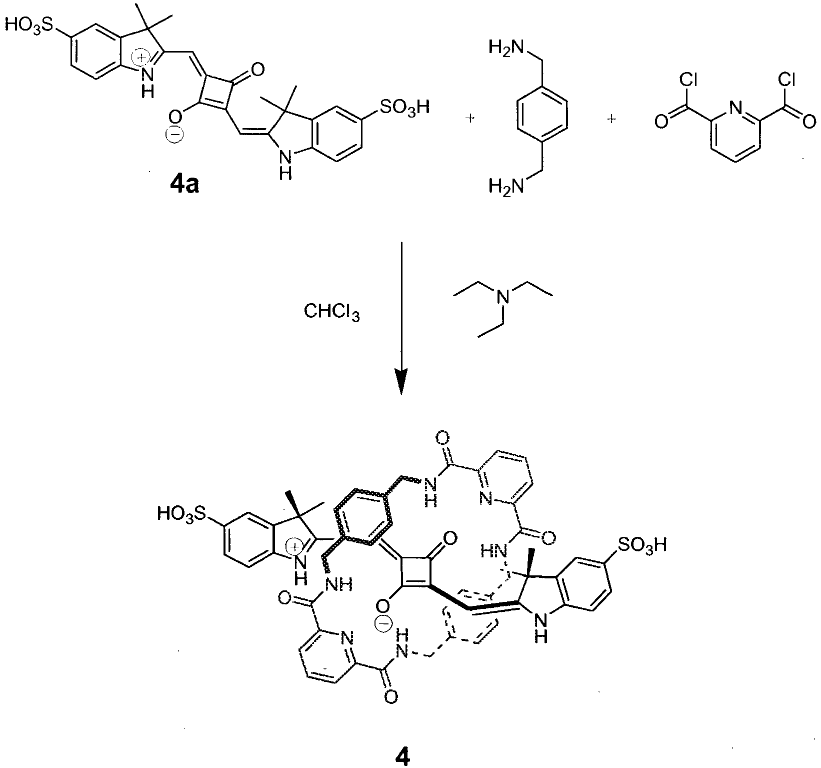

Clear solutions of 2,6-pyridinedicarbonyl dichloride (1.28 mmol) in 5 mL of chloroform and p-xylylenediamine (1.28 mmol) in 5 mL of chloroform were simultaneously added dropwise over a period of 5 h to a stirred solution of **4a** (0.32 mmol) and triethylamine (3.2 mmol) in 40 mL of CHCl₃ and 1.5 ml of methanol. After overnight stirring, the mixture containing rotaxane **4** and polymer co-products was filtered, washed with chloroform (100 mL) to extract rotaxane **4**, and the filtrate was concentrated. The crude product was column purified (RP-18, MeOH/water, gradient) to give rotaxane **4**. λₘₐₓ (abs) = 640 nm (water), λₘₐₓ (em) = 656 nm (water), Q.Y.: 40% (water), ₁ = 2.3 ns (65%), ₂ = 8.45 (35%), ₘₑₐₙ = 4.4 ns (water).

### EXAMPLE 4

### Synthesis of squaraine-rotaxane 5:

Squaraine dye **5a** was synthesized analogously as described in Terpetschnig et al., Dyes & Pigm. 21 (3), 227-234 (1993). λₘₐₓ(abs) = 681 nm, λₘₐₓ(em) = 697 nm, Q.Y.: 58% (chloroform). ₁ = 1.58 ns (65%), ₂ = 8.06 (35%), ₘₑₐₙ = 3.85 ns (chloroform).

Clear solutions of 2,6-pyridinedicarbonyl dichloride (1.28 mmol) in 5 mL of chloroform and p-xylylenediamine (1.28 mmol) in 5 mL of chloroform were simultaneously added dropwise over 5 h to a stirred solution of **5a** (0.32 mmol) and triethylamine (3.2 mmol) in 40 mL of CHCl₃ and 1.5 mL of methanol. After overnight stirring, the mixture containing rotaxane **5** and polymer co-products was filtered, washed with chloroform (100 mL) to extract rotaxane **5**, and the filtrate was concentrated. The crude product was column purified (RP-18, 0-1.5% MeOH in CHCl₃, gradient) to give rotaxane **5**. λₘₐₓ (abs) = 676 nm, λₘₐₓ (em) = 690 nm, Q.Y. 59% (CHCl₃); = 5.35 ns (100%), (CHCl₃).

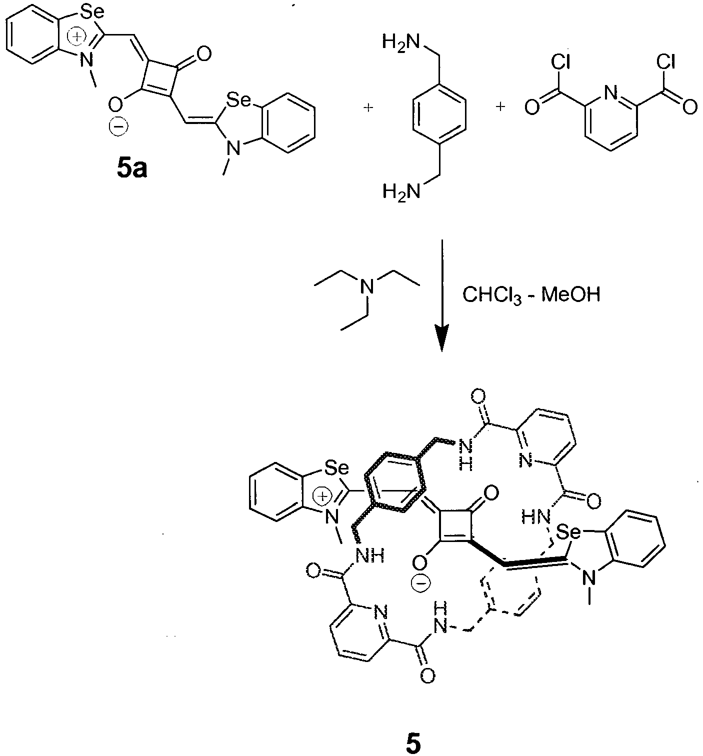

### EXAMPLE 5

### Synthesis of rotaxane 6:

Squaraine dye **6a** was synthesized analogously as described in Terpetschnig et al., Dyes & Pigm. 21 (3), 227-234 (1993). λₘₐₓ(abs) = 681 nm, λₘₐₓ(em) = 698 nm, Q.Y.: 57% (chloroform).

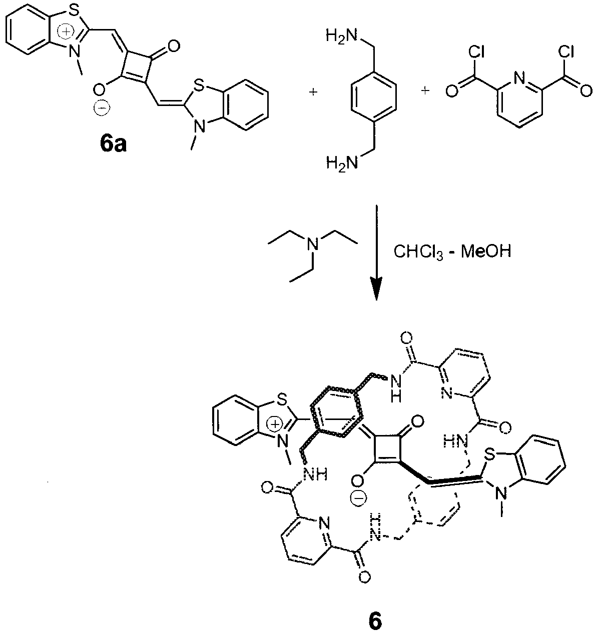

Clear solutions of 2,6-pyridinedicarbonyl dichloride (1.28 mmol) in 5 mL of chloroform and p-xylylenediamine (1.28 mmol) in 5 mL of chloroform were simultaneously added dropwise over 5 h to a stirred solution of squaraine dye 6a (0.32 mmol) and triethylamine (3.2 mmol) in 40 mL of CHCl₃ and 1.5 mL of methanol. After stirring overnight, the mixture containing rotaxane 6 and polymer co-products was filtered, washed with chloroform (100 mL) to extract rotaxane 6, and the filtrate was concentrated. Purification of the crude product by column chromatography using Silica Gel (gradient 0-1.5% MeOH in CHCl₃) gave rotaxane 6. 6: λₘₐₓ (abs) = 661 nm, λₘₐₓ (em) = 674 nm, Q.Y.: 66% (CHCl₃).

### EXAMPLE 6

### Synthesis of squaraine-rotaxane dye 7

### Squaraine dye 7a

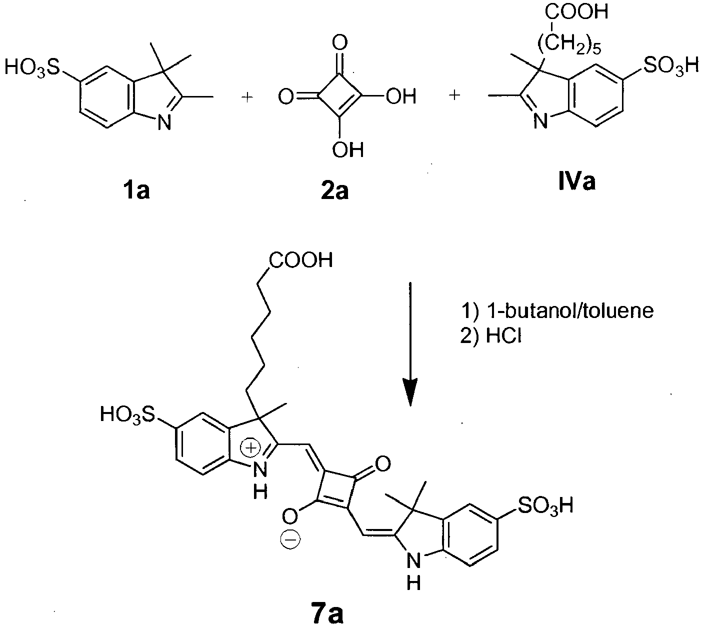

A mixture of 350 mg (1.47 mmol) of 2,3,3-trimethyl-3*H*-5-indolesulfonic acid (**1a**), 500 mg (1.47 mmol) of 6-(2,3-dimethyl-5-sulfo-3*H*-3-indolyl)hexanoic acid (**IVa**), and 220 mg (1.92 mmol) of 3,4-dihydroxy-3-cyclobutene-1,2-dione **2a** was heated at reflux in a mixture of 30 mL of 1-butanol and 10 mL of toluene for 15 h with a Dean-Stark trap. The solvent was removed under reduced pressure and the obtained residue was refluxed with 70 mL of 0.2 M HCl for an hour and then water was removed under reduced pressure. Crude product was twice purified by a column chromatography (Silica gel 60 RP-18, 0-5% methanol-water) to give 65 mg (6.7%) of **7a**; δ_{H} (200 MHz, DMSO-d₆) 12.78 (2H, broad s, N*H*), 7.66 (1 H, s, arom H), 7.61 (1 H, s, arom H), 7.56 (2H, dd, 8.5, 1.2 Hz, arom H), 7.16 (2H, d, 8.1 Hz, arom H), 5.58 (1H, s, C*H*), 5.54 (1H, s, C*H*), 2.07 (2H, t, 6.9 Hz, C*H*₂COOH), 2.02-1.74 (2H, m, C*H*₂), 1.44 (6H, s, (C*H*₃)₂), 1.42 (3H, s, (C*H*₃)₂), 1.37-1.05 (4H, m, CH₂), 1.00-0.45 (2H, m, CH₂). λₘₐₓ (abs): 638 nm, λₘₐₓ (em): 655 nm, (PBS); Q.Y.: 31% (PBS).

### Squaraine-rotaxane 7

Solutions of 2,6-pyridinedicarbonyl dichloride (1.28 mmol) in 5 mL of chloroform and *p-*xylylenediamine (1.28 mmol) in 5 mL of chloroform were simultaneously added dropwise over a period of 5 h to a stirred solution of **7a** (0.32 mmol) and triethylamine (3.2 mmol) in 40 mL of CHCl₃ and 1.5 mL of methanol. After stirring overnight, the mixture containing rotaxane **7** and polymer co-products was filtered, washed with methanol/water 1:1 (100 mL) to extract rotaxane **7**, and the filtrate was concentrated. The crude product was column purified (RP-18, MeOH/water, gradient) to give rotaxane **7**. λₘₐₓ(abs): 642 nm (water); λₘₐₓ(em): 658 nm.

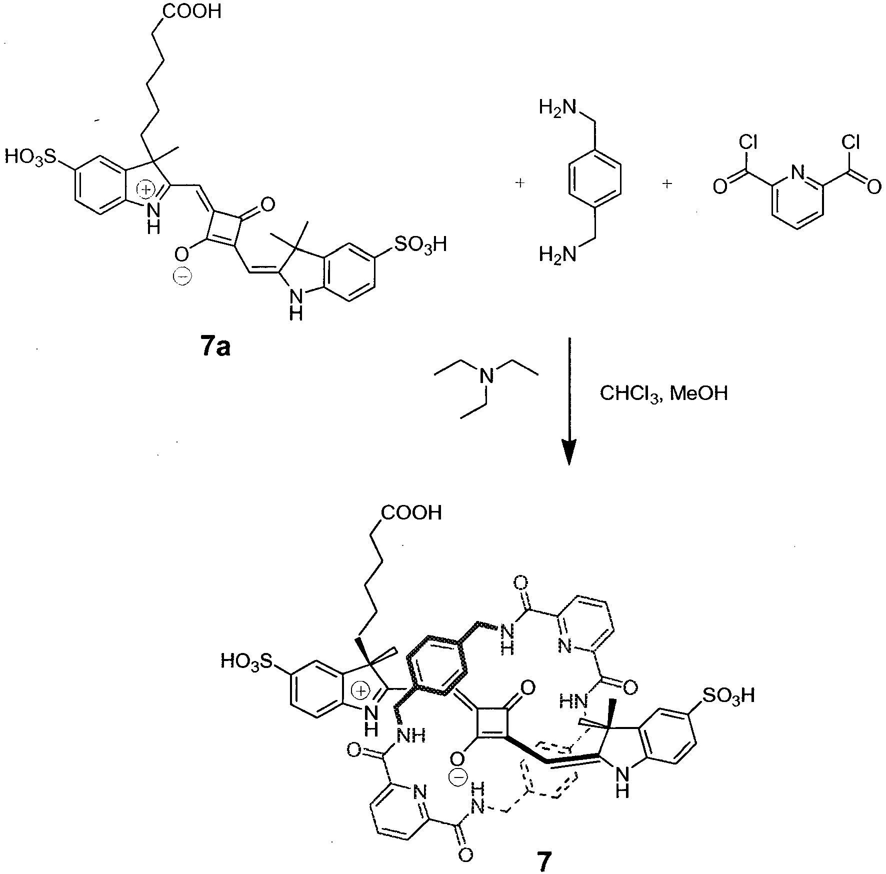

### Synthesis of NHS-ester 7b with TSTU

25.0 µmol of compound **7** (32.22 µmol) TSTU, and 6.5 µL (37.32 µmol) of DIPEA were dissolved in 3 mL of DMF. The solution was stirred at room temperature for 3 h. The reaction was monitored by TLC (RP-18, acetonitrile-water). After completion, the reaction mixture was sealed under argon and stored in the refrigerator until further use. To isolate the solid NHS ester, the solvent was removed under reduced pressure and the residue washed several times with ether.

### EXAMPLE 7

### Synthesis of squaraine-rotaxane 8:

### a) Synthesis of N-diphenylmethanimine

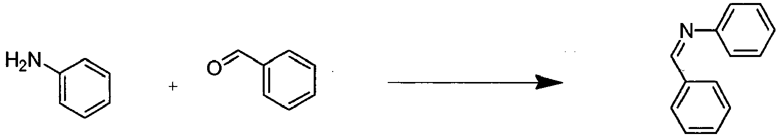

Aniline (53.7 mmol) was added under stirring to benzaldehyde**2** (53.7 mmol), stirred for 15 min and treated with 8 mL of 96% ethanol. After the crystalline N-diphenylmethanimine is formed, it was filtered and recrystallized from 10 mL of 85% aq. ethanol to give N-diphenylmethanimine with 70% yield.

### b) Synthesis of N-benzyl-aniline

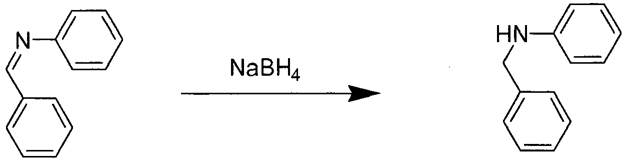

66.21 mmol of sodium borhydride were added over 3 h at room temperature to a solution of N-diphenylmethanimine (16.55 mmol) in 50 mL of methanol. The mixture was stirred for 3 h, diluted with 200 mL of water and the crude N-benzyl-aniline was extracted with methylene chloride. The product was recrystallized from hexane to give N-benzyl-aniline with 84% yield. M.P.: 38 °C.

### c) Synthesis of 6-Benzylanilinohexanoic acid

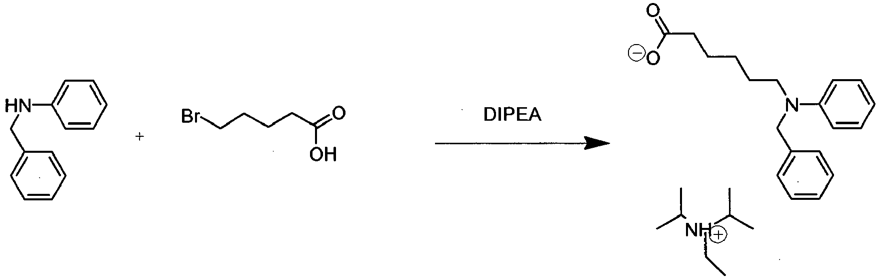

A mixture of N-benzylaniline (10 mmol), 6-bromohexanoic acid 5 (12 mmol) and N,N-diisopropylethylamine (DIPEA, 22 mmol) was heated in 10 mL acetonitrile at 120-130 °C for 6 h in a pressure tube under argon atmosphere. The solvent was evaporated and ether was added. The precipitated DIPEA bromide was filtered and the filtrate was evaporated. The isolated 6-benzylanilinohexanoic acid was dried under vacuum and used for further synthesis without purification.

### d) Synthesis of squaraine dye 8a

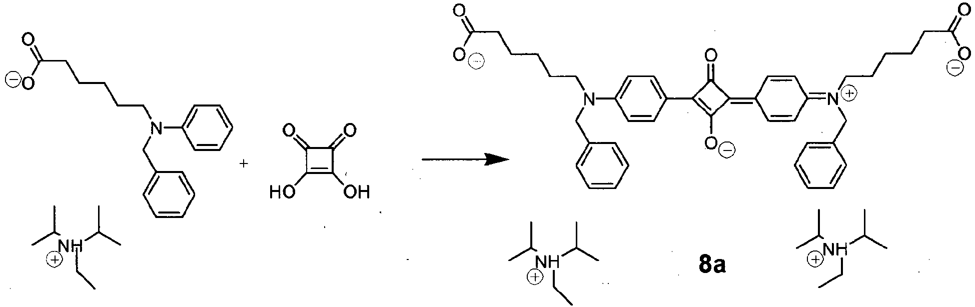

A mixture of the anilinohexanoic acid (1.83 mmol) and squaric acid ( 0.92 mmol) was refluxed in a mixture of n-butanol (15 mL) and toluene (30 mL) for 12 h. The deep green reaction mixture was concentrated and the crude product was precipitated with the addition of 30-40 mL ether. After filtering, the product was washed several times with ether to yield the dark green squaraine dye as a solid. Purification of the crude product by column chromatography using silica gel (gradient 0-1.5% MeOH in CHCl3) gave squaraine **8a**. Yield: 20%. λₘₐₓ (abs): 634 nm (CHCl₃), λₘₐₓ (em): 652 nm (CHCl₃).

### e) Synthesis of squaraine-rotaxane 8

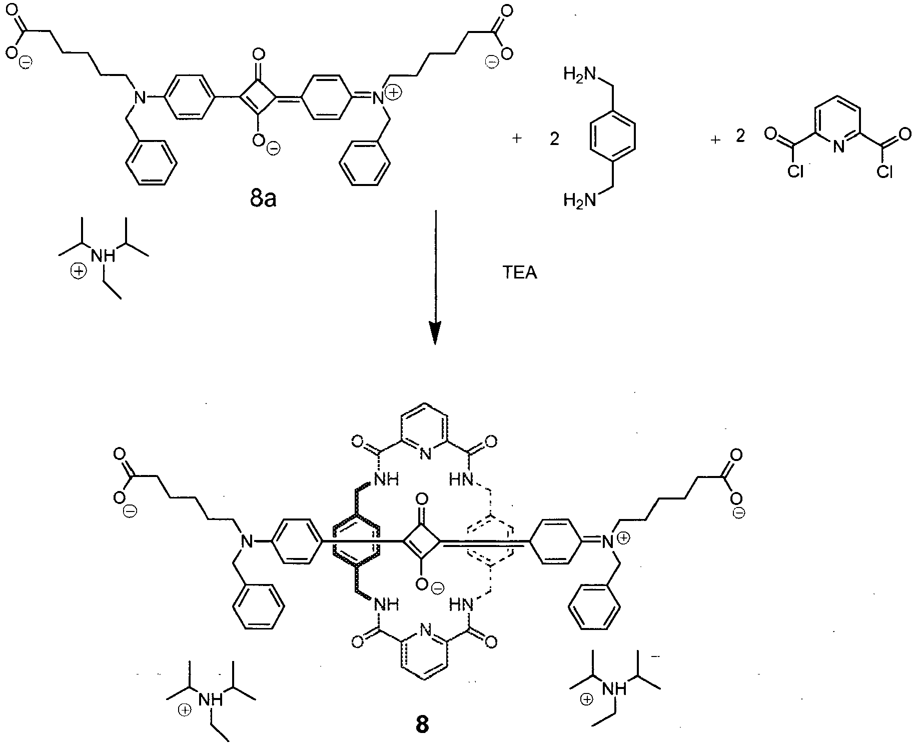

Clear solutions of 2,6-pyridinedicarbonyl dichloride 10 (1.28 mmol) in 5 mL of chloroform and p-xylylenediamine (1.28 mmol) in 5 mL of chloroform were simultaneously added dropwise over 5 h to a stirred solution of squaraine **8a** (0.32 mmol) and triethylamine (3.2 mmol) in 40 mL of CHCl₃. After overnight stirring, the mixture containing rotaxane **8** and polymer co-products was filtered, washed with chloroform (100 mL) to extract rotaxane **8**, and the filtrate was concentrated. Purification of the crude material by column chromatography using silica gel (gradient, 0-1.5% of MeOH in CHCl₃) gave the rotaxane **8**. **8**: λₘₐₓ (abs): 645 nm (CHCl₃), λₘₐₓ (em): 660 nm (CHCl₃); Q.Y.: 70% (CHCl₃).

### EXAMPLE 8

### Synthesis of rotaxane 9:

### a) Synthesis of ethyl 4-(3-ethyloxycarbonylpropylanilino)butanoate

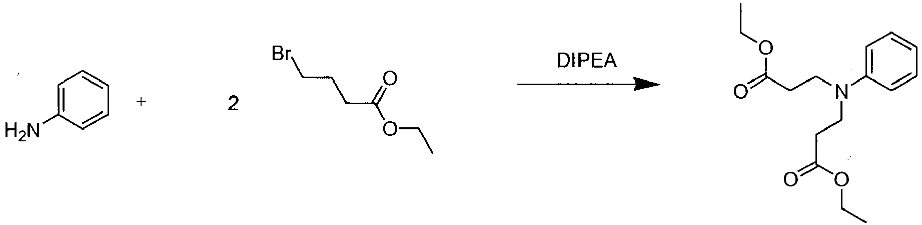

A mixture of aniline (7 mmol), N,N-diisopropylethylamine (DIPEA, 18 mmol) and ethyl 4-bromobutanoate (16 mmol) was heated in 10 mL acetonitrile at 120-130 °C for 9 h in a pressure tube under argon atmosphere. The solvent was evaporated and ethylacetate was added. The precipitated DIPEA bromide was filtered off and the filtrate was evaporated. The residue of ethyl 4-(3-ethyloxycarbonylpropylanilino)butanoate was dried in a vacuum-desiccator and used for further synthesis without purification.

### b) Synthesis of squaraine dye 9a

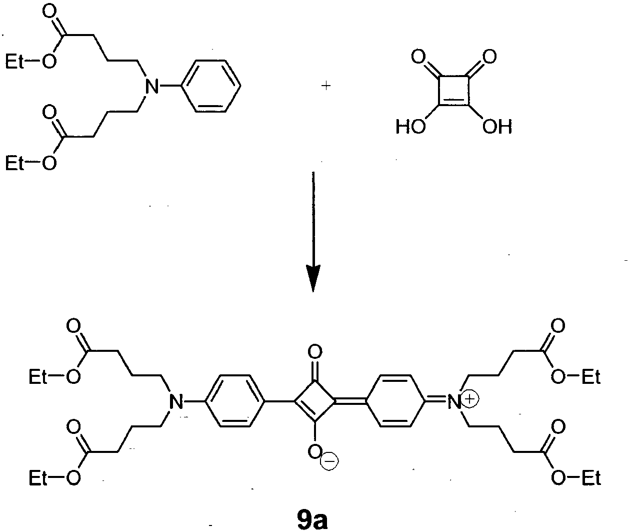

A mixture of ethyl 4-(3-ethyloxycarbonylpropylanilino)butanoate (1.83 mmol) and squaric acid (0.92 mmol) was refluxed in a mixture of n-butanol (15 mL) and toluene (30 mL) for 12 h. After cooling, the solvent was removed on a rotary evaporator, and the residue was purified by column chromatography using silica gel (gradient 0-2% MeOH in CHCl₃) to give squaraine **9a**. **9a:** λₘₐₓ (abs): 633 nm (CHCl₃).

### c) Synthesis of squaraine-rotaxane dye 9b

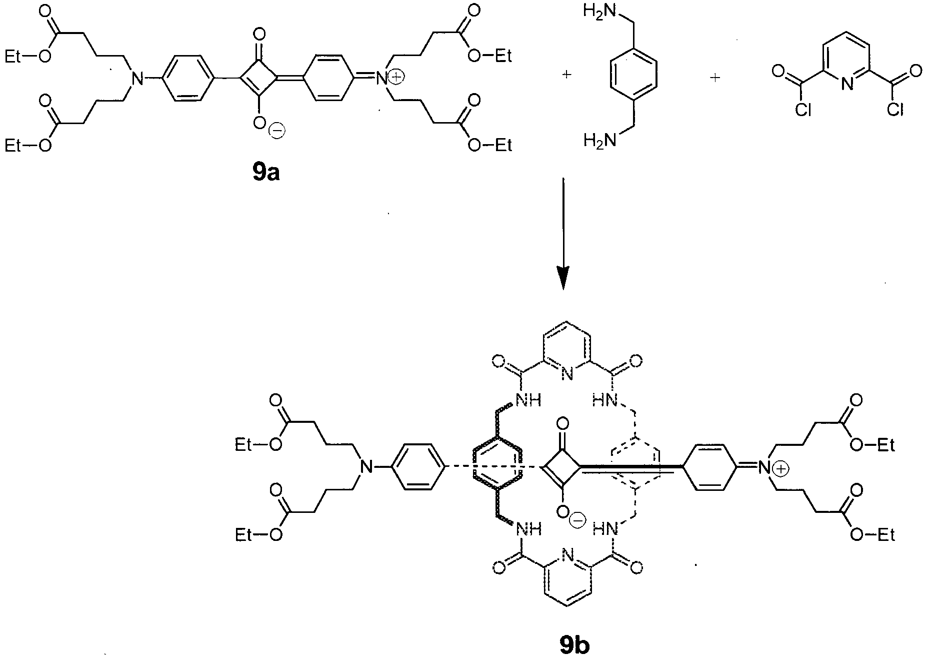

Clear solutions of 2,6-pyridinedicarbonyl dichloride (1.28 mmol) in 5 mL of chloroform and p-xylylenediamine (1.28 mmol) in 5 mL of chloroform were simultaneously added dropwise over 5 h to a stirred solution of squaraine **9a** (0.32 mmol) and triethylamine (3.2 mmol) in 40 mL of CHCl₃. After overnight stirring, the mixture containing rotaxane **9b** and polymer co-products was filtered off, washed with chloroform (100 mL) to extract rotaxane **9b**, and the filtrate was concentrated. Crude product was column purified using silica gel (gradient 0-2% MeOH in CHCl₃) to give rotaxane **9b**. λₘₐₓ (abs): 644 nm (CHCl₃).

### d) Synthesis of squaraine-rotaxane dye 9

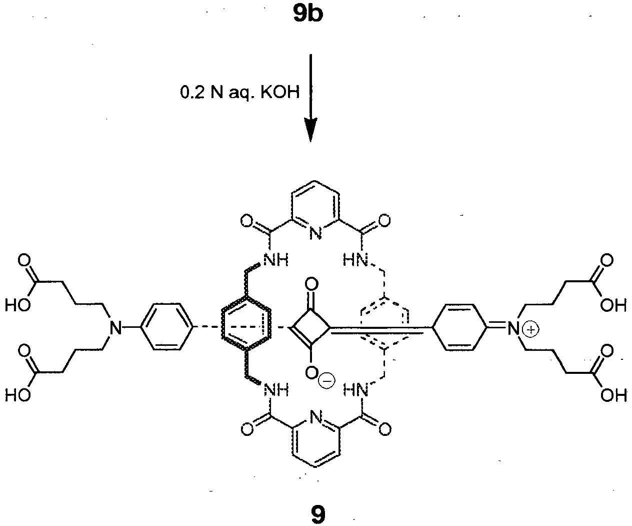

2 mmol of dye **9b** were stirred for 3 h at room temperature in the mixture of 25 mL of ethanol and 25 mL of 0.2 N aqueous solution of KOH. Then the mixture was neutralized with 0.2 N HCl and the resulted solution was column purified (RP-18; methanol/water, gradient) to give squaraine-rotaxane **9**.

### EXAMPLE 9

### Synthesis of squaraine-rotaxane 10:

### a) Synthesize of 3-phenylammonio-1-propanesulfonate

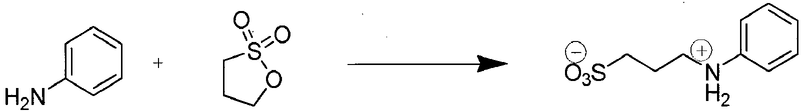

A mixture of aniline (7 mmol) and propane sultone (10 mmol) was heated in 10 mL of acetonitrile at 120-130 °C for 9 h in a pressure tube under argon atmosphere. The product was allowed to cool to room temperature and the solid of 3-phenylammonio-1-propanesulfonate was filtered and air-dried. Yield: 95%.

### b) Synthesis of 3-(3-ethyloxycarbonylpropylanilino)-1-propanesulfonate

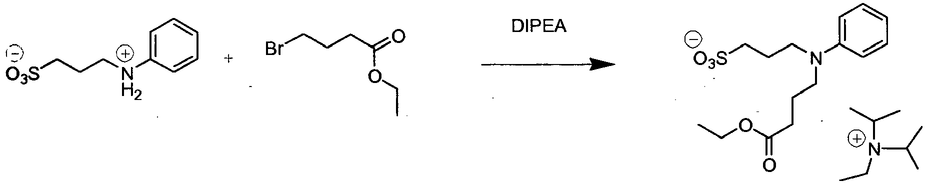

A mixture of 3-phenylammonio-1-propanesulfonate (10 mmol), ethyl 4-bromobutanoate (12 mmol) and N,N-diisopropylethylamine (DIPEA, 22 mmol) was heated in 10 mL acetonitrile at 120-130 °C for 6 h in a pressure tube under argon atmosphere. The solvent was removed under reduced pressure and ethyl acetate was added. The precipitated DIPEA bromide was filtered and the filtrate was evaporated. The residue of ethyl-diisopropyl-ammonium 3-(3-ethyloxycarbonylpropylanilino)-1-propanesulfonate was dried under vacuum and used for further synthesis without purification.

### c) Synthesis of squaraine dye 10a

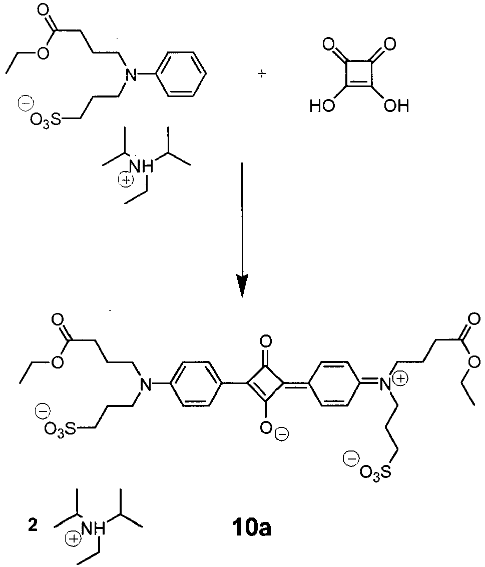

A mixture of dialkylaniline (1.83 mmol) and squaric acid (0.92 mmol) was refluxed in a solvent-mixture of n-butanol (15 mL) and toluene (30 mL) for 12 h. Then the deep green reaction mixture was concentrated and the crude product was precipitated by adding 30-40 mL of ether. After filtering, the product was washed several times with ether to yield the dark green squaraine dye **10a** as a solid. Yield: 30%. λₘₐₓ (abs): 644 nm, λₘₐₓ (em): 670 nm, Q.Y.: 3% (water).

### d) Synthesis of squaraine-rotaxane 10b

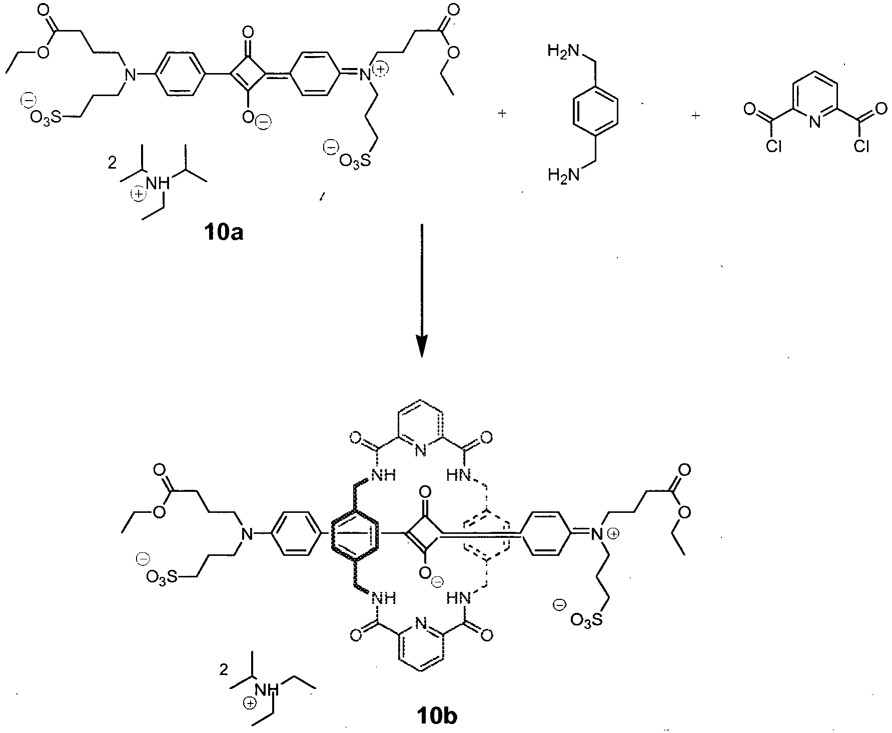

Clear solutions of 2,6-pyridinedicarbonyl dichloride (1.28 mmol) in 5 mL of chloroform and *p*-xylylenediamine (1.28 mmol) in 5 mL of chloroform were simultaneously added dropwise over a 5 h period to a stirred solution of **10a** (200 mg, 0.32 mmol) and triethylamine (3.2 mmol) in 40 mL of CHCl₃. After stirring overnight, the reaction mixture was filtered to remove any polymeric materials, and the resulting crude product was column purified (RP-18, methanol/water gradient) to yield **10b**. λₘₐₓ (abs): 656 nm, λₘₐₓ (em): 674 nm.

### e) Synthesis of squaraine-rotaxane dye 10

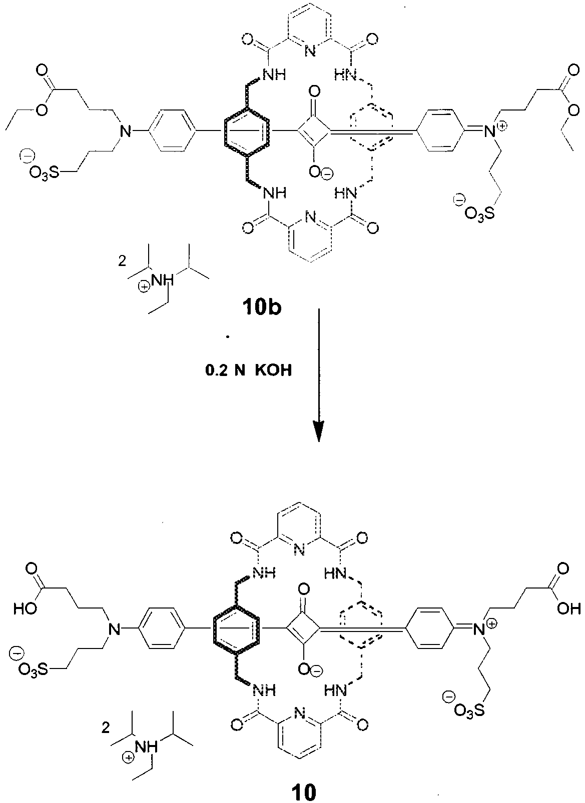

2 mmol **10b** were stirred for 3 h at room temperature in 50 mL of 0.2 N aqueous solution of KOH. Than the mixture was neutralized with 0.2 N HCl and the resulting solution was column purified (RP-18; methanol/water, gradient) to yield squaraine-rotaxane **10**. λₘₐₓ (abs): 655 m, λₘₐₓ (em): 673nm, Q.Y.: 25% (water).

### Synthesis of the NHS ester of rotaxane 10

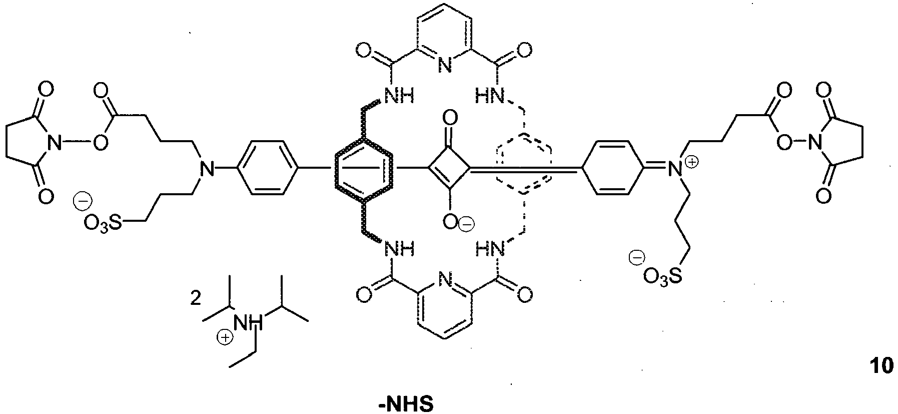

5.2 mg (2.97 µmol) of **10**, 3.0 mg (10 µmol) of TSTU, and 13.0 mg (10 µmol) of DIPEA were dissolved in 1 mL of DMF. The solution was stirred at room temperature for 4 h to give **10-NHS**. The resulted solution was used for labelling.

### EXAMPLE 10

### Synthesis of rotaxane 11:

### Synthesis of the reactive, symmetrical cyanine

The synthesis of the indolenine **1m** is analogous to the synthesis of **1d** except that for the quarternization of the indolenine benzylbromide or p-sulfo-benzylbromide is used. The synthesis of the cyanine **11a** is analogous to the synthesis described in U.S. Patent Application Publication No. US2002/0077487.

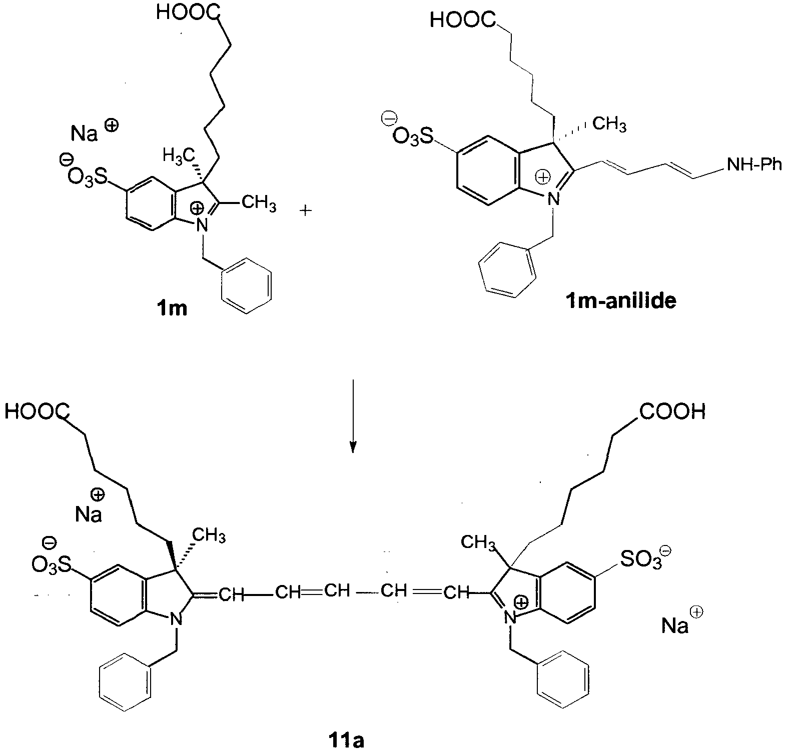

### Synthesis of rotaxane 11:

2,6-pyridinedicarbonyl dichloride (0.2 mmol) and p-xylylenediamine (0.2 mmol) each dissolved in 5 mL CHCl₃ were simultaneously added dropwise over five hours to a stirred solution of 8a (0.05 mmol) and triethylamine (0.5 mmol) in 40 mL of CHCl₃/MeOH. After stirring the reaction mixture overnight, the reaction mixture was filtered, the solvent removed under reduced pressure and the resulting crude product chromatographed using a reversed-phase silica column and water/MeOH as eluent.

### EXAMPLE 11

### Examples of Representative Structures

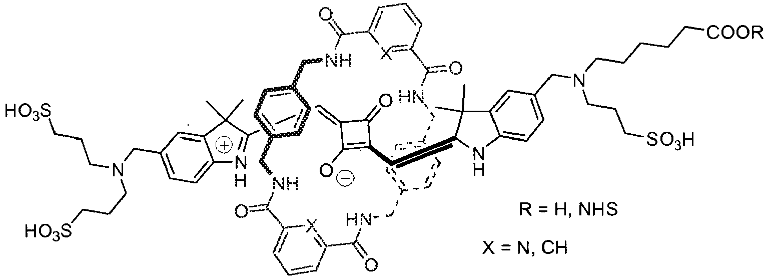

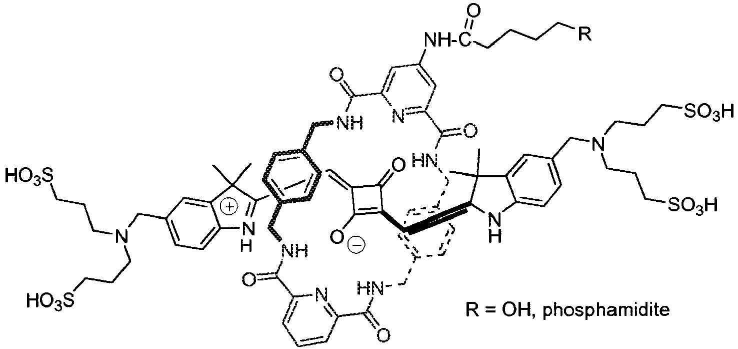

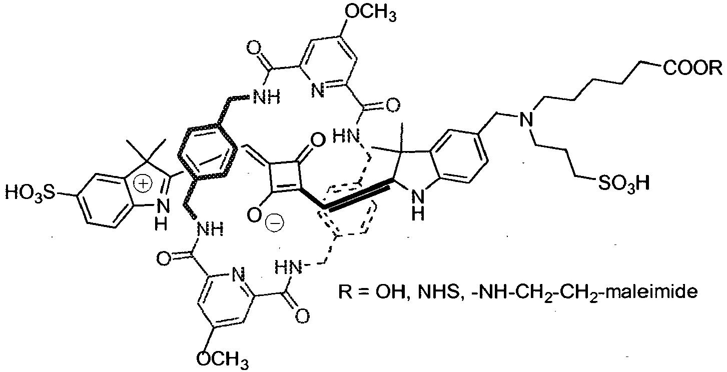

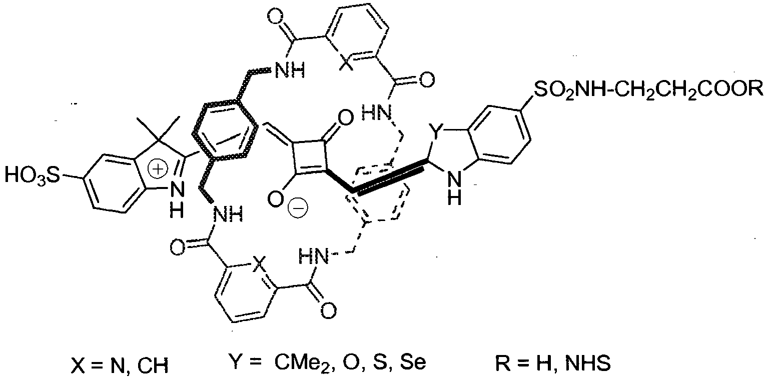

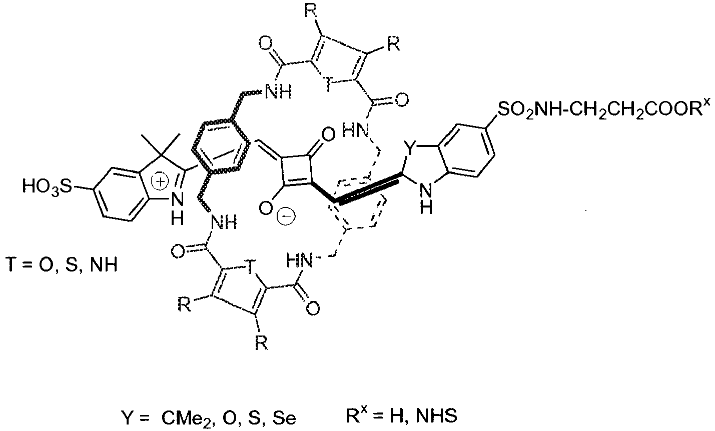

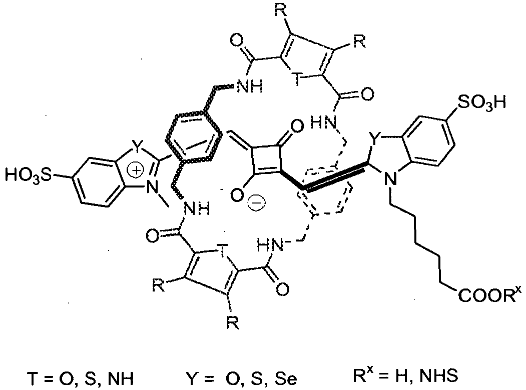

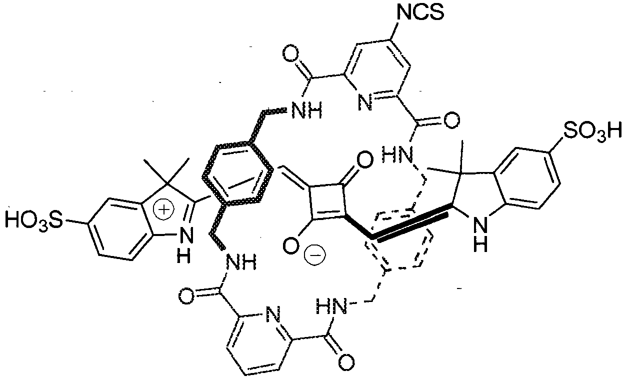

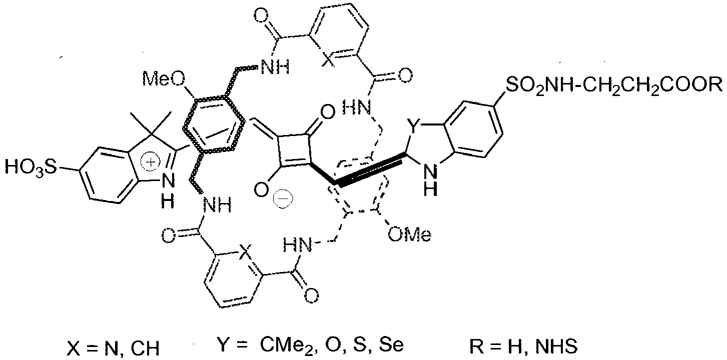

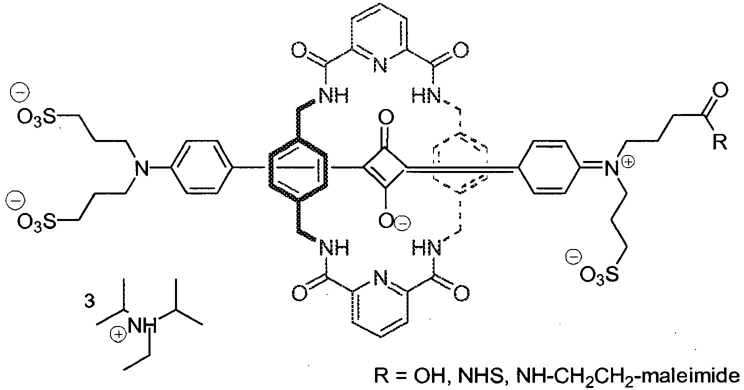

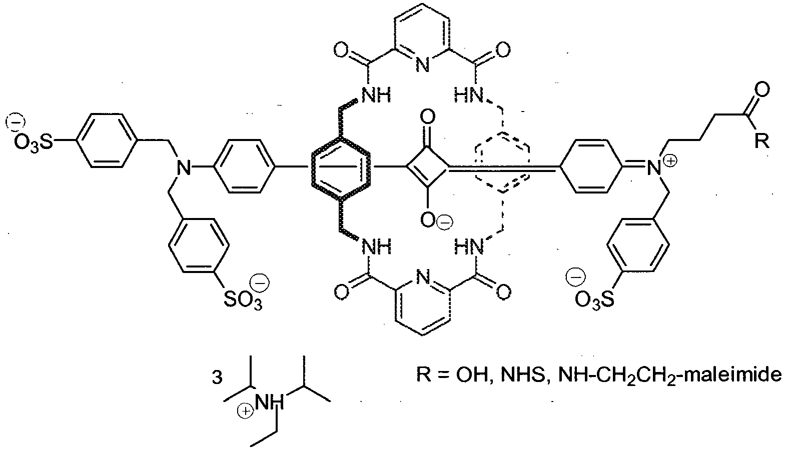

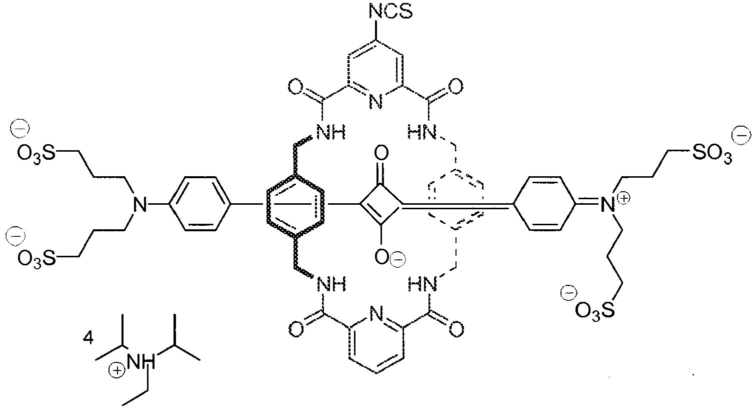

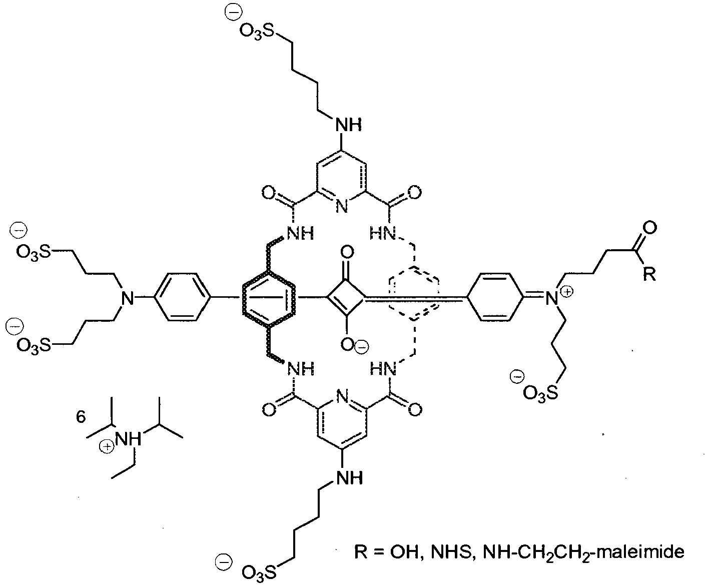

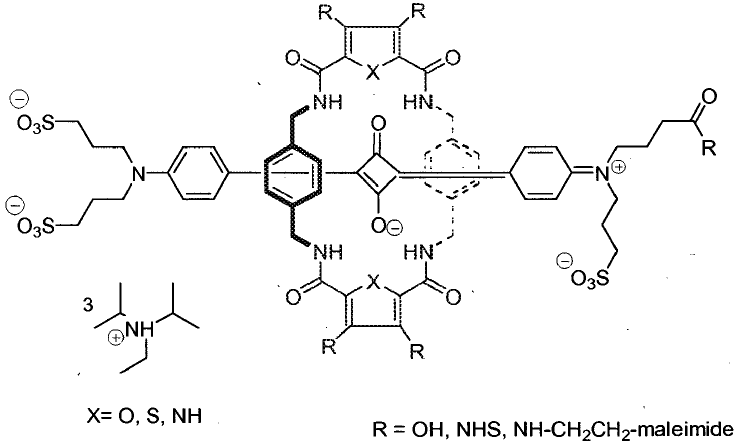

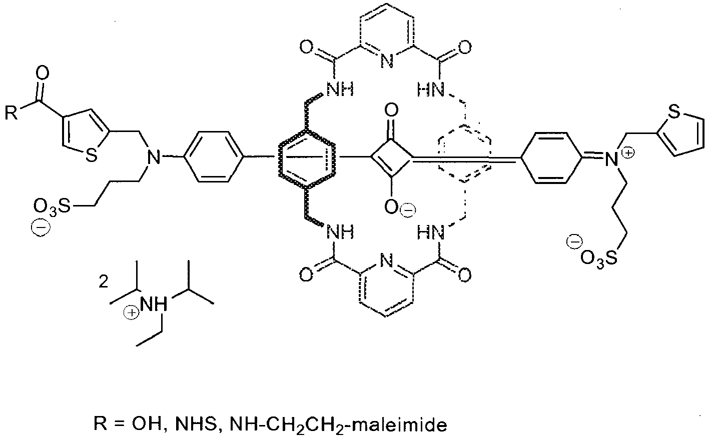

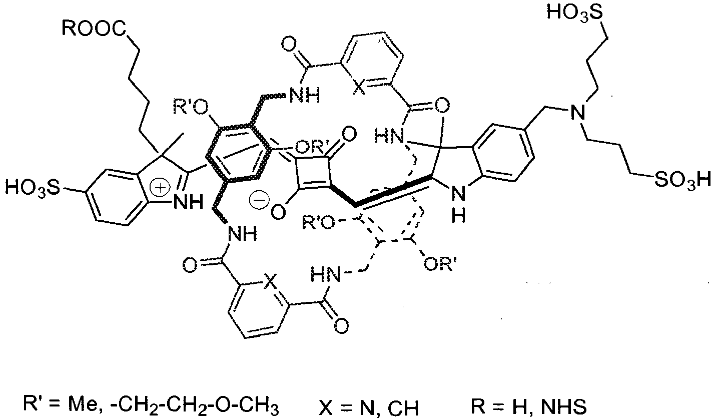

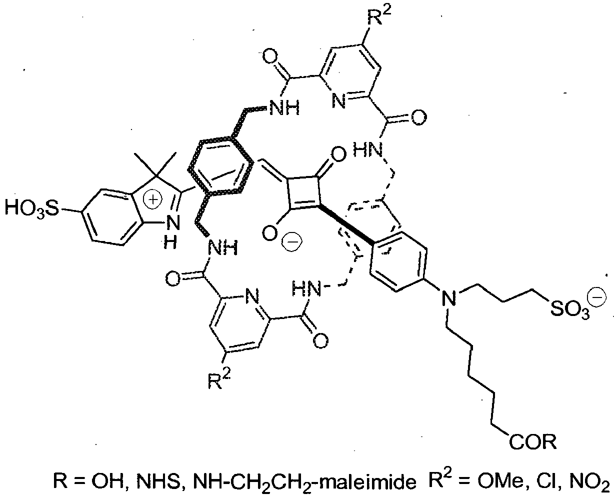

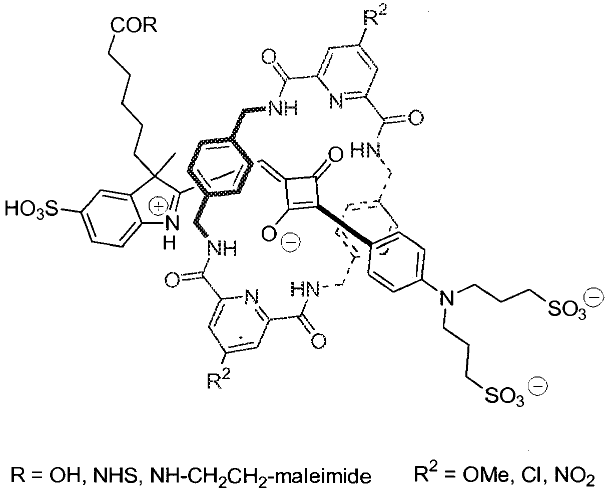

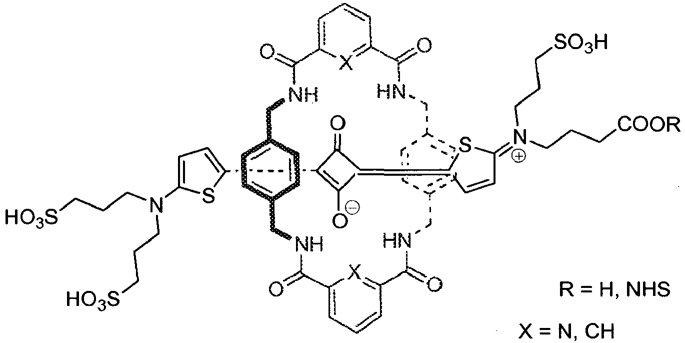

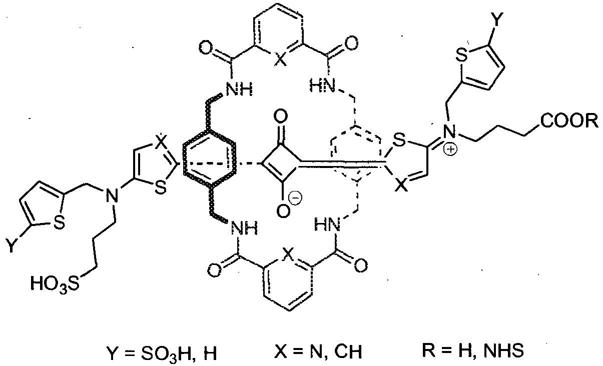

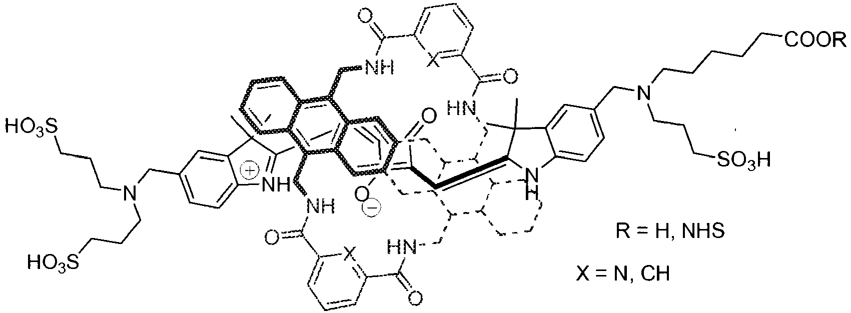

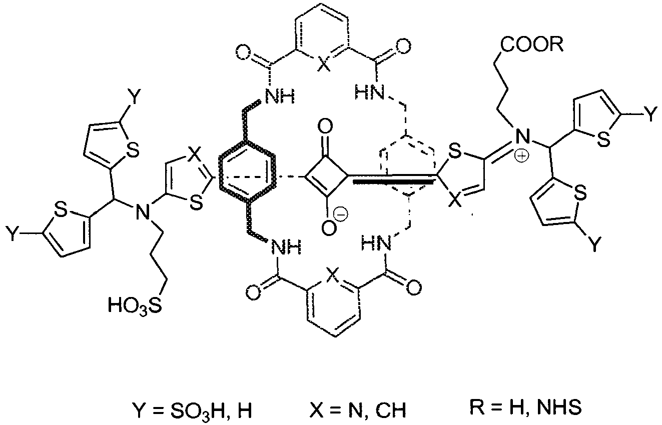

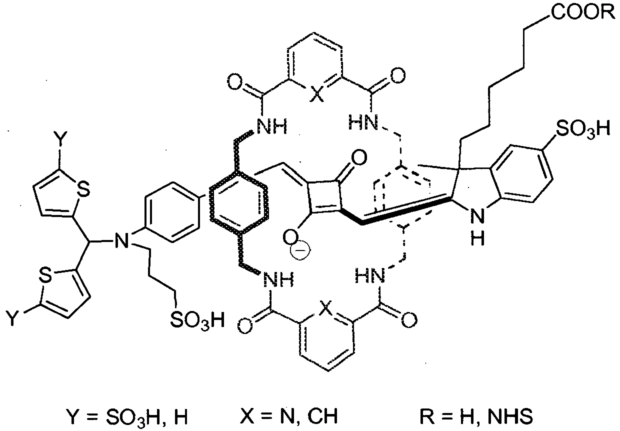

### General Protein Labelling Procedures and Determination of Dye-to-Protein Ratios

Protein labelling reactions are carried out using a 50 mM bicarbonate buffer (pH 9.1). A stock solution of 1 mg of dye in 100 µL of anhydrous DMF is prepared. 10 mg of protein is dissolved in 1 mL of 100 mM bicarbonate buffer (pH 9.1). Dye from the stock solution is added, and the mixture is stirred for 8-12 h at room temperature.

Unconjugated dye is separated from labeled protein using gel permeation chromatography with SEPHADEX G50 (0.5 cm x 20 cm column) and a 22 mM phosphate buffer solution (pH 7.3) as the eluent. The first colored band contains the dye-protein conjugate. The blue band with the much higher retention time contains the separated free dye. A series of labeling reactions using different dye-to-protein starting ratios are set up to obtain different dye-to-protein ratios for the labeled protein. Compared to the free forms, the protein-bound forms of the dyes sometimes show distinct changes in their spectral properties in particular when the dye is a squaraine dye.

The protein concentration is determined using either a BCA Protein Assay Reagent Kit from Pierce (Rockford, Illinois). Alternatively the protein concentration can be determined by measurement of the absorption around 280 nm. The dye-to-protein ratio (D/P) gives the number of dye molecules covalently bound to the protein.

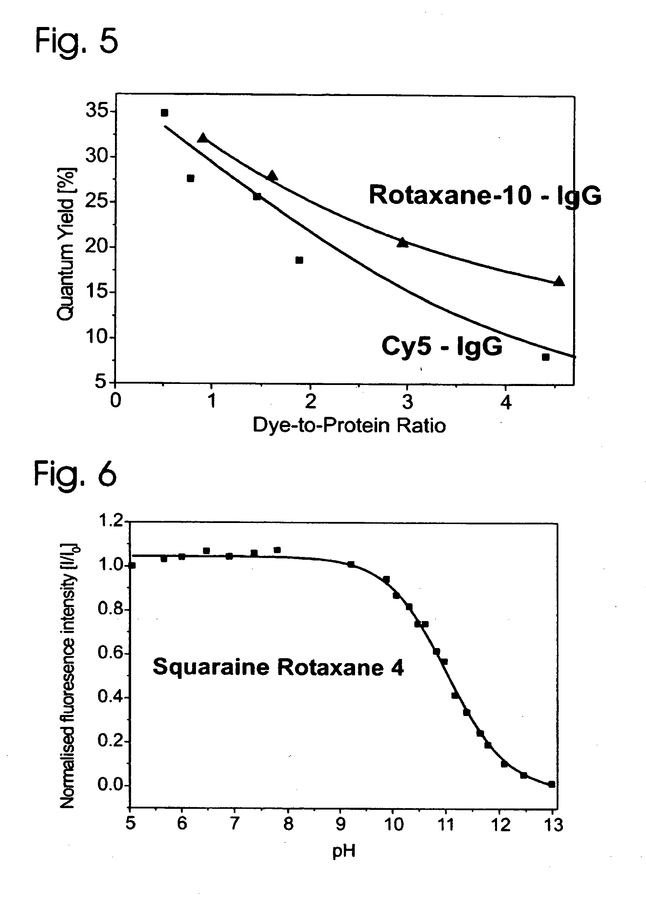

The table below is showing data on the photophysical properties for rotaxane-protein conjugates at various dye-to-protein ratios. The absorption and emission spectrum of a relevant conjugate is shown in Figure 4. A graph of the Q.Y.'s of conjugates with different D/P ratios in comparison to Cy5, a commercially available protein marker, is shown in Figure 5.

**Table: Spectral and photophysical properties of compound 10 and 10-IgG**

| **Sample** | **Dye-to-protein Ratio** | **Absorption max. [nm]** | **Extinction Coefficient [M⁻¹·cm⁻¹]** | **Emission max. [nm]** | **Quantum Yield [%]** |
|---|---|---|---|---|---|
| **Rotaxane 10** | - | 655 | 200,000 | 673 | 25 |
| **10-IgG** conjugate 1 | 1.0 | 657 | | 675 | 31 |
| **10-IgG** conjugate 2 | 2.0 | 657 | | 675 | 25 |
| **10-IgG** conjugate 3 | 3.0 | 657 | | 675 | 21 |
| **10-IgG** conjugate 4 | 4.0 | 657 | | 675 | 18 |

### Covalent labelling to IgG

385 µL (5.2 mg/mL) of IgG is dissolved in a 750 µL bicarbonate buffer (0.1 M, pH 9.0). 1 mg of NHS-ester (e.g. **10-NHS**) is dissolved in 50 µL of DMF and slowly added to the above-prepared protein solution with stirring. After 20 h of stirring, the protein-conjugate is separated from the free dye using Sephadex G50 and a phosphate buffer (22 mM, pH 7.2). The first blue band that is isolated contains the labeled conjugate.

### Conjugation to HSA

0.5 mg of the relevant NHS in 50 µL of DMF are slowly added to a stirred solution of 5 mg of HSA in 750 µL of bicarbonate buffer (0.1 M, pH 9.0). The mixture is stirred for another 6 h at room temperature. The mixture is dialyzed against a phosphate buffer (22 mM, pH 7.2) using a dialysis membrane (1500 FT, Union Carbid) with a cutoff of 10.000.

The labelling procedures of alternative reporter compounds having reactive functional groups are analoguous to the one reported here.

### Description of applications of the invention

The above disclosed compositions exhibit utility for a variety of useful methods and for various assay formats.

The assay may be a competitive assay that includes a recognition moiety, a binding partner, and an analyte. Binding partners and analytes may be selected from the group consisting of biomolecules, drugs, and polymers, among others. In some competitive assay formats, one or more components are labeled with photoluminescent compounds in accordance with the invention. For example, the binding partner may be labeled with such a photoluminescent compound, and the displacement of the compound from an immobilized recognition moiety may be detected by the appearance of fluorescence in a liquid phase of the assay. In other competitive assay formats, an immobilized enzyme may be used to form a complex with the fluorophore-conjugated substrate.

The binding of antagonists to a receptor can be assayed by a competitive binding method in so-called ligand/receptor assays. In such assays, a labeled antagonist competes with an unlabeled ligand for the receptor binding site. One of the binding partners can be, but not necessarily has to be, immobilized. Such assays may also be performed in microplates. Immobilization can be achieved via covalent attachment to the well wall or to the surface of beads.

Other preferred assay formats are immunological assays. There are several such assay formats, including competitive binding assays, in which labeled and unlabeled antigens compete for the binding sites on the surface of an antibody (binding material). Typically, there is a certain incubation time required to provide sufficient time for equilibration. Such assays can be performed in a heterogeneous or homogeneous fashion.

Sandwich assays may use secondary antibodies and excess binding material may be removed from the analyte by a washing step.

Other types of reactions include binding between avidin and biotin, protein A and immunoglobulins, lectins and sugars (e.g., concanavalin A and glucose).

Certain dyes of the invention are charged due to the presence sulfonic groups. These compounds are impermeant to membranes of biological cells. In these cases treatments such as electroporation and shock osmosis can be used to introduce the dye into the cell. Alternatively, such dyes can be physically inserted into the cells by pressure microinjection, scrape loading etc.

The reporter compounds described here also may be used to sequence nucleic acids and peptides. For example, fluorescently-labeled oligonucleotides may be used to trace DNA fragments. Other applications of labeled DNA primers include fluorescence *in-situ* hybridization methods (FISH) and for single nucleotide polymorphism (SNIPS) applications, among others.

Multicolor labeling experiments may permit different biochemical parameters to be monitored simultaneously. For this purpose, two or more reporter compounds are introduced into the biological system to report on different biochemical functions. The technique can be applied to fluorescence *in-situ* hybridization (FISH), DNA sequencing, fluorescence microscopy, and flow cytometry. One way to achieve multicolor analysis is to label biomolecules such as nucleotides, proteins or DNA primers with different luminescent reporters having distinct luminescence properties. Luminophores with narrow emission bandwidths are preferred for multicolor labeling, because they have only a small overlap with other dyes and hence increase the number of dyes possible in a multicolor experiment. Importantly, the emission maxima have to be well separated from each other to allow sufficient resolution of the signal. A suitable multicolor triplet of fluorophores would include a Cy3-analog of this invention, TRITC, and a Cy5-analog as described herein, among others.

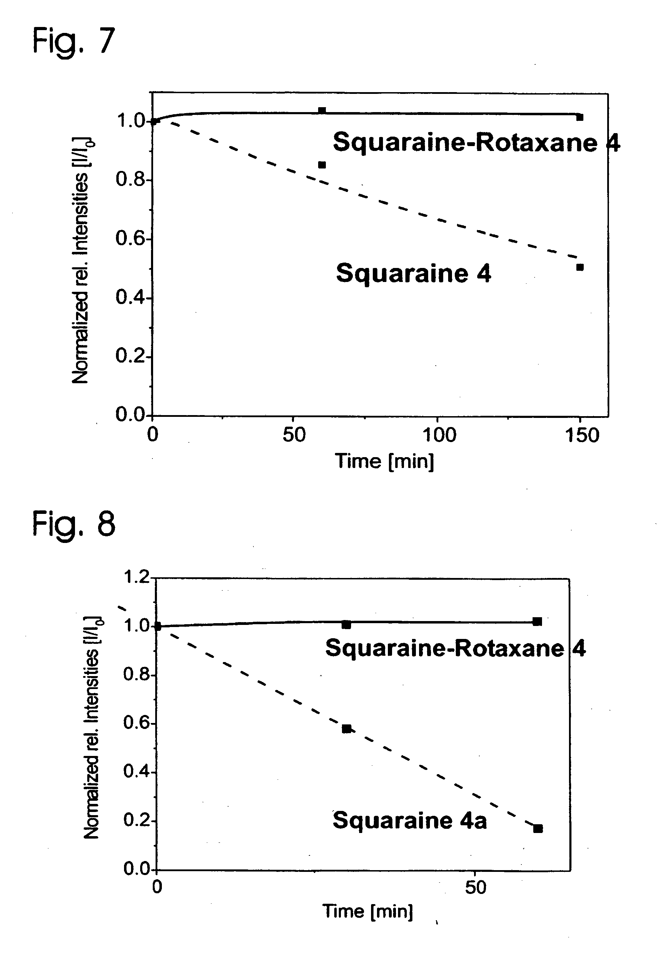

Phosphoramidites are useful functionalities for the covalent attachment of dyes to oligos in automated oligonucleotide synthesizers. They are easily obtained by reacting the hydroxyalkyl-modified dyes of the invention with 2-cyanoethyl-tetraisopropyl-phosphorodiamidite and 1-H tetrazole in methylene chloride. The phosphoramidite chemistry requires these dyes to be stable against deprotection chemistries that involve treatment with a base. We therefore tested the stability of rotaxane 4 at pH 13 in comparison to the squaraine precursor 4a. The result is shown in Figure 8.

The simultaneous use of FISH (fluorescence *in-situ* hybridization) probes in combination with different fluorophores is useful for the detection of chromosomal translocations, for gene mapping on chromosomes, and for tumor diagnosis, to name only a few applications. One way to achieve simultaneous detection of multiple sequences is to use combinatorial labeling. The second way is to label each nucleic acid probe with a luminophore with distinct spectral properties. Similar conjugates can be synthesized from this invention and can be used in a multicolor multi-sequence analysis approach.

In another approach the dye-compositions of this invention might be used to directly stain or label a sample so that the sample can be identified and or quantitated. Such dyes might be added/labeled to a target analyte as a tracer. Such tracers could be used e.g. in photodynamic therapy where the labeled compound is irradiated with a light source and thus producing singlet oxygen that helps to destroy tumor cells and diseased tissue samples.

The reporter compounds of the invention can also be used for screening assays for a combinatorial library of compounds. The compounds can be screened for a number of characteristics, including their specificity and avidity for a particular recognition moiety.

Assays for screening a library of compounds are well known. A screening assay is used to determine compounds that bind to a target molecule, and thereby create a signal change which is generated by a labeled ligand bound to the target molecule. Such assays allow screening of compounds that act as agonists or antagonists of a receptor, or that disrupt a protein-protein interaction. It also can be used to detect hybridization due to binding of DNA and/or RNA.

Other screening assays are based on compounds that affect the enzyme activity. For such purposes, quenched enzyme substrates of the invention could be used to trace the interaction with the substrate. In this approach, the cleavage of the fluorescent substrate leads to a change in the spectral properties such as the excitation and emission maxima, intensity and/or lifetime, which allows distinguishing between the free and the bound luminophore.

The reporter compounds disclosed above may also be relevant to single molecule fluorescence microscopy (SMFM) where detection of single probe molecules depends on the availability of a fluorophore with high fluorescence yield, high photostability, and long excitation wavelength.

The dye compositions are also useful for use as biological stains. There are limitations in some instances to the use of compounds as labels. For example, typically only a limited number of dyes may be attached to a biomolecules without altering the fluorescence properties of the dyes (e.g. quantum yields, lifetime, polarization, emission characteristics, etc.) and/or the biological activity of the bioconjugate. Typically quantum yields may be reduced at higher degrees of labelling. The current invention should help to overcome some of these limitations by reducing the aggregation tendencies of these dye compositions.

Another means to overcome the above limitation for the use of such compounds as fluorescent markers offers encapsulation into beads. Fluorescent beads and polymeric materials are becoming increasingly attractive as labels and materials for bioanalytical and sensing applications. Various companies offer particles with defined sizes ranging from nanometers to micrometers. Noncovalent encapsulation in beads may be achieved by swelling the polymer in an organic solvent, such as toluene or chloroform, containing the dye. Covalent encapsulation may be achieved using appropriate reactive functional groups on both the polymer and the dyes.

In general, hydrophobic versions of the invention may be used for non-covalent encapsulation in polymers, and one or more dyes could be introduced at the same time. Surface-reactive fluorescent particles allow covalent attachment to molecules of biological interest, such as antigens, antibodies, receptors etc. Hydrophobic versions of the invention such as dye having lipophilic substituents such as phospholipids will non-covalently associate with lipids, liposomes, lipoproteins. They are also useful for probing membrane structure and membrane potentials.

Compounds of this invention may also be attached to the surface of metallic nanoparticles such as gold or silver nanoparticles or colloids. It has recently been demonstrated that fluorescent molecules may show increased quantum yields near metallic nanostructures e.g. gold or silver nanoparticles (O. Kulakovich et al., Nanoletters 2(12) 1449-52 (2002)). This enhanced fluorescence may be attributable to the presence of a locally enhanced electromagnetic field around metal nanostructures. The changes in the photophysical properties of a fluorophore in the vicinity of the metal surface may be used to develop novel assays and sensors. In one example the nanoparticle may be labeled with one member of a specific binding pair (antibody, protein, receptor, etc.) and the complementary member (antigen, ligand) may be labeled with a fluorescent molecule in such a way that the interaction of both binding partners leads to an detectable change in one or more fluorescence properties (such as intensity, quantum yield, lifetime, among others). Replacement of the labeled binding partner from the metal surface may lead to a change in fluorescence that can then be used to detect and/or quantify an analyte.

Gold colloids can be synthesized by citrate reduction of a diluted aqueous HAuCl₄ solution. These gold nanoparticles are negatively charged due to chemisorption of citrate ions. Surface functionalization may be achieved by reacting the nanoparticles with thiolated linker groups containing amino or carboxy functions. In another approach, thiolated biomolecules are used directly for coupling to these particles.

In recent studies (T. Fare et al., Anal. Chem. 75(17), 4672-4675 (2003)) researchers made an observation that the fluorescence signals of cyanine dyes such as CY5 dye and the ALEXA 647 dyes in microarrays are strongly dependent on the concentration of ozone during post-hybridization array washing. Controlled exposures of microarrays to ozone confirmed this factor as the root cause, and showed the susceptibility of a class of cyanine dyes (e.g., CY5 dyes, ALEXA 647 dyes) to ozone levels as low as 5-10 ppb for periods as short as 10-30 s.

One of the significant findings was the low dose level (ozone concentration multiplied by exposure time) that could induce the onset of the phenomenon, suggesting many labs may be at risk. For example, it is not uncommon that the environmental ozone levels would exceed 60 ppb during peak traffic hours on a sunny summer afternoon. Reporter compounds present on or in arrays that are exposed to these levels for as short as 1 min may begin to show significant degradation in a typical laboratory setting.

There are ways that help to eliminate the occurrence of ozone effects on microarrays, for example equipping laboratories with HVAC systems having filters to significantly reduce ozone levels, or the use of dye-protecting solutions to avoid signal degradation. However, each of these approaches may add additional costs and/or time to perform the assay. These findings suggest the need for dyes and labels in the 600 to 700 nm wavelength range with improved chemical and photochemical stability. Dye compositions of this invention exhibit increased photochemical but also chemical stability in particular against oxidative reagents such as peroxides (Figure 7) and ozone and should therefore be excellent reagents for the use in microarrays.

### Analytes

The invention may be used to detect an analyte that interacts with a recognition moiety in a detectable manner. As such, the invention can be attached to a recognition moiety which is known to those of skill in the art. Such recognition moieties allow the detection of specific analytes. Examples are pH-, or potassium sensing molecules, e.g., synthesized by introduction of potassium chelators such as crown-ethers (aza crowns, thia crowns etc). Dyes with N-H substitution in the heterocyclic rings such as **4** exhibit pH-sensitive absorption and emission (S. Miltsov et al., Tetrahedron Lett. 40: 4067-68 (1999), M.E. Cooper et al., J. Chem. Soc. Chem. Commun. 2000, 2323-2324), Calcium-sensors based on the BAPTA (1,2-Bis(2-aminophenoxy)ethan-N,N,N',N'-tetra-acetic acid) chelating moiety are frequently used to trace intracellular ion concentrations. The combination of a compound of the invention and the calcium-binding moiety BAPTA may lead to new long-wavelength absorbing and emitting Ca-sensors which could be used for determination of intra- and extracellular calcium concentrations (Akkaya et al., Tetrahedron Lett. 38:4513-4516 (1997)). Additionally, or in the alternative, reporter compounds already having a plurality of carboxyl functional groups may be directly used for sensing and/or quantifying physiologically and environmentally relevant ions.

NH-substituted dyes of this invention are pH sensitive and may also be useful for the assessment of the intracellular pH and for applications where the local pH of the environment changes e.g. cell-based measurements of G-protein coupled receptors as described in M.E. Cooper et al. J. Chem. Soc. Chem. Commun. 2000, 2323-2324. The water-soluble dyes may be used directly or the reactive pH-sensitive dyes of the invention are associated with specific biomolecules which bind to certain domains in cells thus enabling the pH of only that specific environment to be assessed. While the dioxo-squaraines and dioxo-squaraine-rotaxanes (see Example 3) have pKa values in the basic pH range (Figure 6) (Miltsov et al., Tetrahedron Lett. 40, 4067-68 (1999)), the pKa's of squaraine-ring-substituted versions like the thio-derivatives may be closer to the physiological pH range, which would makes them more useful for these type of measurements. It is understood that the dyes pKa's can be tuned to cover a broad pH-range by variation of the substituents on the heterocyclic bases as well as on the squaraine bridge.

### Fluorescence methods

The disclosed reporter compounds may be detected using common intensity-based fluorescence methods. Unrotaxanated squaraine dyes are known to have lifetimes in the range of hundreds of ps to a few ns. The table below provides some of the lifetime data that were measured for the free squaraines and their rotaxanes. The lifetime for the aniline-based squaraine **10a** increases about 2 fold upon conjugation to an antibody and the lifetimes of the IgG-conjugates seem to be independent of the dye-to-protein ratios (Table above).

Importantly, the already long average lifetime of the water-soluble indolenine squaraine **4a** further increases to 4.4 ns upon conversion into the rotaxane **4**. This lifetime is comparable to that of the free squaraine dye **3a** in non-aqueous solution, where less quenching is observed.

The nanosecond lifetime and long-wavelength absorption and emission of these dyes may allow them to be measured using relatively inexpensive instrumentation that employs laser diodes for excitation and avalanche photodiodes for detection. Typical assays based on the measurement of the fluorescence lifetime as a parameter include for example FRET (fluorescence resonance energy transfer) assays. The binding between a fluorescent donor labeled species (typically an antigen) and a fluorescent acceptor labeled species may be accompanied by a change in the intensity and the fluorescence lifetime. The lifetime can be measured using intensity- or phase-modulation-based methods (J.R. LAKOWICZ, PRINCIPLES OF FLUORESCENCE SPECTROSCOPY (2nd ed. 1999)).

The lifetimes in the order of a few ns that have been measured for some of the rotaxane-dye structures in aqueous solution (see Table below) make these labels extremely useful as tracers for applications in fluorescence polarization based assays. Fluorescence polarization immunoassays (FPI) are widely applied to quantify low molecular weight antigens. The assays are based on polarization measurements of antigens labeled with fluorescent probes. The requirement for polarization probes used in FPIs is that emission from the unbound labeled antigen be depolarized and increase upon binding to the antibody. Low molecular weight species labeled with the compounds of the invention can be used in such binding assays, and the unknown analyte concentration can be determined by the change in polarized emission from the fluorescent tracer molecule.

The long fluorescence lifetimes of benzo-selenazole-based squaraines in chloroform suggest that water-soluble rotaxane-analogs of these dyes might be useful as polarization probes.

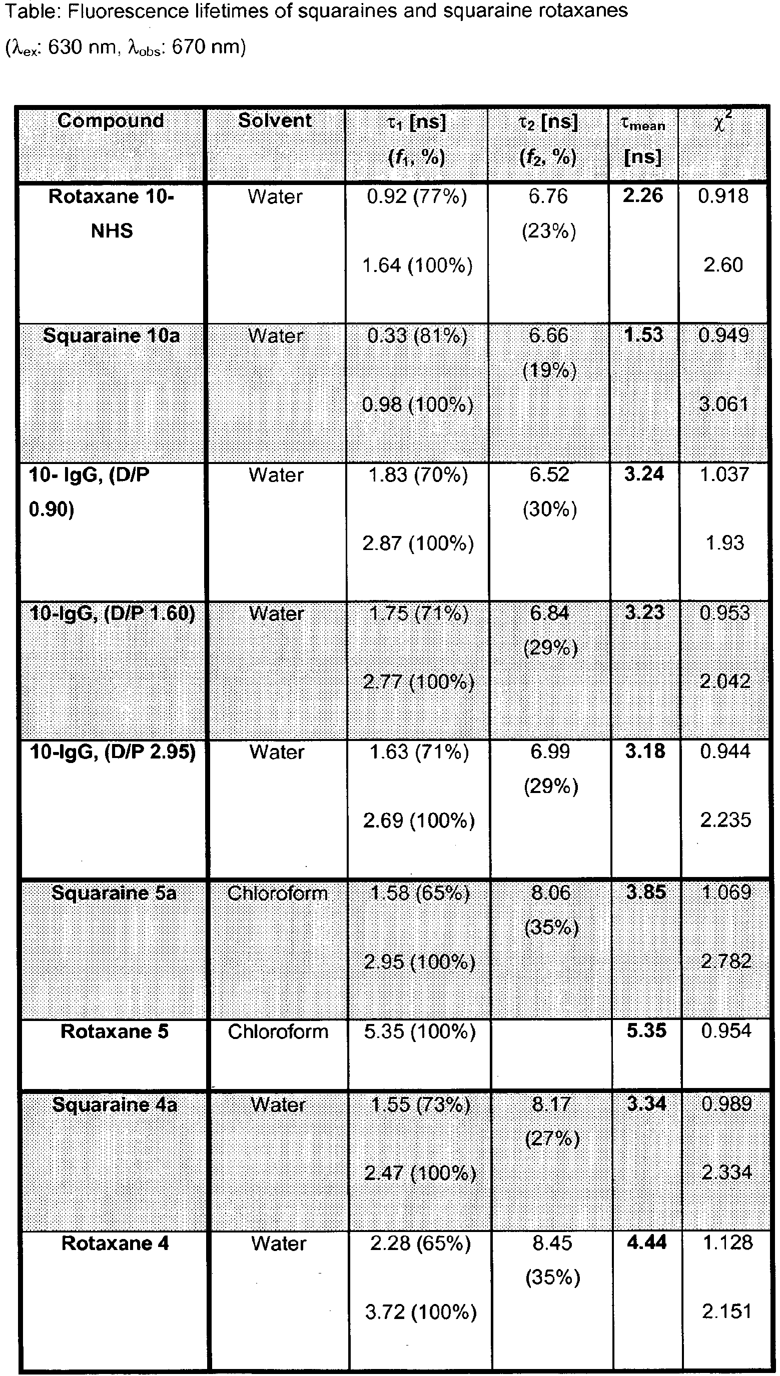

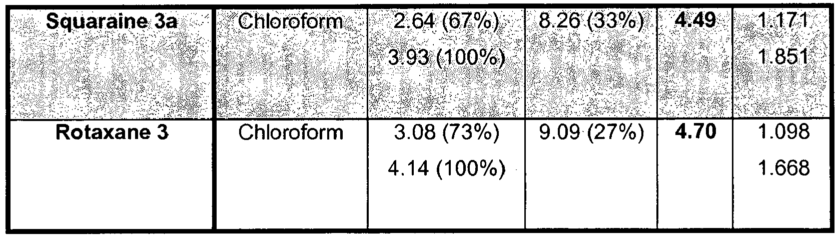

Luminescent dyes of this invention are also useful as fluorescent acceptors in TR-FRET applications with luminescent lanthanides as donors. In these assays the emission from both the donor and the acceptor is collected and ratioed to increase the robustness of the assay.

The luminescent rotaxanes of the invention are also useful for in vivo near infrared diagnostic methods, as described in U.S. Patent No. 6,083,485 (hereby incorporated by reference). Such methods typically include administering a rotaxane of the present invention, diseased tissue to light in the visible and near infrared range, and recording the emitted light produced from the rotaxane.

### Compositions and Kits

The invention also provides compositions, kits and integrated systems for practicing the various aspects and embodiments of the invention, including producing the novel compounds and practicing of assays. Such kits and systems may include a reporter compound as described above, and may optionally include one or more of solvents, buffers, calibration standards, enzymes, enzyme substrates, and additional reporter compounds having similar or distinctly different optical properties.

## Claims

1. A rotaxane having the formula: 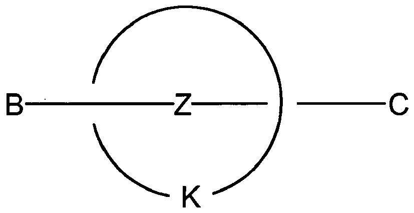 where:
K has the formula K¹, K², K³, where
K¹ is 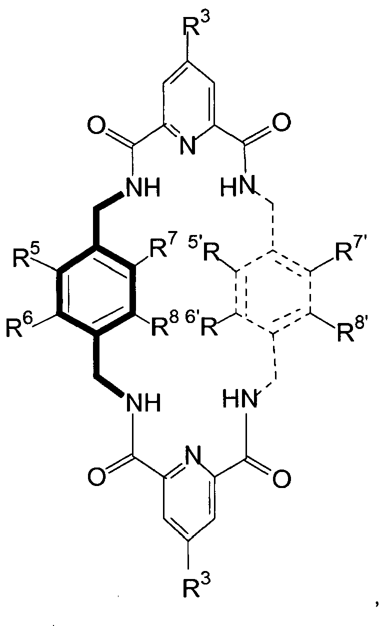
K² is 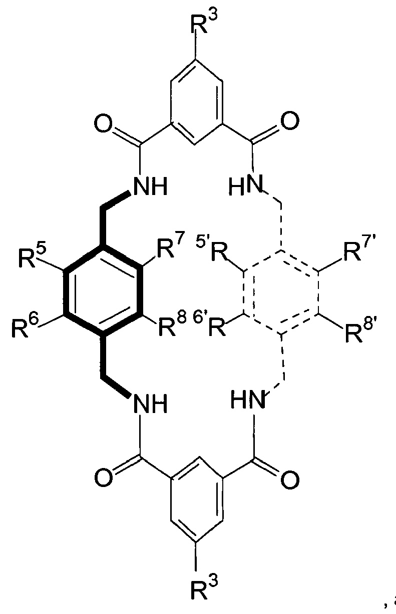 and
K³ is 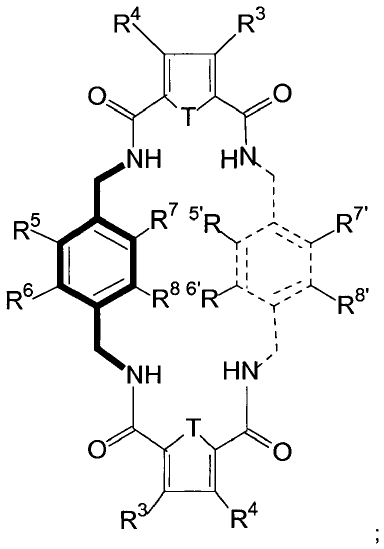 where substituents R³, R⁴, R⁵, R^{5'}, R⁶, R^{6'}, R⁷, R^{7'}, R⁸ and R^{8'}, when present, are independently selected from the group of H, L-R^{x}, L-S_{c} and L-R^{±}, alkyl, aryl, alkoxy, alkyl-aryl, F, Br, Cl, I, OH, nitro, and cyano;
R⁵ in combination with R⁶, R⁷ in combination with R⁸, R⁵ in combination with R^{6'}, and R⁷ in combination with R^{8'} may independently form an aromatic, heterocyclic or aliphatic ring system;
L is a single covalent bond, a covalent linkage that is linear or branched, cyclic or heterocyclic, saturated or unsaturated, having 1-20 nonhydrogen atoms from the group of C, N, P, O and S, in such a way that the linkage contains any combination of ether, thioether, amine, ester, amide bonds; single, double, triple or aromatic carbon-carbon bonds; or carbon-sulfur bonds, carbon-nitrogen bonds, phosphorus-sulfur, nitrogen-nitrogen, nitrogen-oxygen or nitrogen-platinum bonds, or aromatic or heteroaromatic bonds;
R^{x} is a reactive group selected from activated carboxylic ester, acyl azide, acyl halide, acyl nitrile, aldehyde, ketone, alkyl halide, alkyl sulfonate, anhydride, aryl halide, aziridine, boronate, carboxylic acid, carbodiimide, diazoalkane, epoxide, haloacetamide, halotriazine, imido ester, isocyanate, isothiocyanate, maleimide, phosphoramidite, silyl halide, sulfonate ester, and sulfonyl halide, or is selected from N-hydroxysuccinimide esters, isothiocyanates, sulfonylchlorides, iodoacetamides, maleimides, N-hydroxybenztriazole esters, thioesters, p-nitrophenyl esters, alkyl, alkenyl, alkynyl, and aromatic esters, acyl imidazoles, alkylhalides, hydroxyl groups, aldehydes, ketones, hydrazones, oximes, semicarbozones, isocyanates, activated C=C double-bond-containing groups, thiol groups, alkenes, phosphoramidites, primary amines, boronic acid derivatives, pyrylium moieties, haloplatinates, and aryl halides;
S_{c} is a conjugated substance selected from an amino acid, a peptide, a protein, a nucleoside, a nucleotide, a nucleic acid, a carbohydrate, a hapten, a lipid, an ion-chelator, a nonbiological polymer, a cell, or a cellular component, or is selected from a protein, carbohydrate, nucleic acid, nonbiological polymers, and metallic nanoparticles, or is selected from the group consisting of a peptide, a polypeptide, a polynucleotide, a bead, a microplate well surface, a phospholipid, a metallic nanoparticle, an amino acid, a nucleic acid, a sugar, polysacc**h**aride, oligosaccharide, and a second fluorescent dye; and
R^{±} is an ionic group;
T is O, S, N-H;
Z has the formula 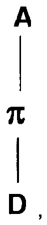
where π is a four-membered aromatic ring and A, B, C and D are the substituents of the four-membered ring;
and where B and C are separated by one of substituents A or D, B is one of W², W⁴, W⁶ or W⁸ and C is one of W¹, W³, W⁵ or W⁷, in which case one of A or D is negatively charged;
where A and D are neutral, they are selected from the group consisting of =O, =S, =Se, =Te, =N-R^{a}, and =C(R^{b})(R^{c}), where R^{a}, R^{b} and R^{c} are selected from the group consisting of H, L-S_{c}, L-R^{x}, L-R^{±}, aliphatic, aromatic, alicyclic, and aryl-alkyl, where each aliphatic residue may incorporate up to six heteroatoms selected from N, O, S, and can be substituted one or more times by F, Cl, Br, I, hydroxy, alkoxy, carboxy, sulfo, phosphate, amino, sulfate, phosphonate, cyano, nitro, azido, alkyl-amino, dialkyl-amino or trialkylammonium; -COOH, -CN, -OH, -SO₃H, -PO₃H₂ , -O-PO₃H₂, -PO₃R₂^{m}, -O-PO₃R₂^{m}-CONHR^{m}, -CONH₂, COO-NHS and COO-R^{m}, where R^{m} is selected from a group consisting of L-S_{c}, L-R^{x}, L-R^{±}, aliphatic substituents and aromatic substituents; or R^{b} and R^{c}, taken in combination, form a cyclic or heterocyclic ring structure;
where A and D are present and negatively charged, they are independently selected from the group consisting of -O^{θ}, -S^{θ}, -Se^{θ}, -Te^{θ}, -(N-R^{a})^{θ}, -(C(R^{b})(R^{c}))^{θ};
W¹, W², W³, W⁴, W⁵, W⁶, W⁷ and W⁸ have the respective formulae
W¹ is 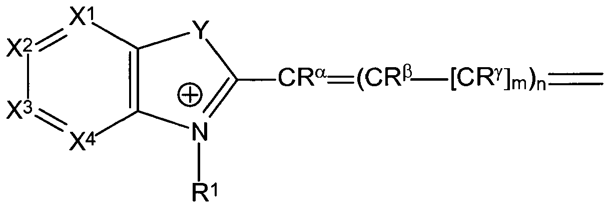
W² is 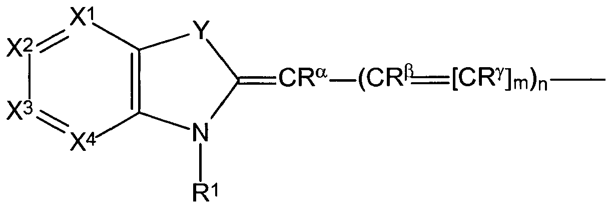
W³ is 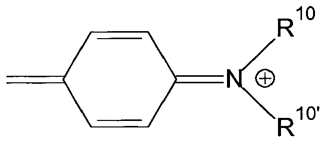
W⁴ is 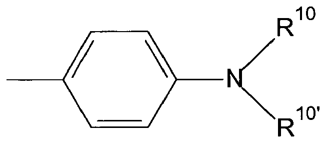
W⁵ is 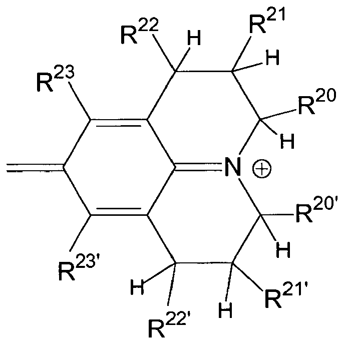
W⁶ is 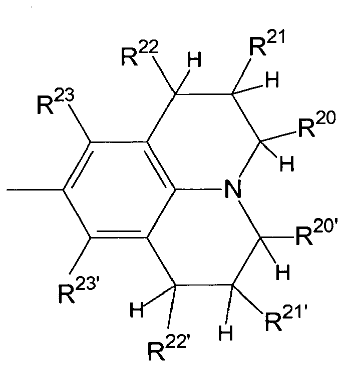
W⁷ is 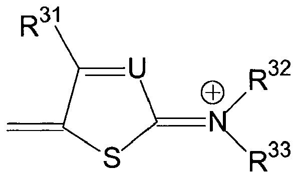
W⁸ is 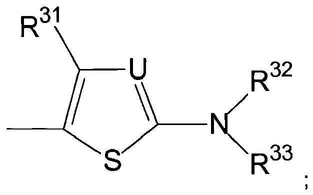 where m and n are independently 0 or 1 for each of B and C;
each Y is independently selected from the group consisting of O, S, N-R^{d}, CR^{e}=CR^{f} and C(Rⁱ)(R^{j}),
where R^{d} is selected from the group consisting of H, L-S_{c}, L-R^{x}, L-R^{±}, -CH₂-CONH-SO₂-Me, aliphatic groups, alicyclic groups, aromatic groups;
R^{e} and R^{f} are independently H, L-S_{c}, L-R^{x}, L-R^{±}, -CH₂-CONH-SO₂-Me, aliphatic groups, alicyclic groups, or aromatic groups; where each aliphatic residue may incorporate up to six heteroatoms selected from N, O, S, and can be substituted one or more times by F, Cl, Br, I, hydroxy, alkoxy, carboxy, sulfo, phosphate, amino, sulfate, phosphonate, cyano, nitro, azido, alkyl-amino, dialkyl-amino or trialkylammonium;
Rⁱ and R^{j} are independently H, L-S_{c}, L-R^{x}, L-R^{±}, -CH₂-CONH-SO₂-Me, aliphatic groups, alicyclic groups, or aromatic groups, where each aliphatic residue may incorporate up to six heteroatoms selected from N, O, S, and can be substituted one or more times by F, Cl, Br, I, hydroxy, alkoxy, carboxy, sulfo, phosphate, amino, sulfate, phosphonate, cyano, nitro, azido, alkyl-amino, dialkyl-amino or trialkylammonium; or Rⁱ and R^{j} taken in combination form a ring-system that is optionally further substituted by one or more reactive or ionic substituents;
R¹ is selected from H, L-S_{c}, L-R^{x}, L-R^{±}, -CH₂-CONH-SO₂-Me, aliphatic groups, alicyclic groups, alkylaryl groups, aromatic groups, benzyl, substituted benzyl; where each aliphatic residue may incorporate up to six heteroatoms selected from N, O, S, and can be substituted one or more times by F, Cl, Br, I, hydroxy, alkoxy, carboxy, sulfo, phosphate, amino, sulfate, phosphonate, cyano, nitro, azido, alkyl-amino, dialkyl-amino or trialkylammonium;
R^{α}, R^{β} and R^{γ} are independently H, L-S_{c}, L-R^{x}, L-R^{±}, -CH₂-CONH-SO₂-Me, aliphatic, alicyclic, aromatic, alkyl-aryl, F, Cl, Br, I, NH₂, -COOH, CH=O, -CN, azido, - OH, -NO₂, -SO₃H, -PO₃^{2θ}, -O-PO₃^{2θ}, -PO₃R^{mθ}, -O-PO₃R^{mθ}, -CONH₂, CONHR^{m}, COO-NHS or COO-R^{m}, where R^{m} is selected from the group consisting of L-S_{c}, L-R^{x}, L-R^{±}, aliphatic substituents, aromatic substituents; where each aliphatic residue may incorporate up to six heteroatoms selected from N, O, S, and can be substituted one or more times by F, Cl, Br, I, hydroxy, alkoxy, carboxy, sulfo, phosphate, amino, sulfate, phosphonate, cyano, nitro, azido, alkyl-amino, dialkyl-amino or trialkylammonium; and
each of X¹, X², X³, and X⁴ are independently selected from the group consisting of N, 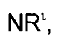 , O, S, and 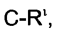 where 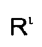 is hydrogen, L-S_{c}, L-R^{x}, L-R^{±}, -CH₂-CONH-SO₂-Me, or an aliphatic, alicyclic, or aromatic group; amino, sulfo, trifluoromethyl, alkoxy, halogen, carboxy, hydroxy, phosphate, or sulfate; or adjacent 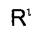 substituents, taken in combination, form a fused aromatic or heterocyclic ring that is itself optionally further substituted by H, L-S_{c}, L-R^{x}, L-R^{±}, alkyl, aryl or cycloalkyl;
R¹⁰ and R^{10'} for W³ and W⁴ is selected from R¹ and 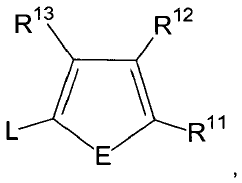 where L is a linker as defined above and E is selected from CR¹⁴=CR¹⁵, O, S, Se, NR¹⁶, CR¹⁴=N, CR¹⁴=N⁺R¹⁶;
where R¹¹, R¹², R¹³, R¹⁴ and R¹⁵ are independently H, L-S_{c}, L-R^{x}, L-R^{±}, -CH₂-CONH-SO₂-Me, aliphatic, alicyclic, aromatic, alkyl-aryl, F, Cl, Br, I, NH₂, -COOH, -CH=O, -CN, azido, -OH, -NO₂, -SO₃H,-PO₃^{2θ}, -O-PO₃^{2θ}, -PO₃R^{mθ}, -O-PO₃R^{mθ}, -CONH₂, CONHR^{m}, COO-NHS and COO-R^{m},
where R^{m} is selected from a group consisting of L-S_{c}, L-R^{x}, L-R^{±}, aliphatic substituents, aromatic substituents; where each aliphatic residue may incorporate up to six heteroatoms selected from N, O, S, and can be substituted one or more times by F, Cl, Br, I, hydroxy, alkoxy, carboxy, sulfo, phosphate, amino, sulfate, phosphonate, cyano, nitro, azido, alkyl-amino, dialkyl-amino or trialkylammonium; and
R¹⁶ is selected from H, L-S_{c}, L-R^{x}, L-R^{±}, -CH₂-CONH-SO₂-Me, aliphatic groups, alicyclic groups, alkylaryl groups, aromatic groups, each aliphatic residue may incorporate up to six heteroatoms selected from N, O, S, and can be substituted one or more times by F, Cl, Br, I, hydroxy, alkoxy, carboxy, sulfo, phosphate, amino, sulfate, phosphonate, cyano, nitro, azido, alkyl-amino, dialkyl-amino or trialkylammonium; and
R²⁰, R²¹, R²², R²³, R^{20'}, R^{21'}, R^{22'}, and R^{23'} of W⁵ and W⁶, and R³¹ and R³⁴ of W⁷ and W⁸ are independently selected from H, L-S_{c}, L-R^{x}, L-R^{±}, -CH₂-CONH-SO₂-Me, aliphatic, alicyclic, aromatic, alkyl-aryl, F, Cl, Br, I, NH₂, -COOH, CH=O, -CN, azido, - OH, -NO₂, -SO₃H, -PO₃^{2θ}, -O-PO₃^{2θ}, -PO₃R^{mθ}, -O-PO₃R^{mθ}, -CONH₂, CONHR^{m}, COO-NHS and COO-R^{m}, where R^{m} is selected from a group consisting of L-S_{c}, L-R^{x}, L-R^{±}, aliphatic substituents, aromatic substituents; where each aliphatic residue may incorporate up to six heteroatoms selected from N, O, S, and can be substituted one or more times by F, Cl, Br, I, hydroxy, alkoxy, carboxy, sulfo, phosphate, amino, sulfate, phosphonate, cyano, nitro, azido, alkyl-amino, dialkyl-amino or trialkylammonium, or R¹⁰ and R^{10'} may be a part of a heterocyclic ring that is itself optionally further substituted by H, L-S_{c}, L-R^{x}, L-R^{±}, alkyl or aryl;
U in W⁷ and W⁸ is independently selected from C(R³⁴) or nitrogen. R³² and R³³ are independently selected from H, L-S_{c}, L-R^{x}, L-R^{±}, -CH₂-CONH-SO₂-Me, aliphatic groups, alicyclic groups, alkylaryl groups, and aromatic groups; alternatively R³² and R³³ may be a part of a heterocyclic ring that is itself optionally further substituted by H, L-S_{c}, L-R^{x}, L-R^{±}, alkyl or aryl;
where at least one substituent of Z comprises an ionic substituent R^{±} selected from the group consisting of -SO₃^{θ}, -O-SO₃^{θ}, -COO^{θ}, -PO₃^{2θ}, -O-PO₃^{2θ}, -PO₃R^{mθ}, -O-PO₃R^{mθ} and -N(R^{l})₃⁺, where R^{m} and R^{l} are independently selected from the group consisting of hydrogen, aliphatic substituents, aromatic substituents, reactive substituents, reactive aromatic substituents, and conjugated substances, said ionic substituent R^{±} capable of increasing the hydrophilicity of the entire rotaxane.

2. The rotaxane of claim 1, further comprising a second reporter molecule selected from the group consisting of luminophores and chromophores.

3. A method of performing a photoluminescence assay, the method comprising:
selecting a photoluminescent rotaxane according to any one of claims 1-2;
exciting the photoluminescent rotaxane; and
detecting light emitted by the photoluminescent rotaxane, and optionally further comprising associating the photoluminescent rotaxane with a second molecule.

4. A kit for fluorescent labeling of a biological sample comprising a dye solution of a rotaxane according to any one of claims 1-2; and a buffer suitable for use with the biological sample.

5. A rotaxane according to any one of claims 1-2 for use in a method of diagnosing diseased tissue, comprising administering a rotaxane according to any one of claims 1-2, exposing the diseased tissue to light in the visible and near infrared range and recording the emitted light produced from the rotaxane.

## Patentansprüche

1. Ein Rotaxan mit der Formel: 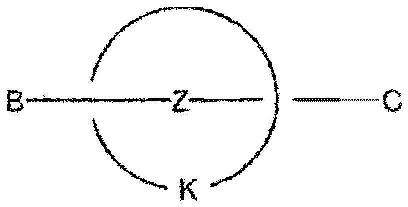 worin:
K die Formel K¹, K², K³ aufweist, wobei
K¹ für 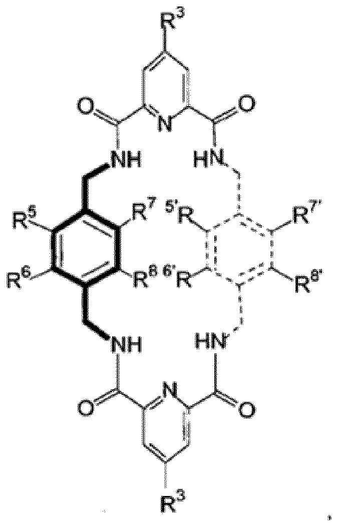 steht,
K² for 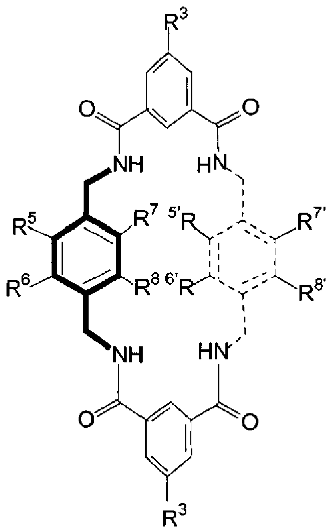 steht und
K³ für 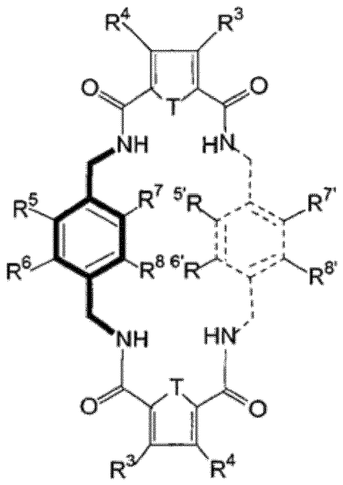 steht;
worin die Substituenten R³, R⁴, R⁵, R^{5'}, R⁶, R^{6'}, R⁷, R^{7'}, R⁸ und R^{8'}, falls vorhanden, jeweils unabhängig ausgewählt werden aus der Gruppe bestehend aus H, L-R^{x}, L-S_{c} and L-R^{±}, Alkyl, Aryl, Alkoxy, Alkylaryl, F, Br, Cl, I, OH, Nitro und Cyano;
R⁵ in Kombination mit R⁶, R⁷ in Kombination mit R⁸, R^{5'} in Kombination mit R^{6'} und R^{7'} in Kombination mit R^{8'} jeweils unabhängig ein aromatisches, heterozyklisches oder aliphatisches Ringsystem bilden kann;
L eine einzelne kovalente Bindung ist, eine kovalente Verknüpfung, die linear und verzweigt ist, zyklisch oder heterozyklisch, gesättigt oder ungesättigt ist und 1-20 Nicht-Wasserstoffatome aus der Gruppe C, N, P, O und S in einer Weise aufweist, dass die Verknüpfung irgendeine Kombination aus Ether-, Thioether-, Amin-, Ester-, Amid-Bindungen; Einzel-, Doppel-, Dreifach- oder aromatischen Kohlenstoff-Kohlenstoff-Bindungen; oder Kohlenstoff-Schwefel-Bindungen, Kohlenstoff-Stickstoff-Bindungen, Phosphor-Schwefel-, Stickstoff-Stickstoff-, Stickstoff-Sauerstoff- oder Stickstoff-Platin-Bindungen oder aromatischen oder heteroaromatischen Bindungen enthält;
R^{x} ist eine reaktive Gruppe, die ausgewählt wird aus aktiviertem Carboxylester, Acylazid, Acylhalid, Acylnitril, Aldehyd, Keton, Alkylhalogenid, Alkylsulfonat, Anhydrid, Arylhalogenid, Aziridin, Boronat, Carboxylsäure, Carbodiimid, Diazoalkan, Epoxid, Haloacetamid, Halotriazin, Imidoester, Isocyanat, Isothiocyanat, Maleimid, Phosphoramidit, Silylhalogenid, Sulfonatester und Sulfonylhalid, oder die ausgewählt wird aus N-Hydroxysuccinimidestern, Isothiocyanaten, Sulfonylchloriden, lodacetamiden, Maleimiden, N-Hydroxybenztriazolestern, Thioestern, p-Nitrophenylestern, Alkyl, Alkenyl, Alkynyl und aromatischen Estern, Acylimidazolen, Alkylhalogeniden, Hydroxylgruppen, Aldehyden, Ketonen, Hydrazonen, Oximen, Semicarbozonen, Isocyanaten, aktivierten C=C-Doppelbindungen, Thiolgruppen, Alkenen, Phosphoramiditen, primären Aminen, Borsäurederivaten, Pyryliumgruppen, Haloplatinaten und Arylhalogeniden;
S_{c} ist eine konjugierte Substanz, die aus einer Aminosäure, einem Peptid, einem Protein, einem Nukleosid, einem Nukleotid, einer Nukleinsäure, einem Kohlehydrat, einem Hapten, einem Lipid, einem Ionen-Chelator, einem nicht-biologischen Polymer, einer Zelle oder einer Zellkomponente ausgewählt wird, oder aus metallischen Nanopartikeln ausgewählt wird, oder ausgewählt wird aus der Gruppe bestehend aus einem Polypeptid, einem Polynukleotid, einem Bead, einer Mikroplattenvertiefungsfläche, einem Phospholipid, einem Zucker, einem Polysaccharid, einem Oligosaccharid und einem zweiten fluoreszierenden Farbstoff; und
R^{±} eine Ionengruppe ist;
T für O, S, N-H steht;
Z die folgende Formel aufweist: 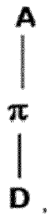 worin π ein viergliedriger aromatischer Ring ist und A, B, C und D die Substituenten des viergliedrigen Rings sind;
und wenn B und C durch einen der Substituenten A oder D getrennt sind, B ist entweder W², W⁴, W⁶ oder W⁸ und C ist W¹, W³, W⁵ oder W⁷, in welchem Fall einer von A oder D negativ geladen ist;
wenn A und D neutral sind, werden sie ausgewählt aus der Gruppe bestehend aus =O, =S, =Se, =Te, =N-R^{a} und =C(R^{b})(R^{c}), worin R^{a}, R^{b} und R^{c} ausgewählt werden aus der Gruppe bestehend aus H, L-S_{c}, L-R^{x}, L-R^{±}, aliphatisch, aromatisch, alizyklisch, sowie Arylalkyl, wobei jeder aliphatische Rest bis zu sechs Heteroatome umfassen kann, die ausgewählt werden aus N, O, S und einmal oder mehrmals substituiert werden können durch F, Cl, Br, I, Hydroxy, Alkoxy, Carboxy, Sulfo, Phosphat, Amino, Sulfat, Phosphonat, Cyano, Nitro, Azido, Alkylamino, Dialkylamino oder Trialkylammonium; - COOH, -CN, -OH, -SO₃H, -Po₃H₂, -O-PO₃H₂, -PO₃R₂^{m}, -O-PO₃R₂^{m},-CONHR^{m}, -CONH₂, COO-NHS und COO-R^{m}, worin R^{m} ausgewählt wird aus einer Gruppe bestehend aus L-S_{c}, L-R^{x}, L-R^{±}, aliphatischen Substituenten und aromatischen Substituenten; oder R^{b} und R^{c} in Kombination eine zyklische oder heterozyklische Ringstruktur bilden;
wenn A und D vorhanden und negativ geladen sind, werden sie jeweils unabhängig aus der Gruppe von -O^{θ}, -S^{θ}, -Se^{θ}, -Te^{θ}, -(N-R^{a})^{θ}, -(C(R^{b})(R^{c}))^{θ} ausgewählt;
W¹, W², W³, W⁴, W⁵, W⁶, W⁷ und W⁸ haben die entsprechenden formeln, wobei
W¹ für 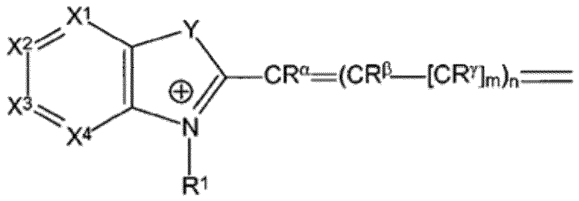 steht,
W² für 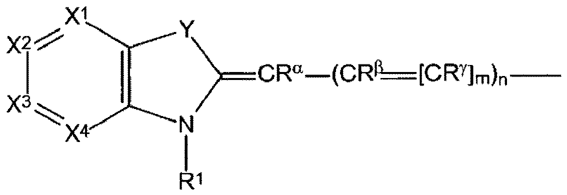 steht,
W³ füᵣ 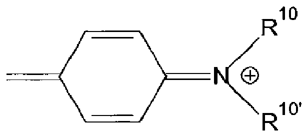 steht,
W⁴ für 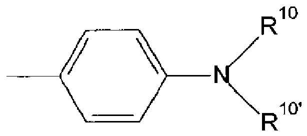 steht,
W⁵ für 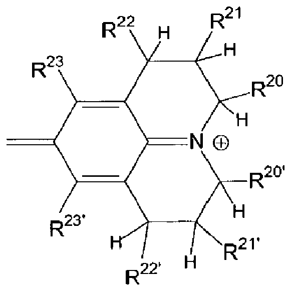 steht,
W⁶ für 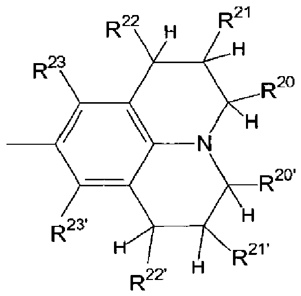 steht,
W⁷ für 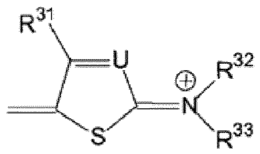 steht,
W⁸ für 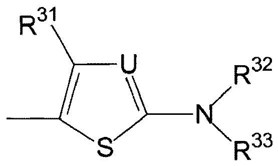 steht;
worin m und n jeweils unabhängig 0 oder 1 für jeden von B und C sind;
jedes Y unabhängig aus der Gruppe bestehend aus O, S, N-R^{d}, CR^{e}=CR^{f} und C(Rⁱ)(R^{j}) ausgewählt wird,
worin R^{d} aus der Gruppe bestehend aus H, L-S_{c}, L- R^{x}, L-R^{±}, -CH₂-CONH-SO₂-Me, aliphatischen Gruppen, alizyklischen Gruppen, aromatischen Gruppen ausgewählt wird;
R^{e} und R^{f} jeweils unabhängig H, L-S_{c}, L-R^{x}, L-R^{±}, -CH₂-CONH-SO₂-Me, aliphatische Gruppen, alizyklische Gruppen oder aromatische Gruppen sind; worin jeder aliphatische Rest bis zu sechs Heteroatome umfassen kann, die ausgewählt werden aus N, O, S, und einmal oder mehrmals durch F, Cl, Br, I, Hydroxy, Alkoxy, Carboxy, Sulfo, Phosphat, Amino, Sulfat, Phosphonat, Cyano, Nitro, Azido, Alkylamino, Dialkylamino oder Trialkylammonium substituiert werden kann;
Rⁱ und R^{j} jeweils unabhängig H, L-S_{c}, L-R^{x}, L-R^{±}, -CH₂-CONH-SO₂-Me, aliphatische Gruppen, alizyklische Gruppen oder aromatische Gruppen sind, worin jeder aliphatische Rest bis zu sechs Heteroatome ausgewählt von N, O, S integriert haben kann und einmal oder mehrmals durch F, Cl, Br, I, Hydroxy, Alkoxy, Carboxy, Sulfo, Phosphat, Amino, Sulfat, Phosphonat, Cyano, Nitro, Azido, Alkylamino, Dialkylamino oder Trialkylammonium substituiert werden kann; oder Rⁱ und R^{j} in Kombination ein Ringsystem bilden, das wahlweise weiter durch einen oder mehrere reaktive oder ionische Substituenten substituiert werden kann;
R¹ wird aus H, L-S_{c}, L-R^{x}, L-R^{±}, -CH₂-CONH-SO₂-Me, aliphatischen Gruppen, alizyklischen Gruppen, Alkylarylgruppen, aromatischen Gruppen, Benzyl, substituiertem Benzyl ausgewählt; worin jeder aliphatische Rest bis zu sechs Heteroatome ausgewählt von N, O, S integriert haben kann und einmal oder mehrmals durch F, Cl, Br, I, Hydroxy, Alkoxy, Carboxy, Sulfo, Phosphat, Amino, Sulfat, Phosphonat, Cyano, Nitro, Azido, Alkylamino, Dialkylamino oder Trialkylammonium substituiert werden kann;
R^{α}, R^{ß} and R^{γ} sind unabhängig H, L-S_{c}, L-R^{x}, L-R^{±}, -CH₂-CONH-SO₂-Me, aliphatisch, alizyklisch, aromatisch, Alkylaryl, F, Cl, Br, I, NH₂,-COOH, CH=O, -CN, Azido, -OH, -NO₂, -SO₃H, -PO₃^{2θ}, -O-PO₃^{2θ}, -PO₃R^{mθ}, -O-PO₃R^{mθ}, -CONH₂, CONHR^{m}, COO-NHS oder COO-R^{m} sind, worin R^{m} aus der Gruppe L-S_{c}, L-R^{x}, L-R^{±}, aliphatischen Substituenten, aromatischen Substituenten ausgewählt wird; worin jeder aliphatische Rest bis zu sechs Heteroatome ausgewählt von N, O, S integriert haben kann und einmal oder mehrmals durch F, Cl, Br, I, Hydroxy, Alkoxy, Carboxy, Sulfo, Phosphat, Amino, Sulfat, Phosphonat, Cyano, Nitro, Azido, Alkylamino, Dialkylamino oder Trialkylammonium substituiert werden kann; und
jedes X¹, X², X³ und X⁴ jeweils unabhängig aus der Gruppe bestehend aus N, NR¹, O, S und C-R¹ ausgewählt wird, worin R¹ Wasserstoff, L-S_{c}, L-R^{x}, L-R^{±}, -CH₂-CONH-SO₂-Me oder eine aliphatische, alizyklische oder aromatische Gruppe ist; Amino, Sulfo, Trifluoromethyl, Alkoxy, Halogen, Carboxy, Hydroxy, Phosphat oder Sulfat ist; oder angrenzende R¹-Substituenten in Kombination einen miteinander verbundenen aromatischen oder heterozyklischen Ring bilden, der selbst wiederum wahlweise weiter durch H, L-S_{c}, L-R^{x}, L-R^{±}, Alkyl, Aryl oder Cycloalkyl substituiert werden kann;
R¹⁰ und R^{10'} für W³ und W⁴ aus R¹ und 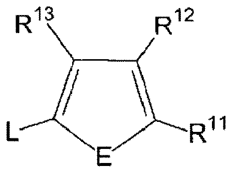 ausgewählt werden,
worin L ein Linker gemäß obiger Definition ist und E aus CR¹⁴=CR¹⁵, O, S, Se, NR¹⁶, CR¹⁴=N, CR¹⁴=N⁺R¹⁶ ausgewählt wird;
worin R¹¹, R¹², R¹³, R¹⁴ und R¹⁵ jeweils unabhängig H, L-S_{c}, L-R^{x}, L-R^{±}, -CH₂-CONH-SO₂-Me, aliphatisch, alizyklisch, aromatisch, Alkylaryl, F, Cl, Br, I, NH₂, -COOH, -CH=O, -CN, Azido, -OH, -NO₂, - SO₃H, -PO₃^{2θ}, -O-PO₃^{2θ}, -PO₃R^{mθ}, -O-PO₃R^{mθ}, -CONH₂, CONHR^{m}, COO-NHS und COO-R^{m} sind,
worin R^{m} ausgewählt wird aus einer Gruppe bestehend aus L-Sc, L-R^{x}, L-R^{±}, aliphatischen Substituenten, aromatischen Substituenten; worin jeder aliphatische Rest bis zu sechs Heteroatome ausgewählt von N, O, S integriert haben kann und einmal oder mehrmals durch F, Cl, Br, I, Hydroxy, Alkoxy, Carboxy, Sulfo, Phosphat, Amino, Sulfat, Phosphonat, Cyano, Nitro, Azido, Alkylamino, Dialkylamino oder Trialkylammonium substituiert werden kann; und
R¹⁶ ausgewählt wird aus H, L-S_{c}, L-R^{x}, L-R^{±}, -CH₂-CONH-SO₂-Me, aliphatischen Gruppen, alizyklischen Gruppen, Alkylarylgruppen, aromatischen Gruppen, worin jeder aliphatische Rest bis zu sechs Heteroatome ausgewählt von N, O, S integriert haben kann und einmal oder mehrmals durch F, Cl, Br, I, Hydroxy, Alkoxy, Carboxy, Sulfo, Phosphat, Amino, Sulfat, Phosphonat, Cyano, Nitro, Azido, Alkylamino, Dialkylamino oder Trialkylammonium substituiert werden kann;und
wobei R²⁰, R²¹, R²², R²³,R^{20'}, R^{21'}, R^{22'} und R^{23'} von W⁵ und W⁶ sowie R³¹ und R³⁴ von W⁷ and W⁸ jeweils unabhängig aus der Gruppe bestehend aus H, L-S_{c}, L-R^{x}, L-R^{±}, -CH₂-CONH-SO₂-Me, aliphatisch, alizyklisch, aromatisch, Alkylaryl, F, Cl, Br, I, NH₂,-COOH, CH=O, -CN, Azido, -OH, -NO₂, -S03H, -PO₃^{2θ}, -O-PO₃^{2θ}, -PO₃R^{mθ}, -O-PO₃R^{mθ}, -CONH₂, CONHR^{m}, COO-NHS und COO-R^{m} ausgewählt werden, worin R^{m} aus einer Gruppe bestehend aus L-S_{c}, L-R^{x}, L-R^{±}, aliphatischen Substituenten, aromatischen Substituenten ausgewählt wird; worin jeder aliphatische Rest bis zu sechs Heteroatome ausgewählt von N, O, S integriert haben kann und einmal oder mehrmals durch F, Cl, Br, I, Hydroxy, Alkoxy, Carboxy, Sulfo, Phosphat, Amino, Sulfat, Phosphonat, Cyano, Nitro, Azido, Alkylamino, Dialkylamino oder Trialkylammonium substituiert werden kann, oder worin R¹⁰ and R¹⁰, Teil eines heterozyklischen Rings sein können, der selbst wiederum wahlweise durch H, L-S_{c}, L-R^{x}, L-R^{±}, Alkyl oder Aryl substituiert werden kann;
U in W⁷ und W⁸ unabhängig aus C(R³⁴) oder Stickstoff ausgewählt wird. R³² und R³³ jeweils unabhängig ausgewählt werden aus H, L-S_{c}, L-R^{x}, L-R^{±}, -CH₂-CONH-SO₂-Me, aliphatischen Gruppen, alizyklischen Gruppen, Alkylarylgruppen und aromatischen Gruppen; alternativ dazu können R³² und R³³ Teil eines heterozyklischen Rings sein, der selbst wiederum wahlweise durch H, L-S_{c}, L-R^{x}, L-R^{±}, Alkyl oder Aryl substituiert werden kann;
worin mindestens ein Substituent von Z einen ionischen Substituenten R^{±} umfasst, der aus der Gruppe -SO₃^{θ}, -O-SO₃^{θ}, -COO^{θ}, -PO₃^{2θ}, -O-PO₃^{2θ}, -PO₃R^{mθ}, - O-PO₃R^{mθ} und -N(R¹)₃⁺ ausgewählt wird, worin R^{m} und R¹ jeweils unabhängig aus der Gruppe bestehend aus Wasserstoff, aliphatischen Substituenten, aromatischen Substituenten, reaktiven Substituenten, reaktiven aromatischen Substituenten und konjugierten Substanzen ausgewählt werden, wobei der genannte ionische Substituent R^{±} in der Lage ist, die Wasserlöslichkeit des gesamten Rotaxans zu erhöhen.

2. Das Rotaxan von Anspruch 1, dass ein zweites Reporter-Molekül, ausgewählt aus der Gruppe bestehend aus Luminophoren und Chromophoren includiert.

3. Ein Verfahren zur Durchführung eines Photolumineszenz-Tests, wobei das Verfahren folgendes umfasst:
die Auswahl eines photolumineszenten Rotaxans nach einem der Ansprüche 1-2;
Anregung des photolumineszenten Rotaxans; und
Dedektion des Lichtes, das von dem photolumineszenten Rotaxan ausgestrahlt wird, und überdies wahlweise die Assoziation des photolumineszenten Rotaxans mit einem zweiten Molekül.

4. Ein Kit zur Fluoreszenz-Markierung einer biologischen Probe, umfassend eine Farbstofflösung eines Rotaxans nach einem der Ansprüche 1-2; und einen Puffer, der zur Verwendung mit einer biologischen Probe geeignet ist.

5. Ein Rotaxan nach einem der Ansprüche 1-2 zur Anwendung in einem Verfahren zur Diagnose von erkranktem Gewebe, die die Verabreichung eines Rotaxans nach einem der Ansprüche 1-2, Exposition des erkrankten Gewebes mit sichtbaren und nahen infrarot Licht und Aufzeichnung des vom Rotaxan produzierten Lichtes umfasst.

## Revendications

1. Rotaxane ayant la formule : 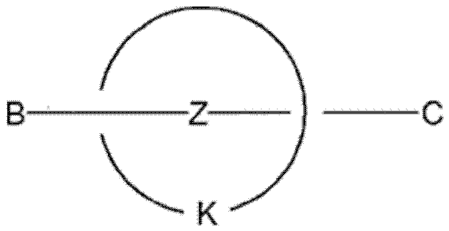 où :
K a la formule K¹, K², K³, où
K¹ est 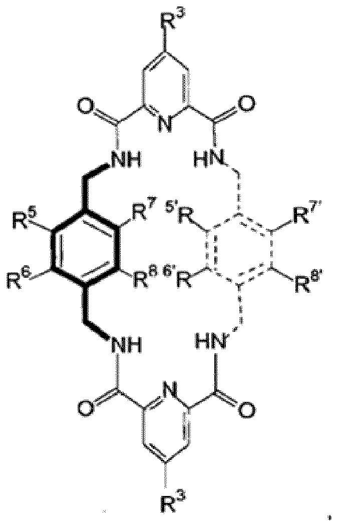
K² est 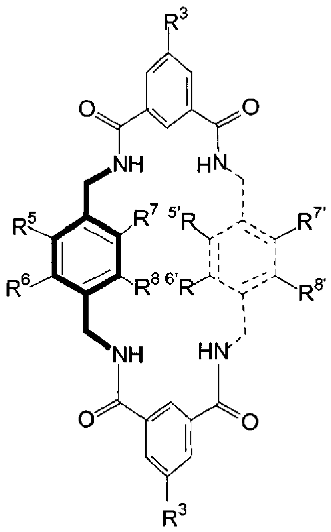 , et
K³ est 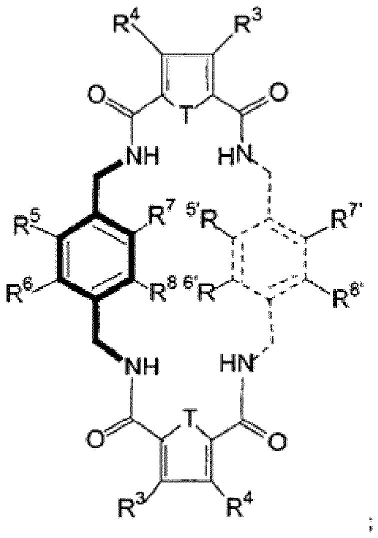 où les substituants R³, R⁴, R⁵, R^{5'}, R⁸, R⁶, R⁷, R⁷, R⁸ et R^{8'}, lorsqu'ils sont présents, sont indépendamment choisis dans le groupe de H, L-R^{x}, L-S_{c} et L-R^{±}, un groupe alkyle, un groupe aryle, alkoxy, alkyle-aryle, F, Br, CI, I, OH, nitro, et cyano ;
R⁵ en association avec R⁶, R⁷ en association avec R⁸, R^{5'} en association avec R^{6'}, et R⁷ en association avec R^{8'} peuvent former indépendamment un cycle aromatique, hétérocyclique ou un système cycle aliphatique ;
L est une liaison covalente simple, une liaison covalente qui est linéaire ou ramifiée, cyclique ou hétérocyclique, saturée ou insaturée, ayant 1 à 20 atomes non hydrogènes choisis dans le groupe de C, N, P, O et S, de telle sorte que la liaison contienne une combinaison d'éther, de thioéther, d'amine, d'ester, de liaisons amide ; de liaisons carbone-carbone simples, doubles, triples ou aromatiques ; ou de liaisons carbone-soufre, de liaisons carbone-azote, de liaisons phosphore-soufre, azote-azote, azote-oxygène ou de liaisons azote-platine, ou de liaisons aromatiques ou hétéroaromatiques ;
R^{x} est un groupe réactif choisi parmi l'ester carboxylique activé, l'azoture d'acyle, l'halogénure d'acyle, le nitrile d'acyle, l'aldéhyde, la cétone, l'halogénure d'alkyle, le sulfonate d'alkyle, l'anhydride, l'halogénure d'aryle, l'aziridine, le boronate, l'acide carboxylique, le carbodiimide, le diazoalcane, l'époxyde, l'haloacétamide, l'halogénotriazine, l'imido ester, l'isocyanate, l'isothiocyanate, le maléimide, le phosphoramidite, l'halogénure de silyle, l'ester de sulfonate, et l'halogénure de sulfonyle, ou est choisi parmi les esters de N-hydroxysuccinimide, les isothiocyanates, les sulfonylchlorides, les iodoacétamides, les maléimides, les esters de N-hydroxybenzotriazole, les thioesters, les esters de p-nitrophényl, les esters d'alkyle, d'alcényle, d'alcynyle, et les esters aromatiques, les imidazoles d'acyle, les halogénures d'alkyle, les groupes hydroxyle, les aldéhydes, les cétones, les hydrazones, les oximes, les semicarbozones, les isocyanates, les groupes contenant une double liaison activée C=C, les groupes thiol, les alcènes, les phosphoramidites, les amines primaires, les dérivés d'acide boronique, les fragments de pyrylium, les haloplatinates, et les halogénures d'aryle ;
S_{c} est une substance conjuguée choisie parmi un acide aminé, un peptide, une protéine, un nucléoside, un nucléotide, un acide nucléique, un hydrate de carbone, un haptène, un lipide, un ion-chélateur, un polymère non biologique, une cellule ou un composant cellulaire ou est choisi parmi des nanoparticules métalliques, ou est choisi dans le groupe constitué par un polypeptide, un polynucléotide, une bille, une surface de puits de microplaque, un phospholipide, un sucre, un polysaccharide, un oligosaccharide, et un deuxième colorant fluorescent ; et
R^{±} est un groupe ionique ;
T est O, S, N-H ;
Z a la formule 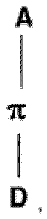 où π est un cycle aromatique à quatre chaînons, et A, B, C et D sont les substituants du cycle à quatre chaînons ;
et où B et C sont séparés par l'un des substituants A ou D, B est l'un des W², W⁴, W⁶ ou W⁸ et C est l'un de W¹, W³, W⁵ or W⁷, dans ce cas, l'un de A ou D est chargé négativement ;
où A et D sont neutres, ils sont choisis dans le groupe constitué par =O, =S, =Se, =Te, =N-R^{a}, et =C(R^{b})(R^{c}), où R^{a}, R^{b} and R^{c} sont choisis dans le groupe constitué de H, L-S_{c}, L-R^{x}, L-R^{±}, aliphatique, aromatique, alicyclique et aryle-alkyle, où chaque résidu aliphatique peut incorporer jusqu'à six hétéroatomes choisis parmi N, O, S, et peut être substitué une ou plusieurs fois par F, CI, Br, I, hydroxy, alkoxy, carboxy, sulfo, phosphate, amino, sulfate, phosphonate, cyano, nitro, azido, amino-alkyle, dialkyl-amino ou trialkylammonium ; -COOH, -CN, -OH, -SO₃H, -PO₃H₂, -O-PO₃H₂, -PO₃R₂^{m},-O-PO₃R₂^{m}-CONHR^{m}, -CONH₂, COO-NHS et COO-R^{m}, où R^{m} est choisi parmi un groupe constitué de L-S_{c}, L-R^{x}, L-R^{±}, des substituants aliphatiques et des substituants aromatiques, ou R^{b} et R^{c}, pris en combinaison, forment une structure de noyau cyclique ou hétérocyclique ;
où A et D sont présents et chargés négativement, ils sont indépendamment choisis dans le groupe constitué de -O^{θ}, -S^{θ}, -Se^{θ}, -Te^{θ}, -(N-R^{a})^{θ}, -(C(R^{b})(R^{c})) ;
W¹, W², W³, W⁴, W⁵, W⁶, W⁷ et W⁸ ont les formules respectives
W¹ est 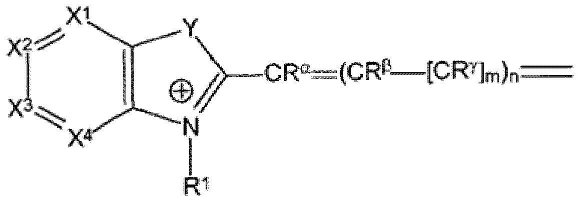
W² est 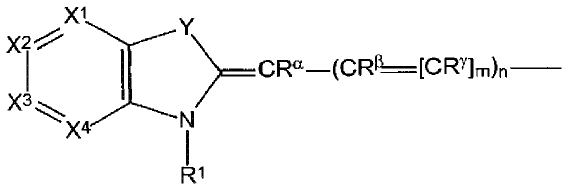
W³ est 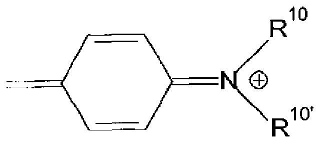
W⁴ est 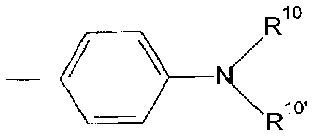
W⁵ est 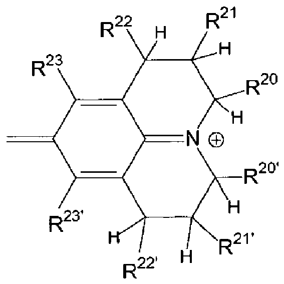
W⁶ est 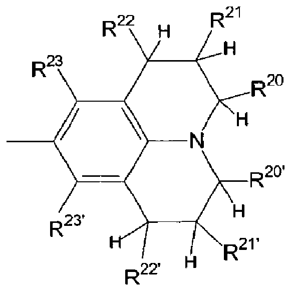
W⁷ est 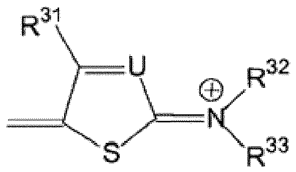
W⁸ est 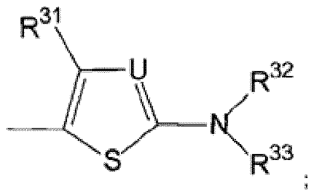 où m et n sont indépendamment 0 ou 1 pour chacun de B et C ;
chaque Y est indépendamment choisi dans le groupe constitué par O, S, N-R^{d}, CR^{e}=CR^{f} et C(Rⁱ)(R^{j}),
où R^{d} est choisi dans le groupe constitué de H, L-S_{c}, L-R^{x}, L-R^{±}, -CH₂-CONH-SO₂-Me, des groupes aliphatiques, des groupes alicycliques, des groupes aromatiques ;
R^{e} et R^{f} sont indépendamment H, L-S_{c}, L-R^{x}, L-R^{±}, -CH₂-CONH-SO₂-Me, des groupes aliphatiques, des groupes alicycliques ou des groupes aromatiques ; où chaque résidu aliphatique peut comporter jusqu'à six hétéroatomes choisis parmi N, O, S, et peut être substitué une ou plusieurs fois par F, CI, Br, I, hydroxy, alkoxy, carboxy, sulfo, phosphate, amino, sulfate, phosphonate, cyano, nitro, azido, amino-alkyle, dialkyl-amino ou trialkylammonium ;
Rⁱ et R^{j} sont indépendamment H, L-S_{c}, L-R^{x}, L-R^{±}, -CH₂-CONH-SO₂-Me, des groupes aliphatiques, des groupes alicycliques ou des groupes aromatiques, où chaque résidu aliphatique peut incorporer jusqu'à six hétéroatomes choisis à partir de N, O, S, et qui peut être substitué une ou plusieurs fois par F, CI, Br, I, hydroxy, alkoxy, carboxy, sulfo, phosphate, amino, sulfate, phosphonate, cyano, nitro, azido, amino-alkyle, dialkyl-amino ou trialkylammonium ; ou Rⁱ et R^{j} pris en combinaison forment un système cyclique qui est éventuellement substitué en outre par un ou plusieurs
substituants réactifs ou ioniques ;
R¹ est choisi parmi H, L-S_{c}, L-R^{x}, L-R^{±}, -CH₂-CONH-SO₂-Me, des groupes aliphatiques, des groupes alicycliques, des groupes alkylaryles, des groupes aromatiques, benzyle, benzyle substitué ; où chaque résidu aliphatique peut comporter jusqu'à six hétéroatomes choisis parmi N, O, S, et peut être substitué une ou plusieurs fois par F, CI, Br, I, hydroxy, alkoxy, carboxy, sulfo, phosphate, amino, sulfate, phosphonate, cyano, nitro, azido, amino-alkyle, dialkyl-amino ou trialkylammonium ;
R^{α}, R^{β} et R^{γ} sont indépendamment H, L-S_{c}, L-R^{x}, L-R^{±}, -CH₂-CONH-SO₂-Me, aliphatiques, alicycliques, aromatiques, alkyl-aryle, F, CI, Br, I, NH₂,-COOH, CH=O, - CN, azido, -OH, -NO₂, -SO₃H, -PO₃^{2θ}, -O-PO₃^{2θ}, -PO₃R^{mθ}, -O-PO₃R^{mθ}, -CONH₂, CONHR^{m}, COO-NHS ou COO-R^{m}, où R^{m} est choisi parmi un groupe constitué de L-S_{c}, L-R^{x}, L-R^{±}, des substituants aliphatiques, des substituants aromatiques ; où chaque résidu aliphatique peut comporter jusqu'à six hétéroatomes choisis parmi N, O, S, et peut être substitué une ou plusieurs fois par F, CI, Br, I, hydroxy, alkoxy, carboxy, sulfo, phosphate, amino, sulfate, phosphonate, cyano, nitro, azido, amino-alkyle, dialkyl-amino ou trialkylammonium ; et
chacun des X¹, X², X³, et X⁴ sont choisis indépendamment dans le groupe constitué de N, NR^{l} O, S, et C-R^{l} est un atome d'hydrogène, L-S_{c}, L-R^{x}, L-R^{±}, -CH₂-CONH-SO₂-Me, ou un groupe aliphatique, alicyclique, ou aromatique; amino, sulfo, trifluorométhyle, alkoxy, halogéno, carboxy, hydroxy, phosphate ou sulfate ; ou les substituants adjacents R^{l}, pris en combinaison, forment un noyau aromatique ou hétérocyclique condensé qui est lui-même éventuellement substitué par H, L-S_{c}, L-R^{x}, L-R^{±}, un groupe alkyle, aryle ou cycloalkyle ;
R¹⁰ et R¹⁰ pour W³ et W⁴ est choisi parmi R¹ et 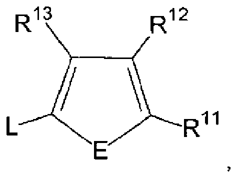 où L est un lieur tel que défini ci-dessus et E est choisi CR¹⁴=CR¹⁵, O, S, Se, NR¹⁶, CR¹⁴=N, CR¹⁴=N+R¹⁶;
où R¹¹, R¹², R¹³, R¹⁴ et R¹⁵ sont indépendamment H, L-S_{c}, L-R^{x}, L-R^{±}, -CH₂-CONH-SO₂-Me, aliphatiques, alicycliques, aromatiques, alkyl-aryle, F, CI, Br, I, NH₂, -COOH, -CH=O, -CN, azido, -OH, -NO₂, - SO₃H, -PO₃^{2θ}, -O-PO₃^{2θ}, -PO₃R^{mθ}, -O-PO₃R^{mθ}, -CONH₂, CONHR^{m}, COO-NHS et COO-R^{m},
où R^{m} est choisi parmi un groupe constitué de L-S_{c}, L-R^{x}, L-R^{±}, des substituants aliphatiques, des substituants aromatiques ; où chaque résidu aliphatique peut comporter jusqu'à six hétéroatomes choisis parmi N, O, S, et peut être substitué une ou plusieurs fois par F, CI, Br, I, hydroxy, alkoxy, carboxy, sulfo, phosphate, amino, sulfate, phosphonate, cyano, nitro, azido, amino-alkyle, dialkyl-amino ou trialkylammonium ; et
R¹⁶ est choisi parmi H, L-S_{c}, L-R^{x}, LR^{±}, -CH₂-CONH-SO₂-Me, des groupes aliphatiques, des groupes alicycliques, des groupes alkylaryle, des groupes aromatiques, chaque résidu aliphatique peut comporter jusqu'à six hétéroatomes choisis parmi N, O, S, et qui peut être substitué une ou plusieurs fois par F, CI, Br, I, hydroxy, alkoxy, carboxy, sulfo, phosphate, amino, sulfate, phosphonate, cyano, nitro, azido, amino-alkyle, dialkyl-amino ou trialkylammonium ; et
R²⁰, R²¹, R²², R²³, R^{20'}, R^{21'}, R^{22'}, et R^{23'} de W⁵ et W⁶ et R³¹ et R³⁴ de W⁷ et W⁸ sont choisis indépendamment parmi H, L-S_{c}, L-R^{x}, L-R^{±}, -CH₂-CONH-SO₂-Me, aliphatiques, alicycliques, aromatiques, alkyl-aryle, F, CI, Br, I, NH₂,-COOH, CH=O, - CN, azido, -OH, -NO₂, -SO₃H, -PO₃^{2θ}, -O-PO₃^{2θ}, -PO₃R^{mθ}, -O-PO₃R^{mθ}, -CONH₂, CONHR^{m}, COO-NHS and COO-R^{m}, où R^{m} est choisi parmi un groupe constitué de L-S_{c}, L-R^{x}, L-R^{±}, des substituants aliphatiques, des substituants aromatiques ; où chaque résidu aliphatique peut comporter jusqu'à six hétéroatomes choisis parmi N, O, S, et peut être substitué une ou plusieurs fois par F, CI, Br, I, hydroxy, alkoxy, carboxy, sulfo, phosphate, amino, sulfate, phosphonate, cyano, nitro, azido, amino-alkyle, dialkyl-amino ou trialkylammonium, ou R¹⁰ et R^{10'} peuvent être une partie d'un noyau hétérocyclique qui est lui-même éventuellement substitué par H, L-S_{c}, L-R^{x}, L-R^{±}, un groupe alkyle ou aryle ;
U en W⁷ et W⁸ est indépendamment choisi parmi C(R³⁴) ou l'azote. R³² et R³³ sont choisis indépendamment parmi H, L-S_{c}, L-R^{x}, L-R^{±}, -CH₂-CONH-SO₂-Me, des groupes aliphatiques, des groupes alicycliques, des groupes alkylaryle, et des groupes aromatiques ; alternativement, R³² et R³³ peuvent faire partie d'un noyau hétérocyclique qui est lui-même éventuellement substitué par H, L-S_{c}, L-R^{x}, L-R^{±}, un groupe alkyle ou aryle ;
où au moins un substituant de Z comporte un substituant R^{±} ionique choisi dans le groupe constitué de -SO₃^{θ}, -O-SO₃^{θ}, -COO^{θ}, -PO₃^{2θ}, -O-PO₃^{2θ}, -PO₃R^{mθ}, -O-PO₃R^{mθ} et -N(R^{l})₃⁺ où R^{m} et R¹ sont indépendamment choisis dans le groupe constitué par l'hydrogène, des substituants aliphatiques, des substituants aromatiques, des substituants réactifs, des substituants aromatiques réactifs, et des substances conjuguées, ledit substituant R^{±} ionique capable d'augmenter le caractère hydrophile de l'ensemble de rotaxane.

2. Rotaxane selon la revendication 1, comprenant en outre une deuxième molécule rapporteuse choisie dans le groupe constitué par les luminophores et les chromophores.

3. Procédé de réalisation d'un essai de photoluminescence, le procédé comprenant :
la sélection d'un rotaxane photoluminescent selon l'une quelconque des revendications 1 à 2 ;
l'excitation du rotaxane photoluminescent ; et
la détection de la lumière émise par le rotaxane photoluminescent, et éventuellement comprenant en outre l'association du rotaxane photoluminescent avec une seconde molécule.

4. Kit pour le marquage fluorescent d'un échantillon biologique comprenant une solution de teinture d'un rotaxane selon l'une quelconque des revendications 1 à 2 ; et un tampon convenable pour une utilisation avec l'échantillon biologique.

5. Rotaxane selon l'une quelconque des revendications 1 à 2 pour une utilisation dans un procédé de diagnostic d'un tissu malade, comprenant l'administration d'un rotaxane selon l'une quelconque des revendications 1 à 2, en exposant le tissu malade à la lumière dans le spectre visible et près du spectre infrarouge et en enregistrant la lumière émise produite à partir du rotaxane.
